# EUROPEAN PATENT SPECIFICATION

(11) **EP 3 510 045 B1**
(45) Date of publication and mention of the grant of the patent: **03.11.2021**
(21) Application number: 17784704.3
(22) Date of filing: 11.09.2017
(51) Int. Cl.: C07K 16/10, A61K 39/42, G01N 33/569, G01N 33/577, C12N 7/00, C07K 14/005

(54) **MONOCLONAL ANTIBODIES AGAINST HEMAGGLUTININ OF H5-SEROTYPE INFLUENZA VIRUSES AND THEIR USES, HYBRIDOMAS PRODUCING SAID ANTIBODIES, COMPOSITIONS AND DIAGNOSTIC KITS**
MONOKLONALE ANTIKÖRPER GEGEN HÄMAGGLUTININ VON H5-SEROTYP-INFLUENZAVIREN UND DEREN VERWENDUNGEN, DIESE ANTIKÖRPER PRODUZIERENDE HYBRIDOME, ZUSAMMENSETZUNGEN UND DIAGNOSEKITS
ANTICORPS MONOCLONAUX CONTRE L'HÉMAGGLUTININE DES VIRUS DE LA GRIPPE DE SÉROTYPE H5 ET LEURS UTILISATIONS, HYBRIDOMES PRODUISANT LESDITS ANTICORPS, COMPOSITIONS ET KITS DE DIAGNOSTIC

(30) Priority: 12.09.2016 PL 41867116
(43) Date of publication of application: 17.07.2019
(73) Proprietor: Siec Badawcza Lukasiewicz - Instytut Chemii Przemyslowej imienia Profesora Ignacego Moscickiego, 01-793 Warszawa (PL); Uniwersytet Gdanski, 80-309 Gdansk (PL)
(72) Inventor: SACZYNSKA, Violetta, PL-01-494 Warszawa (PL); CECUDA-ADAMCZEWSKA, Violetta, PL-03-477 Warszawa (PL); POREBSKA, Anna, PL-01-473 Warszawa (PL); FLORYS, Katarzyna, PL-02-942 Warszawa (PL); BIERCZYNSKA-KRZYSIK, Anna, PL-02-495 Warszawa (PL); BARAN, Piotr, PL-02-495 Warszawa (PL); ROMANIK-CHRUSCIELEWSKA, Agnieszka, PL-05-420 Józefów (PL); PLUCIENNICZAK, Grazyna, PL-04-360 Warszawa (PL); PLUCIENNICZAK, Andrzej, PL-04-360 Warszawa (PL); BOROWICZ, Piotr, PL-00-655 Warszawa (PL); KUCHARCZYK, Krzysztof, PL-02-495 Warszawa (PL); SZEWCZYK, Boguslaw, PL-80-156 Gdansk (PL)
(74) Representative: Twardowska-Czerwinska, Aleksandra
(86) International application number: PCT/PL2017/000084
(87) International publication number: WO 2018/048317

(56) References cited:
- WO-A1-2009/035420
- WO-A1-2013/105896
- WO-A2-2008/110937
- US-A1- 2015 030 607
- YIXIN CHEN ET AL: "Broad Cross-Protection against H5N1 Avian Influenza Virus Infection by Means of Monoclonal Antibodies that Map to Conserved Viral Epitopes", THE JOURNAL OF INFECTIOUS DISEASES, vol. 199, no. 1, 1 January 2009 (2009-01-01), pages 49-58, XP055023361, ISSN: 0022-1899, DOI: 10.1086/594374
- LINA SUN ET AL: "Generation, Characterization and Epitope Mapping of Two Neutralizing and Protective Human Recombinant Antibodies against Influenza A H5N1 Viruses", PLOS ONE, PUBLIC LIBRARY OF SCIENCE, US, vol. 4, no. 5, 7 May 2009 (2009-05-07), pages E5476-1, XP002685196, ISSN: 1932-6203, DOI: 10.1371/JOURNAL.PONE.0005476
- OH S ET AL: "Neutralizing monoclonal antibodies to different clades of Influenza A H5N1 viruses", JOURNAL OF VIROLOGICAL METHODS, ELSEVIER BV, NL, vol. 157, no. 2, 1 May 2009 (2009-05-01), pages 161-167, XP026064566, ISSN: 0166-0934, DOI: 10.1016/J.JVIROMET.2008.12.016 [retrieved on 2009-01-17]

## Description

The object of the invention are monoclonal antibodies against hemagglutinin of H5-serotype influenza viruses and their broad application in diagnostics and immunotherapy of infections evoked by H5-serotype influenza viruses in humans and animals. The invention also provides hybridomas producing said antibodies, as well as compositions and diagnostic kits containing said antibodies for the detection and typing of H5-serotype influenza viruses and antibodies against H5-serotype influenza viruses in biological samples.

### Background of the invention

### H5-serotype influenza viruses

Influenza viruses (IVs) belong to the *Orthomyxoviridae* family, in which six genera are currently distinguished (ICTV 2014). Among them, *Influenzavirus A* genus is of the highest eidemiological importance. Influenza virus type A is surrounded by a lipid-protein envelope and contains genome in the form of a single-stranded ribonucleic acid (RNA) with negative polarity, which is divided into eight segments and codes for 10 proteins.

Type A IV strains are classified according to the serotype of surface glycoproteins: hemagglutinin (HA) and neuraminidase (NA). Up till now, 18 HA serotypes and 11 NA serotypes (Szewczyk B. et al., 2014) have been identified. The HA and NA proteins play an important role in the virus replication cycle. By binding to sialic acid residues of host cell receptors, HA enables IV internalization by endocytosis, and then mediates the fusion of the viral envelope with cell membrane, which leads to the release of the virus's genetic material into the cytoplasm and initiates the process of its replication (Skehel J.J. and Wiley D.C., 2000). NA catalyses the cleavage of glycoside bonds with sialic acid, which determines the release of the virus from attacked cells and thereby its spreading (Lamb R.A. and Choppin P.W., 1983).

The surface glycoproteins of IVs undergo gradual changes as a result of RNA polymerase errors and selective pressure from the host immune system. This phenomenon, referred to as antigenic drift, leads to the escape of the virus out of immune system control and usually causes limited illnesses, rarely epidemics. Greater and more rapid changes of viral antigens, referred to as antigenic shift, are a result of genetic reassortation and can lead to a pandemic. The variation of IVs makes them a real challenge for influenza diagnostics, prevention and treatment.

In birds, there are influenza viruses (AIVs) being combinations of 16 serotypes of HA and 9 serotypes of NA. There are 2 serotypes of type A IVs predominantly circulating in the human population: H1N1 and H3N2. Influenza viruses are host-specific so that AIVs usually do not infect humans. Among the numerous AI viruses, the low-pathogenic (LP) strains are dominant, causing mild infections. Influenza viruses of the H5 and H7 serotypes, usually non-pathogenic in their natural hosts, which are wild birds, especially aquatic birds, may become highly-pathogenic (HP) after transfer to an infection-prone population of domestic birds and through them may pose a threat to human health and life. This was the case of H5N1 HPAIV strain.

Since the disease outbreak in 1996 at the goose farm and human infections in 1997 in China (Xu X. et al., 1999), the spreading of H5N1 HPAI viruses has been observed (Verhagen J.H. et al. 2015). The emerging disease outbreaks among birds, often reaching an epizootic scale, cause a high animal mortality and force stamping out domestic birds. Fatal cases of human infection with H5N1 viruses are still noted. There is still a risk of reassortation of circulating H5N1 AIVs with mammal viruses, leading to new strains capable of direct transmission between humans.

During circulation and spread of the H5N1 viruses, the HA genes diversified into multiple genetic lineages, referred to as clades (Verhagen J.H. et al., 2015). From 2009 onward, the emergence of reassortant H5-serotype HPAIVs: H5N2, H5N5, H5N6 and H5N8, has been noted. After their identification in 2014, the H5N8 HPAI wiruses are observed to spread rapidly in a domestic bird population. As a result of reassortation with H5N8 HPAIV, the novel H5N2 HPAI virus emerged and was identified in disease outbreaks at the chicken and turkey farms in Canada at the end of 2014 (Ip H.S. et al., 2015). In 2015, in the USA, the H5N2 HPAIV caused a high poultry mortality (Hvistendahl M., 2015).

### H5 hemagglutinin - the target antigen for monoclonal antibodies

HA is synthesized as a single polypeptide chain, referred to as the precursor HA - HA0, containing a signal sequence, the HA1 and HA2 subunits separated by the cleavage site for proteolytic enzymes. Cleavage of HA0 is necessary to HA activation, which is prerequisite for IV infectivity, and the protein susceptibility to digestion by proteolytic enzymes is one of the factors determining the virus pathogenicity (Steinhauer D.A., 1999). In the H5 HA proteins, the cleavage site contains multiple basic amino acids, which makes the protein susceptible to digestion by proteases active in most tissues, and the infection with H5- serotype IVs has a systemic nature.

The studies on the structure of HA proteins of various serotypes, including the H5 serotype, showed that antigenically-different hemagglutinins are structurally similar and show the same sub-domain organization (Ha Y. et al., 2002, Wilson I.A. Et al., 1981). The protein is composed of a globular domain formed by the HA1 subunit and the stem domain formed largely by the HA2 subunit and amino acid sequences from the N- and C-termini of the HA1 subunit. The globular domain of HA contains receptor binding subdomain (RBD), through which the virus binds to the host cell, whereas the fusion of viral and endosomal membranes is mediated by the stem domain. The HA2 subunit within the stem domain is responsible for the HA trimer formation and stabilization, as well as its anchorage in the lipid envelope of the virus, where the protein forms characteristic structures.

HA is the most variable IV antigen. This applies, in particular, to the globular domain. The mutation-susceptible protein domain contains epitopes for neutralizing antibodies usually having a limited range of intra-serotype specificities. In the case of H5N1 HPAIVs, a weak cross-reactivity of these antibodies against antigenically distant viruses from different clades is observed. Antibodies directed against the globular domain neutralize infectivity of the viruses mainly by interfering with their binding to the host cell receptors. Activity of neutralizing antibodies, which bind to RBD, is observed *in vitro* as the inhibition of IV hemagglutination activity.

In contrary to the globular domain-forming HA1 subunit, the HA2 subunit, which forms an essential part of the stem region, is relatively well conserved and contains epitopes for neutralizing antibodies with broad-range specificities against viruses of different serotypes. Antibodies directed against the stem domain neutralize virus infectivity by blocking the membrane fusion.

### The use of monoclonal antibodies in immunoprophylaxis and immunotherapy

Currently, the control of infections evoked by HPAIVs relies on the use of antiviral drugs and active or passive immunizations. Immunoprophylaxis and immunotherapy are preferred due to the development of drug resistance observed among IVs and unfavourable side effects of pharmacotherapy. Preventive actions consist mainly in preventive vaccinations. Traditional vaccine production methods are long-lasting and would not be able to generate sufficient number of doses in the case of pandemic threat. Immunization with the use of these vaccines is often accompanied by undesirable side effects. An alternative for conventional vaccines are DNA vaccines and subunit vaccines based on recombinant HA proteins. The H5 HA proteins are produced mainly in eukaryotic cells, but also in bacteria cells. The main challenge of research on the obtaining subunit vaccines against influenza is the production of antigen having the native-HA characteristics. Of particular importance to the vaccine HA quality are the structure accuracy of the HA1 subunit, where conformational epitopes for neutralizing antibodies are localized, and the protein oligomerization.

Anti-HA monoclonal antibodies (mAbs) can be used during the development of influenza vaccines and at various stages of their production and implementation. The first indicator of the HA protein usefulness for vaccine production are the results of antigenicity studies with well-characterized antibodies (Chiu F.F. et al., 2009). Particularly useful in this regard are mAbs recognizing conformational, neutralizing epitopes of HA. MAbs can be also used in the studies on the oligomerization level of HA proteins, beside the erythrocyte agglutination and fetuin binding tests. Using mAbs in affinity chromatography or immunoprecipitation, one can also isolate and purify the H5 HA proteins. Antigens isolated and purified in this way can be used as components of prototype vaccines against H5-serotype IVs, intended for a preliminary evaluation of the vaccine ability to evoke protective immune responses in animals. MAbs can be also used in the stability control of vaccine H5 HA, storaged separately or in immunogenic compositions. The most important application of serotype-specific mAbs related to the active immunization is their use in the so-called DIVA tests (*Differentiation of Infected from Vaccinated Animals*)*.* Providing possibility to differentiate infected from vaccinated animals is prerequisite for vaccinations against avian influenza (Suarez D.L., 2005).

Another way of preventing IV infections, but mostly of influenza treatment, is the use of mAbs for passive immunization. These are mainly humanized antibodies or, preferably, antibodies produced *de novo,* as human mAbs. Predictably, neutralizing antibodies directed against the variable and mutation-prone HA1 subunit of H5 hemagglutinin may not be effective against heterologous or antigen-drifted virus strains. In this context, the obtaining the neutralizing mAbs against epitopes of the HA1 subunit of HA, which are conserved among the H5N1 IVs, is of great importance (Cao Z. et al., 2012, Du L. et al., 2013, Oh H.L. Et al., 2010, Wu R. et al., 2014).
In the recent years, there are increasing reports on the generation of mAbs recognizing conserved epitopes in the HA2 subunit of the HA stem region, which show hetero-serotypic neutralizing activity (Corti D. et al., 2011, Ekiert D.C. Et al., 2009, Okuno Y. et al., 1993, Sui J. et al., 2009).

### Prior Art

### Disadvantages of so far applied diagnostic methods using antibodies

Research on the evolution, spreading and occurrence of the novel HPAI virus strains of the H5 serotype require using the specific and reliable methods for early detection, IV typing and strain identification (Petric M. et al. 2006). There is also a demand for the tests to recognize infections evoked by H5-serotype AIVs in humans, which could be also used in the monitoring of viral infection development and/or the effectiveness evaluation of the applied treatment regimen. Objective difficulty, and simultaneously a challenge for diagnostics, is variability of IVs. Virus susceptibility to mutations should be taken into account already at the stages of reagent preparation and diagnostic test development.

In influenza diagnostics, various conventional methods and techniques are used. Commonly accepted method is reverse transcription polymerase chain reaction (RT-PCR). The PCR-based diagnostics requires RNA extraction, the use of specialist equipment, can be carried out by qualified personnel only and is expensive. In the recognition of IV infection, the following serological tests are also used: agar gel immunodiffusion (AGID), hemagglutination inhibition (HI) and microneutralization (MN) tests.

The AGID test is the reference screening assay for the detection of antibodies against IV in sera of domestic and wild animals. The results of this test are not reliable in the case of some animal groups, such as aquatic birds, which do not produce precipitating antibodies. Moreover, the test is prone to misinterpretation because of subjectivity of result reading.

The HI test, recommended as the reference test in animal serodiagnostics, is the so-called "gold standard" in serotyping of antibodies against IVs. An important limitation of the test is that it shows maximum sensitivity in the case of using the homologous IVs, and the use of antigenically distant viral strain may lead to the false-negative results. The HI test requires using erythrocyte preparation, which is obtained each time from SPF animal blood. Additionally, when using non-chicken erythrocytes, it is necessary to use reagents removing the inhibitors of non-specific hemagglutination inhibition from serum. The HI test is not optimal in the case of analysis of a large number of serum samples, as it cannot be automated, and therefore is time-consuming and labor-intensive. Hemagglutination inhibition is evaluated visually, thereby the test result is subjective.

The MN test, recommended in the case of human infections with H5N1 HPAIVs, cannot be used in routine diagnostics. It requires using the live viruses, therefore it is carried out in laboratories of the appropriate level of safety by qualified personnel. In conclusion, the MN test and other conventional diagnostics methods have substantial limitations.

Early influenza diagnostics uses various immunological methods and techniques to detect influenza viruses. Among them, there are the tests based on the ELISA (*enzyme-linked immunosorbent assay*) and immunochromatography methods and the tests using immunosensors. These types of tests are easy to perform and enable examination of a large number of samples in a relatively short time.

Yang M. et al. (2009) developed the test based on the sandwich ELISA method with the use of 2 different antibody clones. One of them was the capture antibody, and the other the detection antibody. In comparison to the tests using mAbs and polyclonal antibodies (pAbs), the tests using 2 mAb clones are usually more specific and easier for standardization. The antibodies, generated by the hybridoma method with the use of H5N1 IV as the immunogen, recognized conformational epitopes of HA, conserved among the H5-serotype viruses. Antibodies had no HI activity. The test did not show cross-reactions with the tested IVs of serotypes other than H5, however, one of the tested H5N2 IV strains was detected with significantly reduced sensitivity.

Miyagawa E. et al. (2011), using the hybridoma method and the H5N1 IV as the immunogen, obtained 3 antibody clones, which recognized different conformational epitopes in the region of the HA1 subunit of HA, identified as less prone to mutations. The antibodies showed no neutralizing activity. Three variants of immunochromatographic test were developed using all of the obtained clones. The tests specifically detected the H5N1 and H5N3 IV strains. No reactivities with IV type A of serotypes other than H5 or with IV type B were found. However, none of the test variants detected the examined H5N2 IV strain.

Electrochemical biosensors for IV detection are a subject of the recently published review article (Grabowska I. et al., 2014). Jarocka U. et al. (2014) developed the immunosensor for the detection of peptides derived from the viral HA using the monoclonal antibody Fab' fragment. The Immunosensor detected recombinant hemagglutinin proteins based on the HA1 subunit and ectodomain of HAs with sequences from the H5N1 HPAIVs. However, the differences in the interactions were observed that depended on the length and the sequence of HA protein.

In addition to the influenza diagnostics based on the virus detection, the immunological methods and techniques are used for the detection of specific antibodies. These methods are of special importance for the assessment of a potential contact of animals and humans with IVs. Among the tests for antibody detection, the indirect ELISA (iELISA) and ELISAs in the blocking (bELISA) or the competitive (cELISA) formats are applied.

The iELISAs tests are designed for testing of humans or specific animal species. They require using highly pure antigen and species-specific secondary antibodies, which are not always available. The major disadvantage of iELISAs is their low specificity against the tested IV serotype. This is a result of cross-reactions of antibodies induced by infections with various serotype viruses. In the case of adults, the specificity of an exemplary iELISA for the detection of antibodies against H5N1 AIVs was only 62%, with the sensitivity level of 80% (Rowe T. et al., 1999).

In the bELISA/cELISA tests, the key reagents are mAbs or their functional equivalents. In contrary to iELISAs, the bELISA/cELISA tests can be used to detect antigen-specific antibodies in sera of various origins. In influenza diagnostics, commonly used are the bELISA/cELISA tests based on the use of mAbs against nucleoprotein (NP), which is highly conserved among the type A IVs. The tests for the detection of antibodies against NP, used as an alternative for AGID tests, have a screening nature. Unlike them, the bELIA/cELISA tests using the serotype-specific mAbs against HA enable the identification of infectious virus serotype and, consequently, the differentiation between infections evoked by HPIVs and LPIVs.

Yang M. et al. (2009), using the hybridoma method and the H5N1 IV as the immunogen, obtained a clone of antibodies, which recognized conformational epitope in the HA1 subunit of HA and had no HI activity. Using the obtained mAb, the cELISA was developed and its efficacy was evaluated by analyses of chicken sera. No cross-reactions with antisera against IVs of serotypes other than H5 were found. The test detected antibodies against the H5-serotype IVs, but the test evaluation was performed using a narrow range of anti-H5 positive sera.

Prabakaran M. et al. (2009), using the hybridoma method and recombinant HA protein from a bacterial expression system as the immunogen, obtained a clone of antibodies recognizing a linear epitope in the HA1 subunit of HA. The epitope sequence for the obtained mAb was identified. The epitope turned out to be strongly conserved among the H5N1 IV isolates from humans (100%) and birds (96.9%), and weakly conserved in the HAs of the H5-serotype viruses having the NA serotypes other than N1 (54.3%). The antibodies showed no neutralizing activity. Using the obtained mAbs, the bELISA was developed and its efficacy was evaluated by analyses of chicken and human sera. No cross-reactions with antisera against IVs of serotypes other than H5 were found. The test detected antibodies against HAs of various-origin H5N1 viruses, but it did not provide detection of H5-serotype IVs other than H5N1.

Dlugolenski D. et al. (2010), using the hybridoma method and the H5 HA protein from a mammalian expression system as the immunogen, obtained a clone of antibodies reactive with H5N1, H5N2 and H5N3 LPIVs and showing no HI activity. Using the obtained mAb, the cELISA was developed and the analyses of chicken, turkey and duck sera were carried out. No cross-reactions with antisera against IVs of serotypes other than H5 were found. Antibodies against the H5 HA were detected in different animal species with various specificity and sensitivity. The levels of diagnostic specificity and sensitivity of the test, determined for the chicken sera, were low and were 63% and 66%, respectively.

For the construction of the bELISA test, Postel A. et al. (2011) used mAb (ClonDiag) recognizing a linear epitope in the HA2 subunit of the H5 HA stem region, strongly conserved among the different IV strains, including the HP and LP IVs, of the H5 serotype: H5N1, H5N2, H5N3, H5N6, H5N9. Research carried out with sera from different groups of birds showed high levels of diagnostic specificity (91.5%) and sensitivity (98.1%) of the test and its capability to detect antibodies induced in the chickens by the antigen-drifted variant of H5N1 HPAIV. However, a significant cross-reactivity of antisera specific against the H2 serotype and, to a less extent, of antisera specific against the H1 and H6 serotypes was observed in the test. Predictably, the test could detect the novel IV variants of the H5 serotype, while not providing a clear distinction between antisera against the H5 HAs and HAs of the H2, H1 and H6 serotypes.

Lebarbenchon C. et al. (2013) evaluated the usefulness of the FLUAc H5 test (IDVet) for the detection of antibodies against H5 HA in aquatic birds. It was found that the test could provide a valuable data regarding the contact of the analyzed group of birds with H5-serotype IVs following introduction of modifications into manufacturer's protocol. Using the FLUAc H5 test (IDVet), Postel A. et al. (2011) analyzed serum samples of chickens immunized with antigenically distant H5N1 HPAIV. A large number of false-negative results (7/9 of analyzed samples) were obtained. This indicates the limited ability of the test to detect the novel H5N1 HPAIV strains.

Stelzer-Braid S. et al. (2008) evaluated the diagnostic value of the ELISA H5-HA antibody kit (Dialab), with declared by manufacturer 100% specificity and 98% sensitivity. It was shown that the test is reliable when patients' sera contained high levels of antibodies against H5 HA. It was also shown that antibodies against seasonal H3N2 and H1N1 IVs may cross-react with H5 HA antigen, providing positive results in the test being evaluated.

WO2009/035420 discloses monoclonal antibodies and related binding proteins that bind specifically to the envelope glycoprotein of H5 subtypes or neuraminidase glycoprotein of N1 subtypes of avian influenza virus (AIV). The monoclonal antibodies and related binding proteins are useful for the detection of H5 and N1 subtypes of AIV, including H5N1 subtypes and provide means for the diagnosis, surveillance and treatment of dangerous viral infections. In particular, a binding protein that binds specifically to a conformational epitope of the hemagglutinin envelope glycoprotein of an H5 subtype of avian influenza virus that has substantially the immunological binding characteristics of monoclonal antibody 5C5, 2D9, 4F8, ICI, 3B6, 2F11, 9CI or 3H11 is disclosed.

WO2013/105896 relates to murine monoclonal antibodies C, F and H which target major neutralizing epitopes of influenza H5 hemagglutinin of clade 2.3 and active fragments thereof. The present invention also relates to methods and compositions for the prophylaxis and treatment of H5N1 influenza using murine monoclonal antibodies C, F or H or active fragments thereof. The publication discloses a monoclonal antibody or antibody fragment which specifically binds to a conformational epitope of H5 hemagglutinin, wherein the conformational epitope is selected from the group consisting of: (a) a conformational epitope comprised of amino acid 152Lys, 184Ala and 190Pro of the mature HA protein; (b) a conformational epitope comprised of amino acid 152Lys and 221Gly of the mature HA protein; and (c) a conformational epitope comprised of amino acid 141 Pro and 152Lys of the mature HA protein.

US2015/030607 discloses neutralizing antibodies with cross-neutralizing activity and cross-protective effects against divergent strains of influenza virus, which are specific for an epitope having at least 90% homology to amino acids +72-115 of the HA1 domain of H5N1 influenza virus hemagglutinin. The document provides a neutralizing antibody specifically binding an epitope of the HA1 domain of influenza virus H5N1 hemagglutinin, the epitope having an amino acid sequence of SEQ ID NO:15, and wherein the antibody competes for specific binding to SEQ ID NO:15 with a murine monoclonal antibody secreted by hybridoma HA-3 (ATCC accession number PTA-12174) or hybridoma HA-7 (ATCC accession number PTA-12173).

Yixin Chen et al. "Broad cross-protection against H5N1 Avian Influenza Virus infection by means of monoclonal antibodies that map to conserved viral epitopes" The Journal of Infectious Diseases , vol. 199, no. 1, 1 Jan 2009, pages 49-58 describe cross-protection against antigenically diverse H5N1 strains with H5-specific MAbs, generated by successive immunization of antigenically distinct strains, that was evaluated in mice. Authors summarize that generation of broadly cross-protective MAbs against H5N1 influenza virus may be optimized by selecting MAbs that target conserved sites in hemagglutinin. H5 MAbs such as 13D4 may prove to have therapeutic value in controlling infection due to current and future H5N1 variants.

Lina Sun et al. "Generation, characterization and epitope maping of two neutralizing and protective human recombinant antibodies against influenza A H5N1 viruses" Plos One Library of Science, US, vol. 4, no. 5, 7 May 2009, pages E5476-1 have characterized two recombinant baculovirus-expressed human antibodies (rhAbs), AVFIulgG01 and AVFIulgG03, generated by screening a Fab antibody phage library derived from a patient recovered from infection with a highly pathogenic avian influenza A H5N1 clade 2.3 virus. AVFIuIgG01 cross-neutralized the most of clade 0, clade 1, and clade 2 viruses tested, in contrast, AVFIulgG03 only neutralized clade 2 viruses. Passive immunization of mice with either AVFIuIgG01 or AVFIuIgG03 antibody resulted in protection from a lethal H5N1 clade 2.3 virus infection. Furthermore, through epitope mapping, two distinct epitopes on H5 HA molecule recognized by these rhAbs were identified and the potential of rhAbs to protect against a lethal H5N1 virus infection was demonstrated in a mouse model.

Oh S et al. "Neutralizing monoclonal antibodies to different clades of influenza A H5N1 viruses", Journal of Virological Methods, Elsevier BV, NL, vol. 157, no. 2, 1 May 2009, pages 161-167 describe four IgG-1 kappa monoclonal antibodies (mAbs) against Influenza A/Chicken/Vietnam/8/2004 (H5N1) virus. Three of these showed neutralizing activities against H5N1 strains from clades 1, 2 and 3 using a retroviral pseudotype or live virus microneutralization assay. All four mAbs reacted specifically to the immunogen by immunohistochemical staining and to A/Hong Kong/483/1997 (H5N1) and A/Thailand/1(KAN-1)/2004 (H5N1)-infected MDCK cells by immunofluorescence. ELISA titrations of the mAbs showed specificity for H5N1 haemagglutinin (HA) and no cross-reactivity to 15 other Influenza A subtypes. Only mAbs 1C1 and the non-neutralizing 1F7 reacted with HA1, the cleaved subunit of HA, by Western blot.

Human antibodies that can neutralize a H5N1 strain of influenza A virus are provided in WO2008/110937. The publication discloses antibodies that can neutralize a strain of influenza A virus in clade 2 of the H5 subtype, that can neutralize a H5N1 strain of influenza A virus and have a lambda light chain, and that are IgG antibodies (but not with a IgGI heavy chain) that can neutralize a H5N1 strain of influenza A virus.

### The aim of the invention

Considering the threat to health and life of animals and humans, the H5-serotype HPAIVs are subject of epidemiological surveillance and various prophylactic and therapeutic strategies are developed against infections evoked by these viruses. Preventive activities are focused on the high virus pathogenicity-determining H5 HA, which, at the same time, is the main target for antibodies neutralizing the virus infectivity. Monoclonal antibodies and antigen-binding proteins derived from them play an important role in these activities as efficient diagnostic and therapeutic agents. Antigen variability makes influenza viruses a real challenge for influenza diagnostics, prevention and treatment.

The above presented solutions in the art indicate that independently from the method and technique of immunological test, the basic condition of correct diagnosis is the use of mAbs or their functional equivalents, which are specific against HA of the H5 serotype and do not cross-react with HAs of other serotypes. Only a broad range of antibody specificities against the H5 HAs can provide serotype identification of various-origin infectious strains. It is desirable for mAbs to recognize the native forms of H5 HA antigens. Wang S.F. et al. (2009) obtained clones of antibodies reacting with denatured H5 HA protein only. The usefulness of such mAbs is limited to a narrow range of immunological methods.

Epitopes for diagnostically valuable mAbs should be localized in the HA1 subunit, which determines the virus serotype. Diagnostic application of mAbs against the HA1 subunit of H5 hemagglutinin, which are active in the HI or MN tests, becomes problematic when epitopes for these antibodies are not conserved. Such mAbs and tests using them may lose the ability to detect infections evoked by H5-serotype IVs in the case of mutation of epitopes for these antibodies under conditions of immunological pressure. This, in turn, will cause that the test will not detect the novel, mutated virus strains. On the other hand, in the tests using mAbs recognizing conserved epitopes in the HA stem region, some level of inter-serotype reactivity is inevitable (Postel A. et al., 2011). Therefore, optimally for the diagnostic effect is to use mAbs or their functional equivalents specific against epitopes in the HA1 subunit of HA, which are conserved among the H5-serotype viruses. Equally important is the availability of different clones of mAbs with desired properties so to ensure the detection of currently circulating and the novel IVs of H5 serotype.

Thus, the aim of the invention is to provide tools for effective immunoprophylaxis and immunotherapy of infections evoked by influenza viruses meeting the above criteria. The aim of the invention is to provide a set of antibody clones, which will meet the above requirements for mAbs of high diagnostic value. Unexpectedly, this aim was realized in the present invention and a solution for problems existing in the art was obtained.

### Disclosure of the Invention

The object of the invention is a composition of monoclonal antibodies against hemagglutinin of H5-serotype influenza viruses lacking hemagglutination inhibiting (HI) activity that bind to epitopes of native forms of H5 antigens, and exhibit no cross-reactivity with non-H5 serotype viruses, comprising:
(i) G-1-31-22 produced by mouse hybridoma cell line G-1-31-22 deposited with the DSMZ under the number DSM ACC3292,
(ii) G-2-14-10 produced by mouse hybridoma cell line G-2-14-10 deposited with the DSMZ under the number DSM ACC3293,
(iii) G-5-32-5 produced by mouse hybridoma cell line G-5-32-5 deposited with the DSMZ under the number DSM ACC3294,
(iv) G-6-42-42 produced by mouse hybridoma cell line G-6-42-42 deposited with the DSMZ under the number DSM ACC3295,
(v) G-7-24-17 produced by mouse hybridoma cell line G-7-24-17 deposited with the DSMZ under the number DSM ACC3296, and
(vi) G-7-27-18 produced by mouse hybridoma cell line G-7-27-18 deposited with the DSMZ under the number DSM ACC3297;
or wherein the composition comprises fragments of each of said antibodies wherein said fragments are Fab, Fab', F(ab')2 , Fv or single-chain variable fragments (scFv) consisting of VL and VH domains bound to each other by a peptide linker.

Preferably, hemagglutinin is from influenza viruses of the H5 serotype, including H5N1, H5N2, H5N3 and H5N9.

Preferably, a monoclonal antibody is conjugated with an analytically detectable label, prodrug, drug, therapeutic agent, peptide, protein, enzyme, virus, lipid, PEG.

The other subjects of the invention are mouse hybridoma cell lines selected from the group comprising:
(i) G-1-31-22 deposited with the DSMZ under the number DSM ACC3292,
(ii) G-2-14-10 deposited with the DSMZ under the number DSM ACC3293,
(iii) G-5-32-5 deposited with the DSMZ under the number DSM ACC3294,
(iv) G-6-42-42 deposited with the DSMZ under the number DSM ACC3295,
(v) G-7-24-17 deposited with the DSMZ under the number DSM ACC3296 and
(vi) G-7-27-18 deposited with the DSMZ under the number DSM ACC3297.

Another subject of the invention is a pharmaceutical or diagnostic composition comprising a composition of antibodies defined above and a suitable carrier or label.

Another subject of the invention is a composition as defined above for use in treatment of infections evoked by influenza viruses of the H5 serotype.

Another subject of the invention is a method for in vitro diagnosing infections with influenza viruses of the H5 serotype, comprising contacting a biological sample with a composition of monoclonal antibodies as defined above and detecting the binding of said antibodies with H5-serotype influenza viruses or H5 hemagglutinin protein.

Another subject of the invention is a diagnostic kit suitable for in vitro detection of infections with H5-serotype influenza viruses, containing the composition of monoclonal antibodies as defined above or their fragments as defined above or a composition of conjugates of monoclonal antibodies as defined above, and reagents for the detection of antibodies or their functional fragments bound with H5-serotype influenza viruses or H5 hemagglutinin protein.

Another subject of the invention is a diagnostic kit suitable for in vitro detection, quantification or semi-quantification of H5-serotype influenza viruses in biological samples, comprising the composition of monoclonal antibodies or their conjugates as defined above and tools and reagents commonly used in the immunological tests.

Another subject of the invention is a diagnostic kit suitable for in vitro detection, quantification or semi-quantification of antibodies against H5-serotype influenza viruses in biological samples, comprising the composition of monoclonal antibodies or their conjugates as defined above and tools and reagents commonly used in the immunological tests.

Preferably, the antibodies in the kits are in the non-isolated form or are isolated and purified.

Preferably, kits are used in the tests selected from the group comprising: immunoenzymatic, preferably bELISA H5, immunofluorescent, immunochemiluminescent, radioimmunological, immunochromatographic, immunodiffusion, immunoprecipitation tests, a test using immunosensors that can be used as so-called DIVA tests for the differentiation of infected from vaccinated animals. Preferably, above kits allow the differentiation of infected from vaccinated animals also known as DIVA (Differentiation of Infected from Vaccinated Animals) test.

The invention provides a composition of new monoclonal antibodies against hemagglutinin of H5-serotype influenza viruses: G-1-31-22, G-2-14-10, G-5-32-5, G-6-42-42, G-7-24-17 and G-7-27-18. The antibodies are designated analogically to the mouse hybridoma cell lines producing them. According to that, the antibodies: G-1-31-22, G-2-14-10, G-5-32-5, G-6-42-42, G-7-24-17 and G-7-27-18 are produced by the mouse hybridoma cell lines: G-1-31-22, G-2-14-10, G-5-32-5, G-6-42-42, G-7-24-17 and G-7-27-18, respectively. All of the obtained clones are the mouse antibodies of the IgG1 isotype. However, it does not limit the isotype of antibodies being the object of the invention. The isotype of the antibodies can be changed with the use of genetic engineering techniques known in the art. The G-1-31-22, G-2- 14-10, G-5-32-5, G-6-42-42, G-7-24-17 and G-7-27-18 mAbs specifically bind to the H5-serotype IVs and H5-serotype HA proteins.

The specificity of mAbs was examined with the use of the H5 HA antigens with varied properties. Among them, there were the HA ectodomain-based recombinant proteins (rHA) from mammalian, baculovirus and bacterial expression systems, the proteins based on the HA1 subunit of HA (rHA1), obtained in mammalian cells and the AIVs of the H5 serotype. Most of the H5 HA antigens (13/14) showed native-protein characteristics. Conformational antigens contained the HA sequences from twelve AIV strains of the H5 serotype, wherein some of them were isolated from infected humans. This amount includes the H5N3 (1 strain), H5N9 (1 strain), H5N2 (2 strains) viruses and the H5N1 viruses (8 strains), classified into five clades: 0, 1, 2.2, 4 and EA-nonGsGD.

The obtained clones of mAbs: G-1-31-22, G-2-14-10, G-5-32-5, G-6-42-42, G-7-24-17 and G-7-27-18 recognized all of the conformational, glycosylated H5 HA antigens: the rHA (6/6) and rHA1 (3/3) proteins from an eukaryotic expression system and AIVs of the H5 serotype (4/4). Under the testing conditions, for some clones (4/7), significant reactivity towards the non-glycosylated bacterial H5 HA protein was also shown. None of the obtained mAb clones bound to the non-conformational rHA protein. The produced mAbs bound to the H5 HA antigens both before and after purification from the culture medium.

The G-1-31-22, G-2-14-10, G-5-32-5, G-6-42-42, G-7-24-17 and G-7-27-18 mAbs recognized and bound to the conformational epitopes in the HA1 subunit of the H5 HA antigens with diverse amino acid sequences of the HA1 subunit. The homology, measured by the amino acid identity between the HA1 subunit of conformational HA antigens and the HA1 subunit of the immunogen, used in the mAb production procedure, was from 99% to 88%. The particularly broad range of specificities of the obtained antibodies indicates that the epitopes for the obtained antibody clones are highly conserved among the H5-serotype viruses.

The analysis of immunoreactivity profiles and peptide maps of Fab antibody fragments consistently showed that the G-1-31-22, G-2-14-10, G-5-32-5, G-6-42-42, G-7-24-17 and G-7-27-18 mAbs are the distinct clones. The obtained mAbs recognize different epitopes specific for the H5 serotype of hemagglutinin, therefore they can bind to one molecule of the H5 HA protein's monomer.

The G-1-31-22, G-2-14-10, G-5-32-5, G-6-42-42, G-7-24-17 and G-7-27-18 mAbs do not have the ability to inhibit hemagglutination activity of the H5-serotype IVs, especially of the H5N2 and H5N3 AIVs. Therefore, the epitopes for the antibodies of the invention are not essential for the binding of IVs to the host cell receptors, and thereby they do not undergo mutations under the HI antibodies, which are known to inhibit viral infection. Predictably, the ability of the obtained mAbs to recognize H5-serotype IVs can be preserved even in the case of HA antigen change under conditions of immunological pressure. The confirmation of the invariability of epitopes recognized by antibodies of the invention is the broad range of their specificities against the H5 HA antigens.

Negative selection of the obtained antibody clones was performed using twenty-one AIV strains representing HA serotypes other than H5. None of the antibody clones from the group comprising the G-1-31-22, G-2-14-10, G-5-32-5, G-6-42-42, G-7-24-17 and G-7-27-18 mAbs bound to the IVs of the H1-H4 and H6-H16 serotypes.

In summary, among the six of the obtained mAb clones: G-1-31-22, G-2-14-10, G-5-32-5, G-6-42-42, G-7-24-17 and G-7-27-18, each recognizes a distinct conformational epitope in the HA1 subunit of HA, shows a broad-range specificity against the H5-serotype HAs and at the same time, does not cross-react with HAs of the H1-H4 and H6-H16 serotypes. Preferably, the epitopes for these antibodies are probably highly conserved among the IVs of the H5 serotype and do not undergo changes under HI antibodies inhibiting the viral infection.

The H5 HA antigen-binding molecules can take various forms. The binding molecules of the invention are the G-1-31-22, G-2-14-10, G-5-32-5, G-6-42-42, G-7-24-17 and G-7-27-18 mAbs, which are the intact mouse immunoglobulin molecules of the IgG1 isotype. They contain the constant fragments of the crystallizable region (Fc) and the antigen-binding regions (Fab). Such complete forms of the H5 HA-binding molecules are composed of two identical light chains (L) and two identical heavy chains (H), each of which contains the constant and variable (V_{L,}, V_{H}) domains. Localized within the variable parts of the heavy and light chains, the complementarity-determining regions (CDRs) are involved in specific epitope recognition and antigen-antibody complex formation.

The object of the invention are also fragments, and immunoconjugates of the G-1-31-22, G-2-14-10, G-5-32-5, G-6-42-42, G-7-24-17 and G-7-27-18 mAbs, further disclosed are also variants of the mAbs, which preserve the ability to recognize and bind to the antigen of the original antibody clones. Functional equivalence of different forms of the binding molecules is expressed in that they compete with the intact immunoglobulins for the binding site in the H5 HA antigens.

The H5 HA-binding antibody fragments include, but are not limited to: Fab, Fab', F(ab')2, Fv, scFv, V_{H}, V_{L}. The antibody fragments of the invention can be also multivalent or multispecific structures formed by antibody fragments, e.g., above-mentioned. Preferably, the H5 HA-binding fragments can be the single-chain variable fragments (scFv), consisting of the V_{L} and V_{H} domains bound to each other by a peptide linker. The scFv molecules can be part of, *inter alia,* multimers or minibodies having the desired valency level and specificity range. Functional antibody fragments are preferred particularly when they can be produced with higher yield, especially with the use of genetic engineering methods and/or when their use in some applications is more advantageous than the intact antibody molecules.

Functional mAb variants and their fragments can be obtained by modifications of the starting binding molecules, relying on the sequence change and/or derivative generation. This kind of changes, introduced intentionally, aim to improve properties of the H5 HA antigen-binding molecules, such as affinity or avidity.

Each form of the H5 HA antigen-binding molecule can be a part of immunoconjugate, which comprises at least one label facilitating its purification or detection using the known measurement techniques. Immunoconjugates comprising an analytically detectable label can be used in the diagnostic tests.

The G-1-31-22, G-2-14-10, G-5-32-5, G-6-42-42, G-7-24-17 and G-7-27-18 mAbs were obtained with the use of conventional hybridoma technology. The present invention is related to the mouse hybridoma cell lines producing mAbs of the invention, designated as G-1-31-22, G-2-14-10, G-5-32-5, G-6-42-42, G-7-24-17 and G-7-27-18. Hybridomas secreting antibodies can be grown on a large-scale *in vitro* using suitable media or *in vivo* in the mouse peritoneal cavity. The mAbs secreted by hybridomas can be used without purification, but preferably after removing components of the culture medium or ascitic fluid that may interfere or prevent their use in some applications. If needed, mAbs can be purified with the use of various chromatographic methods, such as HPLC or Protein A or G affinity chromatography. Methods for antibody purification, including mAbs, are well known in the art.

In one embodiment, mAb clones of the invention were grown *in vitro* using the RPMI-1640 medium (Sigma-Aldrich) with addition of FBS, glutamine, sodium pyruvate and antibiotics. Antibodies were purified from the hybridoma culture supernatants by affinity chromatography with the use of HiTrap Protein G HP (GE Healthcare).

Antibody production is not the only application of the G-1-31-22, G-2-14-10, G-5-32-5, G-6-42-42, G-6-42-71, G-7-24-17 and G-7-27-18 hybridomas. The hybridomas of the invention are also a source of mRNA for synthesis of cDNA molecules encoding the obtained antibodies or their fragments, which can be then cloned and used for the expression of the H5 HA antigen-binding molecules in heterologous systems. For the preparation of DNA sequences encoding antibodies, their sequence variants or fragments, the standard molecular biology techniques can be used.

Production of recombinant proteins in host cells, such as bacteria (e.g., E. *coli*)*,* yeast, cells of higher plants and animals, can be carried out according to methods well known in the art. Bacterial and other microbiological systems can be used for the expression of fragments, such as Fab, F(ab')2, and especially Fv and scFv. Eukaryotic systems, e.g., mammalian, can be used for the production of the larger H5 HA-binding molecules, including the whole antibody molecules. The production procedure of the binding molecules of the invention by recombinant DNA methods would generally include: the culturing recombinant host cells under conditions providing a high expression level, the isolation of a product from cell extract or, preferably, from culture medium and its purification.

Functional antibody fragments can be also produced *in vitro* by proteolytic digestion of intact immunoglobulins in the absence or presence of a reducing agent, according to protocols known in the art. In one embodiment, the Fab fragments of the G-1-31-22, G-2-14-10, G-5-32-5, G-6-42-42, G-7-24-17 and G-7-27-18 antibodies were obtained by ficin digestion of immunoglobulins using the commercial "Pierce_{TM} Mouse IgG₁ Fab and F(ab')₂ Micro Preparation Kit" (Pierce/Thermo Scientific).

Immunoconjugates can be produced by chemical conjugation of antigen-binding molecules with labels. Techniques for conjugation of labels with antigen-binding molecules are well known in the art. Alternatively, immunoconjugates can be produced as fusion proteins containing the binding molecules of the invention and a suitable label.

The H5 HA antigen-binding molecules have a special application in the methods for diagnosis of infection evoked by H5-serotype IVs in animals and humans being the useful tools in diagnostic tests. In patients with identified infection, the diagnostic tests containing the binding molecules of the invention can be also used for the monitoring of the virus infection development and/or the efficacy evaluation of the applied treatment regimen.

Diagnostics of IV infections based on immunological methods consists in the detection of viral particles or antibodies against the virus antigens. According to that, two types of immunological tests are used in influenza diagnostics. The H5 HA antigen-binding molecules can be used in both types of diagnostic tests. In view of the broad-range specificity against the H5 HAs, the use of the binding molecules of the invention in diagnostics can provide identification of the infectious strain. The test for the detection of antibodies against H5 HA using the binding molecules of the invention can be also used as a so-called DIVA test intended to differentiate the infected from vaccinated animals.

The binding molecules of the invention can be used in the immunological methods, carried out *in vivo, in situ* or *in vitro,* that rely on the localization and/or quantification or semi-quantification of the H5-serotype IVs in the organism, tissues or cells, or in the suitable biological samples. In another embodiment of the invention, the composition of H5 HA antigen-binding molecules can be used in the methods for the detection, quantification or semi-quantification of antibodies against IVs, especially of the H5 serotype, in biological samples, such as blood serum or plasma. In these methods, mAbs or their functional equivalents compete with antibodies present in the sample for the binding site on the antigen. The H5 HA antigen-binding molecules of the invention can be used in the immunological tests in various ways. For example, they can be used in an unisolated form or following isolation and purification. To obtain the desired diagnostic effect, the H5 HA antigen-binding molecules recognizing the specified epitope of the antigen can be used together with the molecule or molecules recognizing other epitopes of the antigen. It is further disclosed that they can be used separately. The H5 HA antigen-binding molecules can bind to the antigen in a solution or a suspension. Alternatively, these molecules can be also immobilized on the solid surfaces and react with the antigen contained in a liquid phase.

The H5 HA antigen-binding molecules of the composition of the invention can be used in the immunological tests as non-labelled proteins and/or in the form of conjugates containing an analytically detectable label. Labelling of the H5 HA antigen-binding molecules of the composition of the invention can facilitate their use in diagnostics and extend the range of their applications. Various labels are described in the art, which are detectable with the use of available measuring apparatuses, similarly as the methods for the conjugation of the antigen-binding molecules with these labels. Immunoconjugates of the H5 HA-binding molecules will contain labels suitable for the specific detection and analysis techniques and/or the applied methods or techniques of immunological tests, in which they will find use.

The H5 HA antigen-binding molecules of the composition of the invention can be used in the most of the well-known techniques and methods of immunological tests. Examples include the following tests: immunoenzymatic, e.g., ELISA, immunofluorescent, immunochemiluminescent, radioimmunological, immunochromatographic, immunodiffusion, immunoprecipitation, based on the use of immunosensors. The H5 HA antigen-binding molecules can be also used for immunohistochemical staining or sorting of fluorescently-stained cells using a flow cytometer. Nevertheless, the above examples are not limiting to the technique or method of the test, in which the H5 HA antigen-binding molecules can be used. Because the binding molecules of the composition of the invention recognize conformational epitopes, they cannot be used in the Western blot-type analyses, where the molecules recognizing linear epitopes of antigens are used.

Preferably, the test for the detection or quantification, or semi-quantification of H5-serotype IVs using the H5 HA antigen-binding molecules of the composition of the invention is a sandwich ELISA. Preferably, the test for the detection or quantification, or semi-quantification of antibodies against H5-serotype IVs with the use of the binding molecules of the invention is ELISA in the blocking (bELISA) or competitive (cELISA) format. In the case of early diagnostics, it is preferable to use the binding molecules of the invention, e.g., in the immunochromatographic tests or immunosensors for the rapid and technically simple detection of H5-serotype IVs.

In order to show the applicability of the binding molecules of the invention in diagnostics, the bELISA H5 test (IBA) was developed and optimized for the detection of antibodies against H5 HA of influenza viruses in chicken sera. In the bELISA H5 test, one of the antibody clones of the composition of the invention, i.e., the G-7-27-18 mAb, was used as a key detecting antibody of the diagnostic test. For coating of the microtiter plates, the H5 HA protein produced in a baculovirus expression system (OET) was used. Assays were carried out in the presence of control samples. The control of maximum binding of the 7-27-18 mAb to the H5 HA antigen (mAb control) was obtained by performing analysis in the absence of serum. As a negative control, the normal chicken serum (Abcam) was used, whereas as strong and weak positive controls - antisera of chickens immunized with inactivated AIVs of the H5 serotype (x-OvO). The level of inhibition for the 7-27-18 mAb-antigen binding by sera, expressed as a percentage, was calculated according to the formula: inhibition% = 100 - [(A₄₅₀ sample/A₄₅₀ mAb control) ×100].

The preliminary evaluation of the diagnostic value for the bELISA H5 test was conducted using the samples previously classified as anti-H5 positive or negative based on the results of the HI test. In the validation procedures of the tests for diagnostics of IV infections, the HI test is treated as a so-called "gold standard". The cut-off value was determined based on the assay results for the anti-H5-negative serum samples (mean value of % inhibition + 2xSD).

The obtained results indicate that the bELISA H5 test allows to differentiate the chickens immunized with H5 HA antigens from the anti-H5-negative chickens. Importantly, antisera against AIVs of H1-H4 and H6-H16 serotypes do not show cross-reactions in the developed test. Presumably, the bELISA H5 test would enable also unambiguous diagnostics of the animals infected with H5-serotype IVs, such as the H5N1 HPAIV. In the case of vaccinations against HPAIVs with the use of the H5 HA proteins, the bELISA H5 test together with the test for the detection of antibodies against other AIV antigens, e.g., neuraminidase, could be used as a so-called DIVA test designated for the differentiation of infected from vaccinated animals.

Characterization of the prototype bELISA H5 test was expanded with preliminary determination of the key validation parameters of the diagnostic test. The obtained results showed that the bELISA H5 test is characterized by a satisfying repeatability (RSD: 7.1% - 10.0%) and the high factors of the analytical specificity (Asp: 100%), as well as the diagnostic specificity and sensitivity (Dsp: 97.6%; Dse 1: 98.0%; Dse 2: 99.1%).

The invention includes the diagnostic kits for the identification of viral infection in animals and humans, especially these evoked by H5-serotype IVs. The diagnostic kits of the invention enable the detection of IVs and antibodies directed against them, while simultaneously identifying serotype of infectious virus.

The diagnostic kits of the invention can be produced in many ways. Besides the binding molecules of the invention, which are crucial and characteristic for the recognition of infections with H5-serotype IVs, the kit can also contain commercially available components of any other immunological diagnostic test. The diagnostic kit of the invention contains a composition of the binding molecules of the invention. These molecules are mAbs of the composition comprising the G-1-31-22, G-2-14-10, G-5-32-5, G-6-42-42, G-7-24-17 and G-7-27-18 antibodies, but also their fragments or immunoconjugates, further their variants are disclosed.

Diagnostic kit for the detection of H5-serotype IVs that is disclosed can contain two different antibody clones selected from the group comprising the G-1-31-22, G-2-14-10, G-5-32-5, G-6-42-42, G-7-24-17 and G-7-27-18 mAbs, but also their fragments, variants or immunoconjugates. For example, the test can be carried out using the sandwich-type ELISA method. One antibody clone is used to coat microtiter plates, whereas the other, the enzyme-conjugated clone is used for the detection of antigen-antibody complex by the reaction of the labelling enzyme with its substrate, resulting in a detectable product.

Diagnostic kit for the detection of antibodies against H5-serotype IVs that is diclosed can contain one clone of antibodies selected from the group comprising the G-1-31-22, G-2-14-10, G-5-32-5, G-6-42-42, G-7-24-17 and G-7-27-18 mAbs or its fragments, variants or immunoconjugates. Use of the composition of the H5 HA-binding molecules of the invention is provided. For example, the assay can be conducted by the ELISA method in the blocking (bELISA) or competitive (cELISA) formats. In the bELISA/cELISA tests, the H5 HA antigen-binding molecule or molecules are labelled directly (immunoconjugates) or indirectly (e.g., using the labelled secondary antibodies), which provides detectability of the antigen-antibody complex.

The diagnostic kits of the invention contain tools and reagents commonly used in the immunological tests. When the H5 HA antigen-binding molecules are components of diagnostic kits based on the ELISA method, such kits contain additionally:
- plates coated with the mAbs of the invention (sandwich ELISA) or the H5 HA antigen (bELISA, cELISA), in which the non-specific binding sites are completely saturated with the use of an appropriate blocking substance,
- buffers for washing the plates, dilution of the tested samples and reagents,
- substrate for an enzyme and a stop solution for enzymatic reaction.

Each diagnostic kit using the H5 HA antigen-binding molecules includes also control samples: positive (PC) and negative (NC) controls, which contain viral particles or antibodies related to the H5-serotype IV infection (PC) or do not contain them (NC). Reagents for diagnostic kit are provided in the separate, labelled containers. An instruction for conducting the test is also attached to diagnostic tests.

The disclosed diagnostic test - bELISA H5 (IBA) for detecting antibodies against H5 HA of influenza viruses in chicken sera, contains:
- MediSorp plates (NUNC) coated with recombinant H5 HA protein (OET) and blocked with Protein-Free T20 (PBS) Blocking Buffer (Pierce/Thermo Scientific),
- concentrated solution of the antibodies (7-27-18 mAb) in Antibody Stabilizer PBS (Candor Bioscience),
- concentrated solution of anti-mouse IgG antibodies labelled with HRP, specific to γ chain (Sigma-Aldrich) in HRP-Protector (Candor Bioscience),
- positive control, PC (anti-H5 positive chicken serum),
- negative control, NC (normal chicken serum),
- incubation buffer (BSA/PBS + Proclin 300),
- buffers for preparing working dilutions of the antibodies of the invention and the secondary antibodies: Antibody Stabilizer PBS and HRP-Protector (Candor Bioscience), respectively,
- concentrated plate washing buffer (PBST + Proclin 300),
- TMB (Sigma-Aldrich),
- 0.5 M H₂SO₄.

Other applications of the H5 HA antigen-binding molecules of the invention comprise their use in epidemiological studies for early detection and typing of IVs, as well as for mapping of H5-specific epitopes in the HA antigens.

The binding molecules of the invention are directed against distinct, conformational epitopes in the HA1 subunit, which are probably conserved in the H5 HA proteins. It justifies their use for early detection and typing of IVs, and for molecular identification and characterization of the H5 HA antigens (epitope mapping). This kind of studies provides an important epidemiological data on the evolution and spreading of IVs, particularly of the H5 serotype, and the occurrence of novel strains. In the above applications the G-1-31-22, G-2-14-10, G-5-32-5, G-6-42-42, G-7-24-17 and G-7-27-18 mAbs can be used, but also their fragments, and immunoconjugates, further use of their variants is disclosed.

Other applications of the H5 HA antigen-binding molecules of the invention also include their use for the quality monitoring of the H5 HA antigens.

The binding molecules of the invention can be used for monitoring the quality of the H5 HA antigens intended for the use as vaccine antigens or reagents in diagnostic tests. In particular, the binding molecules of the invention can be used to check, whether the HA antigen contains epitopes specific for H5 serotype in the proper conformation. Moreover, the binding molecules of the invention can be used in the studies of the degree of oligomerization of the H5 HA proteins. For example, such study can be conducted using a sandwich ELISA test, in which one specified antibody clone, its variant or fragment is used in two forms, i.e., as a non-labelled protein and as an immunoconjugate comprising an analytically detectable label. In this type of the test, the antigen is captured by the non-labelled binding molecule of the invention adsorbed on a microtiter plate. The formed antigen-antibody complex is detected by the immunoconjugate providing that the antigen contains more than one epitope recognized by the binding molecule, i.e., when it is in the form of dimer, trimer or oligomer. The degree of oligomerization is an important element of property evaluation of the HA proteins, particularly these, which are intended for the production of influenza vaccines

The quality monitoring of the H5 HA proteins, which are the vaccine antigens or reagents in diagnostic tests, can be carried out using the G-1-31-22, G-2-14-10, G-5-32-5, G-6-42-42, G-7-24-17 and G-7-27-18 mAbs, but also their fragments, variants or immunoconjugates.

The main challenge of work on the subunit vaccines against influenza that are based on the use of HA, produced with genetic engineering methods, is to obtain the protein resembling the viral antigen. Especially important for the vaccine HA quality is the structure accuracy of the HA1 subunit, where the conformational epitopes for neutralizing antibodies are localized. The first indicator of the usefulness of HA proteins for vaccine production are the results of antigenicity studies using the well-characterized antibodies. The novel mAbs recognize only conformational epitopes in the HA1 subunit, probably conserved in the H5 HA proteins. Therefore, they can be successfully used in the studies of properties of antigens for the production of vaccines against H5-serotype IVs, independently from the source sequences of the vaccine antigens. It is also envisioned to use the obtained antibodies in the stability control of the vaccine H5 HA, storaged separately or in immunogenic compositions.

Another possible application of the obtained mAbs is their use to monitor quality of the H5 HA proteins intended for the use or already used as reagents in diagnostic tests. The novel mAbs show a broad range of specificities against the H5 HAs, thus they can be used to verify quality of the reagents, especially in these diagnostic tests, which are intended for the identification of the serotype of infectious strain.

It is disclosed that the H5 HA protein from a bacterial expression system was analyzed before and after denaturation under reducing conditions with the use of the G-6-42-42 and G-7-27-18 mAbs and the ELISA method. These analyses revealed that the HA protein looses its proper conformation under denaturing and reducing agents. The obtained results confirm usefulness of the produced antibodies for studying properties of the newly obtained H5 HA proteins as well as for the detection of changes in these proteins during storage.

Still another disclosed application of the H5 HA antigen-binding molecules includes their use for the isolation or purification of HA proteins.

The binding molecules can be used for the H5 HA antigen isolation or purification. For this purpose, the standard techniques can be used, such as affinity chromatography or immunoprecipitation. The HA antigens isolated and purified in this way can be used as reagents in diagnostic tests or as components of the prototype vaccines against H5-serotype IVs, intended for a preliminary evaluation of the vaccine ability to evoke protective immune responses in animals.

The description discloses the method for producing mAbs against influenza virus HA, showing a broad range of specificities against HAs of one specified serotype and not cross-reacting with HAs of the other serotypes. Preferably, the epitopes for the desired mAbs produced according to the disclosed procedure are strongly conserved within the serotype. The substance of the procedure of the invention is the use of recombinant protein with native-HA characteristics as an immunogen, the use of diverse group of HA antigens for the selection of antibodies for serotype specificity and the use of unique methods for clone differentiation. One of these methods is based on the immunoreactivity profiles, and the other on the peptide maps of Fab fragments of the selected mAbs.

According to the method disclosed, mAbs can be obtained by the hybridoma method described for the first time by Köhler and Milstein (1975). The method comprises as follows: immunization of mice with HA protein, isolation of lymphocytes from spleens of immunized animals, fusion of splenocytes with mouse myeloma cells and culturing, cloning and selection of hybridomas. In most of the described procedures for the production of mAbs against HA, IVs were used for immunization. What distinguishes the procedure disclosed is the use of recombinant protein preserving characteristics of the viral HA as an immunogen. Preferably, the first dose of antigen is administered in the form of an emulsion with CFA, prepared using two syringes connected by an emulsifying needle.

According to the standard procedure, the obtained antibodies are selected for the desired function and/or specificity using the methods known in the art. Selection for the ability of antibodies to protect against IV infection is carried out with the use of the HI and/or MN tests. Thus selected antibodies are directed against these HA epitopes, which are under strong selective pressure during a natural process of virus replication. As a result, the target epitopes may change, which in turn leads to narrowing of the specificity range of the obtained mAbs. Identification of antibodies of desired specificity is accomplished using immunological tests, such as ELISA, in which influenza viruses are usually used as antigens. The number of viral strains used is often limited, thereby the selection involves only the narrow range of HA protein variation.

What distinguishes the procedure disclosed is that the different HA antigen forms are used in antibody screening, preferably with various properties, expectably affecting the presentation of epitopes to the desired mAbs. Preferably, the HA proteins used in the immunoreactivity studies should display properties of viral antigen. It is also justified to broaden the panel of the antigens with proteins, which are devoid of native-HA features, as this will enable distinguishing between antibodies recognizing conformational and linear epitopes of the antigen. According to the procedure disclosed, the panel of HA antigens of a specified serotype, intended for immunoreactivity analyses, is characterized by a greate diversity of the homology with the immunogen used in the mAb obtaining procedure. For the serotype-specific selection of antibodies, it is necessary to perform negative selection with the use of HA antigens of the remaining fifteen serotypes.

In summary, the applied strategy for the selection of mAbs increases the probability of identification of these hybridomas, which produce the desired, serotype-specific antibodies with a broad specificity range. Moreover, the use of the test detecting functional properties of the antibodies will allow establishing, whether epitopes for these antibodies can be under immunological pressure during virus propagation.

At the final stage of the procedure, it is important to determine, how much the obtained clones differ from each other. This information can be provided by a comparison of the sequences encoding the complementarity-determining regions of the individual antibody clones. Obtaining these types of data is laborious and time-consuming, as it requires not only sequencing of the CDR-coding genes but also sequence verification by cloning and expression. Another way to differentiate the selected mAbs is based on the studies of their competition for the binding site on the antigen. In this type of tests, the lowering of the binding of one antibody clone by the other clone is interpreted as indicating that antibodies bind to the same epitope or to epitopes, which overlap or are located close to each other within the antigen molecule.

In the disclosed procedure for obtaining serotype-specific mAbs, the clone differentiation is based on the immunoreactivity profiles and peptide maps of Fab fragments of the generated antibodies. The applied differentiation methods are not so labor-consuming as establishing the sequences of the variable antibody parts and, in contrast to the methods based on antibody competition, give unambiguous results Thus, the invention provides a composition of six different clones of mAbs: G-1-31-22, G-2-14-10, G-5-32-5, G-6-42-42, G-7-24-17 and G-7-27-18, each of which recognizes the distinct, conformational epitope in the HA1 subunit of HA, shows a broad-range specificity against the H5-serotype HAs and at the same time, does not cross-react with HAs of the H1-H4 and H6-H16 serotypes. Preferably, the epitopes for these antibodies are probably highly conserved among the IVs of the H5 serotype and do not undergo changes under HI antibodies.

The antibodies of the invention were obtained by the hybridoma method using recombinant H5 HA protein with native-HA chracteristics as an immunogen. The first vaccination of mice was done with the use of an emulsion of the H5 HA protein and CFA, prepared using two syringes connected by an emulsifying needle. The screening of mAbs for serotype specificity was performed by the ELISA method using different forms of the H5 HA antigens having a varied homology with the immunogen (positive selection) and IVs of serotypes other than H5 (negative selection). As a result of selection, the G-1-31-22, G-2-14-10, G-5-32-5, G-6-42-42, G-6-42-71, G-7-24-17 and G-7-27-18 mAbs were chosen for further studies. The HI tests were performed showing that the selected antibodies do not have hemagglutination inhibition activity.

Based on the reactivity studies with H5 HA antigens, the immunoreactivity profiles of the obtained mAbs were created. As a result of ficin digestion, the Fc and Fab fragments of immunoglobulins were obtained, and then, the peptide maps of the Fab and Fc fragments from the individual clones were generated by means of trypsinolysis. Of major importance for the identification of the obtained antibody clones was a comparative analysis of peptide maps of antibody variable fragments responsible for antigen binding, i.e., Fab fragments. Both of the applied differentiation methods gave consistent results. It was shown that the G-1-31-22, G-2-14-10, G-5-32-5, G-7-24-17 and G-7-27-18 mAbs are the different clones, whereas the G-6-42-42 and G-6-42-71 mAbs represent one antibody clone, different from the remaining five clones.

### Description of the figures

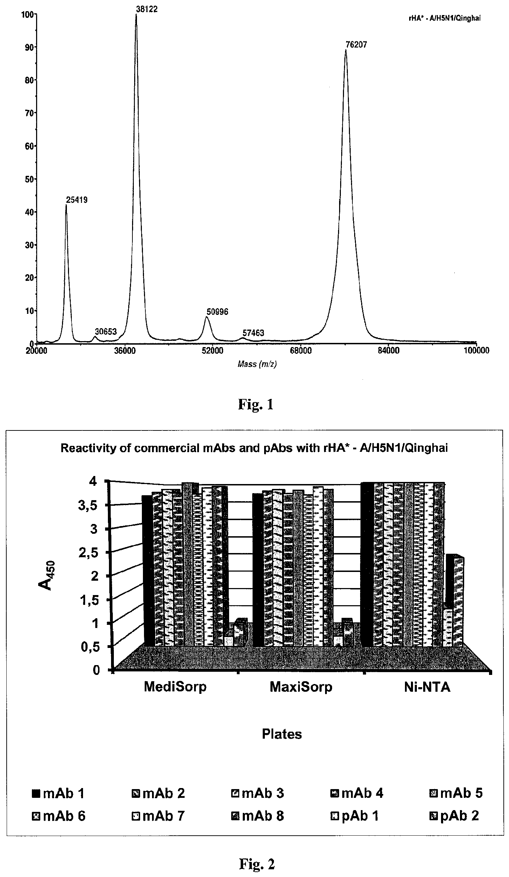

**Fig. 1** shows the mass spectrum of rHA - A/H5N1/Qinghai protein from (*) a mammalian expression system. The protein (17-530 aa, ΔRRRKKR, 6x His) was produced based on the sequence of HA from the A/Bar-headed Goose/Qinghai/12/05(H5N1) AIV strain (ITC). The mass spectrum was obtained using a MALDI TOF/TOF (4800 Plus, AB SCIEX) mass spectrometer.
**Fig. 2** shows the results of antigenicity studies of rHA - A/H5N1/Qinghai protein from (*) a mammalian expression system. The protein (17-530 aa, ΔRRRKKR, 6x His) was produced based on the sequence of HA from the A/Bar-headed Goose/Qinghai/12/05(H5N1) AIV strain (ITC). The studies were carried out using ELISA on MediSorp, MaxiSorp (NUNC) and Ni-NTA (Qiagen) plates. Commercially available mAbs against H5 HA (USBiological, ABR/Thermo Scientific, Acris Antibodies) and pAbs against the HA1 (pAb 1) and HA2 (pAb 2) subunits of the antigen (ITC) were used in the tests.

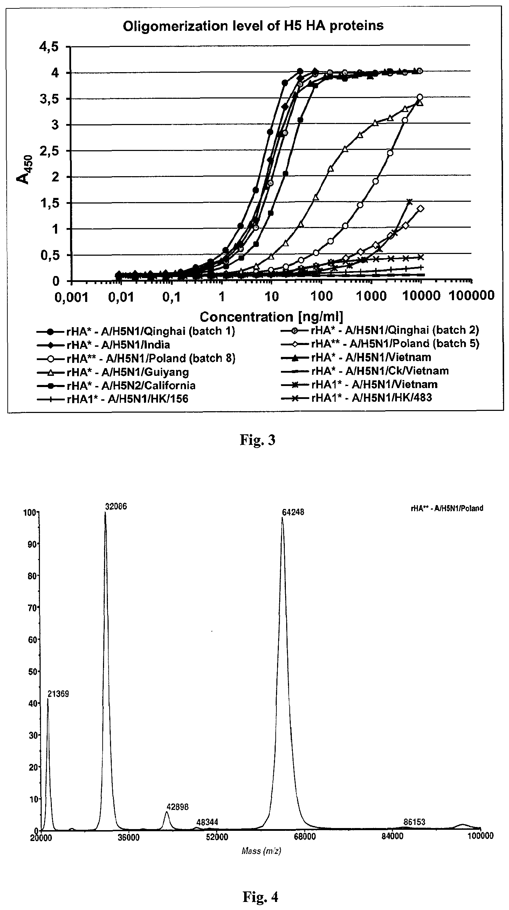

**Fig. 3** shows the results from testing the oligomerization levels of recombinant H5 HA proteins with sequences from various, H5-serotype IV strains. Proteins based on the ectodomain (rHA) or the HA1 subunit (rHA1) of the antigen were produced in (*) mammalian (ITC) or (**) baculovirus (OET) expression systems. The studies were carried out by the sandwich-type ELISA method using non-labelled and HRP-labelled commercial antibodies against H5 HA (Acris Antibodies).
**Fig. 4** shows the mass spectrum of HA - A/H5N1/Poland protein from (**) a baculovirus expression system. The protein (17-530 aa, ΔRRRKKR, 6x His) was produced based on the sequence of HA from the A/swan/Poland/305-135V08/2006(H5N1) AIV strain (OET, batch 2). The mass spectrum was obtained using a MALDI TOF/TOF (4800 Plus, AB SCIEX) mass spectrometer.

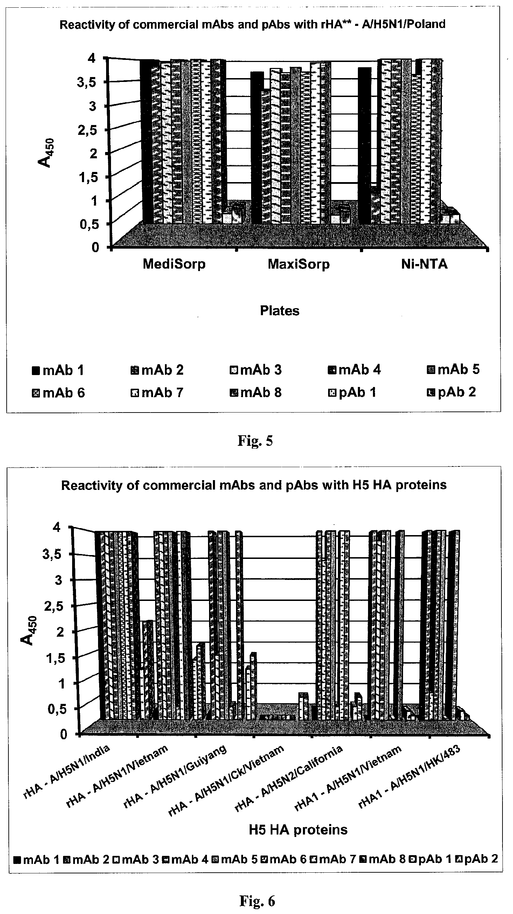

**Fig. 5** shows the results of antigenicity studies of rHA - A/H5N1/Poland protein from (**) a baculovirus expression system. The protein (17-530 aa, ΔRRRKKR, 6x His) was produced based on the sequence of HA from the A/swan/Poland/305-135V08/2006(H5N1) AIV strain (OET, batch 2). The studies were carried out using ELISA on MediSorp, MaxiSorp (NUNC) and Ni-NTA (Qiagen) plates. Commercially available mAbs against H5 HA (USBiological, ABR/Thermo Scientific, Acris Antibodies) and pAbs against the HA1 (pAb 1) and HA2 (pAb 2) subunits of the antigen (ITC) were used in the tests.
**Fig. 6** shows the results of antigenicity studies of recombinant proteins with HA sequences from various, H5-serotype IV strains. The H5 HA proteins, based on the ectodomain (rHA) and HA1 subunit (rHA) of the antigen, were produced in a mammalian expression system (ITC). The studies were carried out using ELISA on Ni-NTA (Qiagen) plates. Commercially available mAbs against H5 HA (USBiological, ABR/Thermo Scientific, Acris Antibodies) and pAbs against the HA1 (pAb 1) and the HA2 (pAb 2) subunits of the antigen (ITC) were used in the tests.

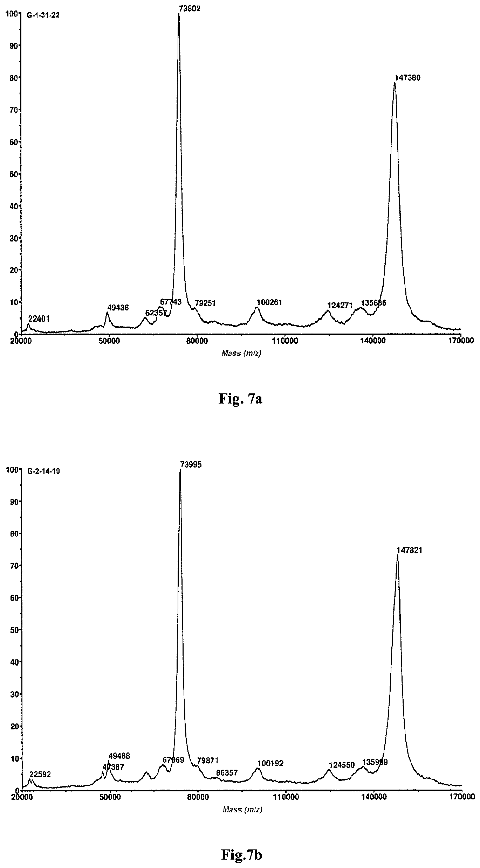

**Fig.7a****-g** show the mass spectra of the produced mAbs: (**a**) G-1-31-22, (**b**) G-2-14-10, (c) G-5-32-5, (d) G-6-42-42, (**e**) G-6-42-71, (**f**) G-7-24-17, (**g**) G-7-27-18. The mass spectra were obtained using a MALDI TOF/TOF (4800 Plus, AB SCIEX) mass spectrometer.

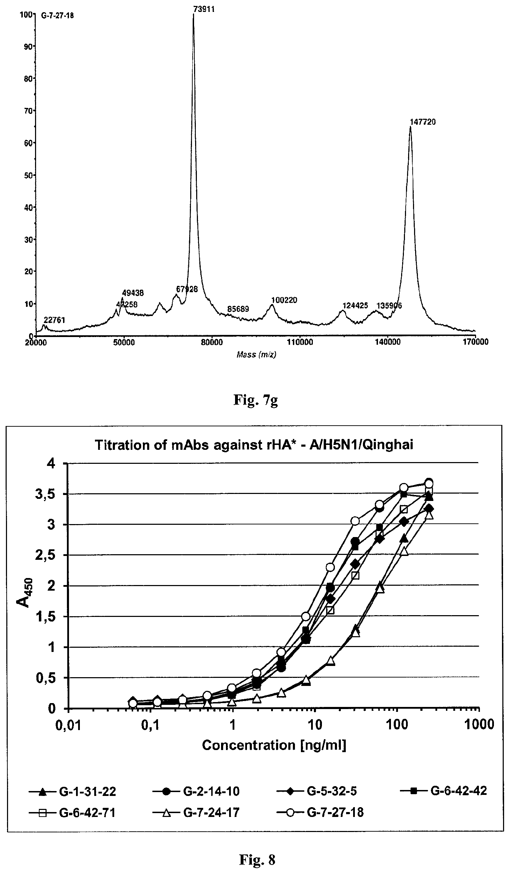

**Fig. 8** shows the titration curves of the G-1-31-22, G-2-14-10, G-5-32-5, G-6-42-42, G-6-42-71, G-7-24-17 and G-7-27-18 mAbs against rHA - A/H5N1/Qinghai protein from (*) a mammalian expression system. The protein (17-530 aa, ΔRRRKKR, 6x His) was produced based on the sequence of HA from the A/Bar-headed Goose/Qinghai/12/05(H5N1) AIV strain (ITC). The analysis was performed by ELISA on MediSorp plates (NUNC) coated with antigen at a concentration of 1 µg/mL.

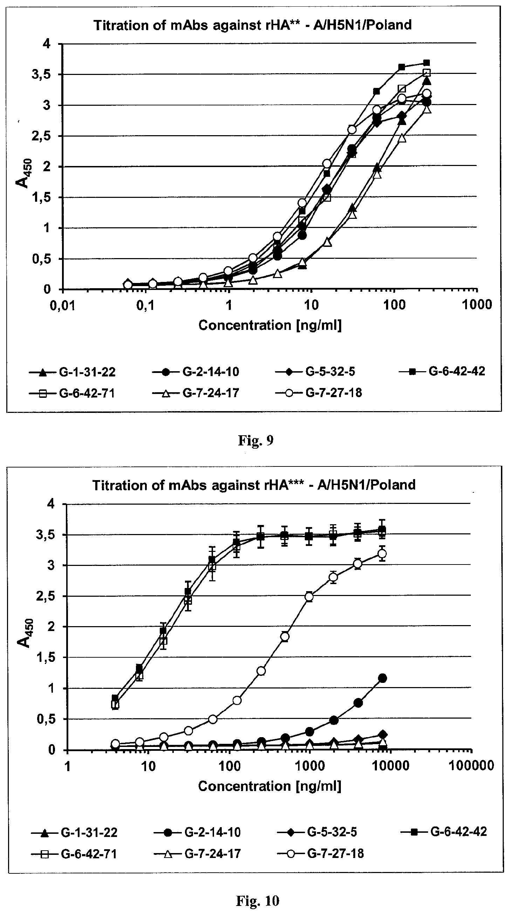

**Fig. 9** shows the titration curves of the G-1-31-22, G-2-14-10, G-5-32-5, G-6-42-42, G-6-42-71, G-7-24-17 and G-7-27-18 mAbs against rHA - A/H5N1/Poland protein from (**) a baculovirus expression system. The protein (17-530 aa, ΔRRRKKR, 6x His) was produced based on the sequence of HA from the A/swan/Poland/305-135V08/2006(H5N1) AIV strain (OET, batch 8). The analysis was performed by ELISA on MediSorp plates (NUNC) coated with antigen at a concentration of 1 µg/mL.
**Fig. 10** shows the titration curves of the G-1-31-22, G-2-14-10, G-5-32-5, G-6-42-42, G-6-42-71, G-7-24-17 and G-7-27-18 mAbs against rHA - A/H5N1/Poland protein from (***) a bacterial expression system. The protein (17-522 aa, ΔRRRKKR) was produced based on the sequence of HA from the A/swan/Poland/305-135V08/2006(H5N1) AIV strain (IBA). The analysis was performed by ELISA on PolySorp, MediSorp, MaxiSorp and MultiSorp plates (NUNC) coated with rHA preparation, ~80% pure, which contained the refoled hemagglutinin at a concentration of ~1 µg/mL.The results are shown as the mean A₄₅₀ values ± SD obtained using particular types of polystyrene plates.

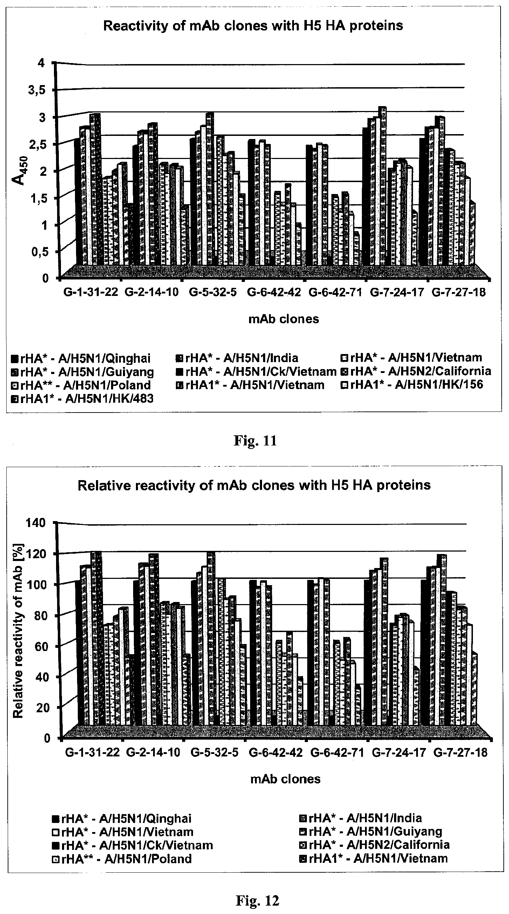

**Fig. 11** shows the results of reactivity studies of the G-1-31-22, G-2-14-10, G-5-32-5, G-6-42-42, G-6-42-71, G-7-24-17 and G-7-27-18 mAbs against H5 HA proteins with sequences from various IV strains. The studies were performed by ELISA using recombinant antigens based on the ectodomain (rHA) and HA1 subunit (rHA1) of hemagglutinin (List B) at concentrations of 1 µg/mL for coating of the Ni-NTA plates (Qiagen). The rHA - A/H5N1/Poland protein was produced in (**) a baculovirus expression system (OET, batch 8). The remaining rHA and rHA1 proteins originated from (*) a mammalian expression system (ITC). The purified antibodies were tested at the concentrations from the linear range of the titration curves against rHA - A/H5N1/Vietnam, for which the signal (A₄₅₀) levels were ~2.5 .The results are shown as the A₄₅₀ values.
**Fig. 12** shows the reactivity levels of the G-1-31-22, G-2-14-10, G-5-32-5, G-6-42-42, G-6-42-71, G-7-24-17 and G-7-27-18 mAbs against H5 HA proteins with various IV sequences relative to their reactivities against immunogen. The study was performed by ELISA using recombinant antigens based on the ectodomain (rHA) and HA1 subunit (rHA1) of hemagglutinin (List B) at a concentration of 1 µg/mL for coating of the Ni-NTA plates (Qiagen). The rHA - A/H5N1/Poland protein was produced in (**) a baculovirus expression system (OET, batch 8). The remaining rHA and rHA1 proteins originated from (*) a mammalian expression system (ITC). The purified antibodies were tested at the concentrations from the linear range of the titration curves against rHA - A/H5N1/Vietnam, for which the signal (A₄₅₀) levels were ~2.5. The signal (A₄₅₀) values obtained in the reactivity studies of the produced antibodies against recombinant H5 HA proteins are shown as % of the A₄₅₀ values read in the tests using the rHA - A/H5N1/Qinghai (relative reactivity against recombinant H5 HA proteins).

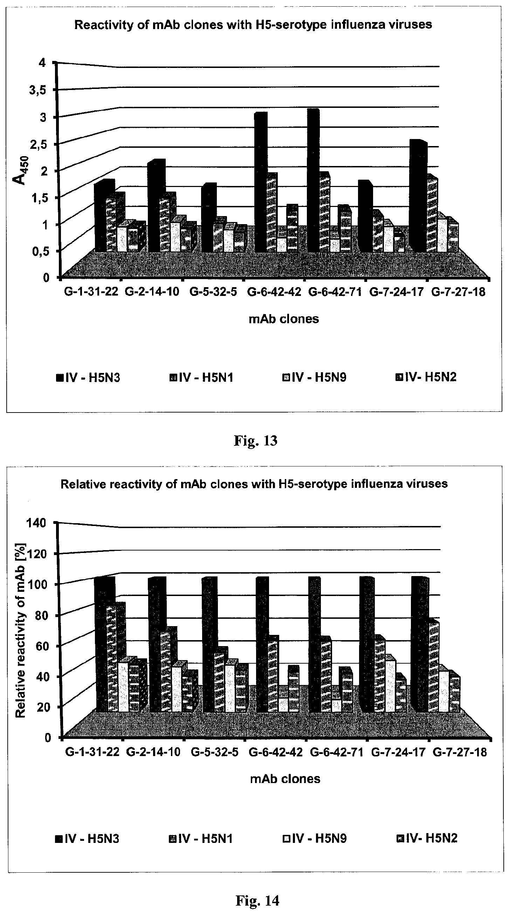

**Fig. 13** shows the results of reactivity studies of the G-1-31-22, G-2-14-10, G-5-32-5, G-6-42-42, G-6-42-71, G-7-24-17 and G-7-27-18 mAbs against H5-serotype LPAIVs. The studies were performed using ELISA by coating of MaxiSorp plates (NUNC) with H5N3, H5N1, H5N9 and H5N2 viruses diluted to 4000 HAU/mL based on the values specified by the manufacturer. Influenza viruses (x-OvO) originated from the IZSVe. The purified antibodies were tested at a concentration of 20 µg/mL. The results are shown as the A₄₅₀ values.
**Fig. 14** shows the reactivity levels of the G-1-31-22, G-2-14-10, G-5-32-5, G-6-42-42, G-6-42-71, G-7-2417 and G-7-27-18 mAbs against H5N1, H5N9 and H5N2 LPAIVs relative to their reactivities against H5N3 AIV. The studies were performed using ELISA by coating of MaxiSorp plates (NUNC) with H5N3, H5N1, H5N9 and H5N2 viruses diluted to 4000 HAU/mL based on the values specified by the manufacturer. Influenza viruses (x-OvO) originated from the IZSVe. The purified antibodies were tested at a concentration of 20 µg/mL. The signal (A₄₅₀) values obtained in the reactivity studies of the produced antibodies against H5N1, H5N9 and H5N2 AIVs are shown as % of the A₄₅₀ values read in the tests using the H5N3 AIV (relative reactivity against H5-serotype AIVs).

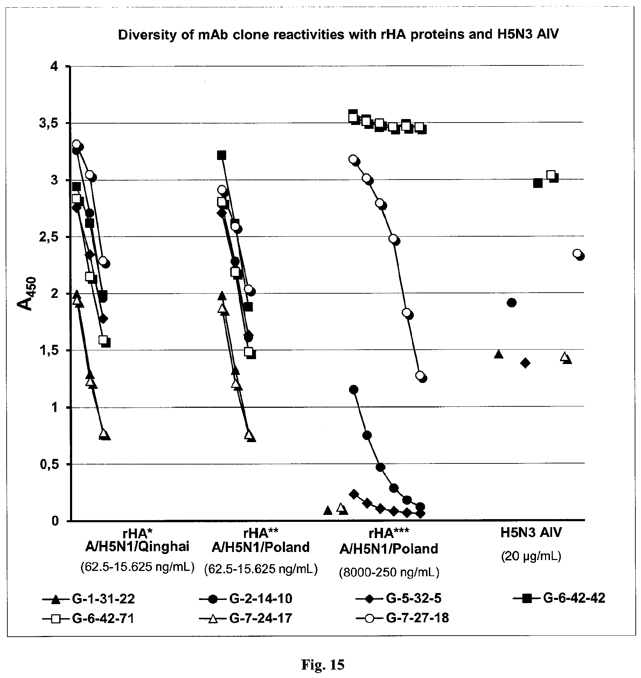

**Fig. 15** shows the reactivity profiles of the G-1-31-22, G-2-14-10, G-5-32-5, G-6-42-42, G-6-42-71, G-7-24-17 and G-7-27-18 mAbs against recombinant HA proteins and avian influenza virus with the highest homology to the immunogen among the used rHA proteins and H5-serotype AIVs, respectively. The antigen: 17-530 aa (ΔRRRKKR, 6x His) with the HA sequence from the A/Bar-headed Goose/Qinghai/12/05(H5N1) AIV strain was produced in (*) a mammalian expression system (ITC). The HA proteins: 17-530 aa (ΔRRRKKR, 6x His) and 17-522 aa (ΔRRRKKR) were produced in (**) baculovirus (OET, batch 8) and (***) bacterial (IBA) expression systems, respectively, based on the HA sequence from the A/swan/Poland/305-135V08/2006(H5N1) AIV strain. The H5N3 influenza virus (x-OvO) originated from the IZSVe. For coating with rHA proteins (1 µg/mL), the MediSorp (rHA*, rHA**), PolySorp, MediSorp, MaxiSorp and MultiSorp (rHA***) plates (NUNC) were used, whereas with the H5N3 virus (4000 HAU/mL) the MaxiSorp plates (NUNC), The results are shown as the signal (A₄₅₀) values obtained using the generated mAbs at concentrations of 62.5-15.625 ng/mL (rHA*, rHA**), 8000-250 ng/mL (rHA***) and 20 µg/mL (AIV H5N3).

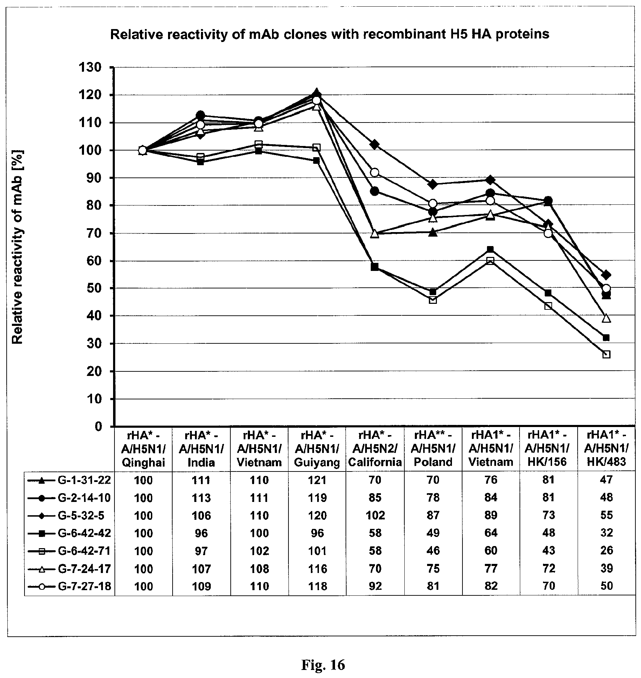

**Fig. 16** shows the profiles of relative reactivities of the G-1-31-22, G-2-14-10, G-5-32-5, G-6-42-42, G-6-42-71, G-7-24-17 and G-7-27-18 mAbs against proteins with the H5 HA sequences from various IV strains. The analysis was performed by ELISA using recombinant antigens based on the ectodomain (rHA) and HA1 subunit (rHA1) of hemagglutinin (List B) at a concentration of 1 µg/mL for coating of the Ni-NTA plates (Qiagen). The rHA - A/H5N1/Poland protein was produced in (**) a baculovirus expression system (OET, batch 8). The remaining rHA and rHA1 proteins originated from (*) a mammalian expression system (ITC). The purified antibodies were tested at the concentrations from the linear range of the titration curves against rHA - A/H5N1/Vietnam, for which the signal (A₄₅₀) levels were ~2.5. The relative reactivities of the obtained mAbs against recombinant H5 HA proteins are the reactivity levels expressed as % of the A₄₅₀ values read in the tests using the rHA* - A/H5N1/Qinghai.

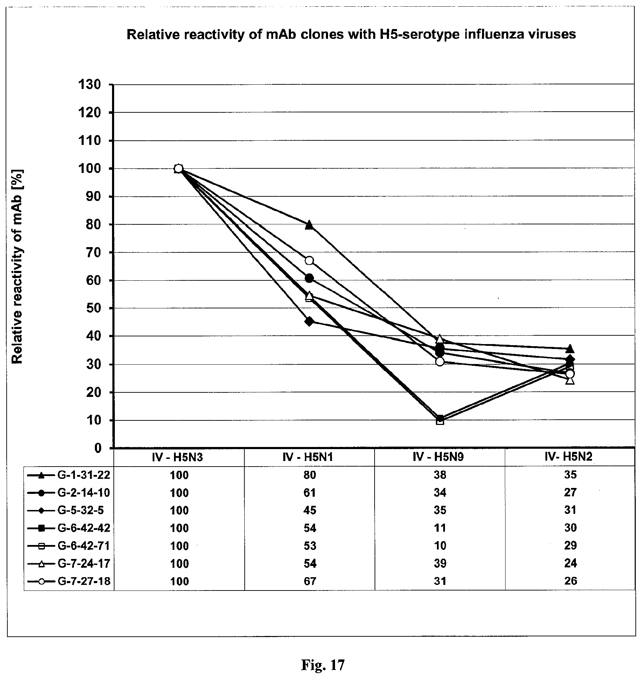

**Fig. 17** shows the profiles of relative reactivities of the G-1-31-22, G-2-14-10, G-5-32-5, G-6-42-42, G-6-42-71, G-7-24-17 and G-7-27-18 mAbs against the H5N1, H5N9 and H5N2 AIVs. The assay was performed using ELISA by coating of MaxiSorp plates (NUNC) with H5N3, H5N1, H5N9 and H5N2 viruses diluted do 4000 HAU/mL. Influenza viruses of H5 serotype (x-OvO) originated from the IZSVe. The purified antibodies were tested at a concentration of 20 µg/mL. The relative reactivities of the obtained mAbs against H5-serotype AIVs are the reactivity levels expressed as % of the A₄₅₀ values read in the tests using the H5N3 AIV.

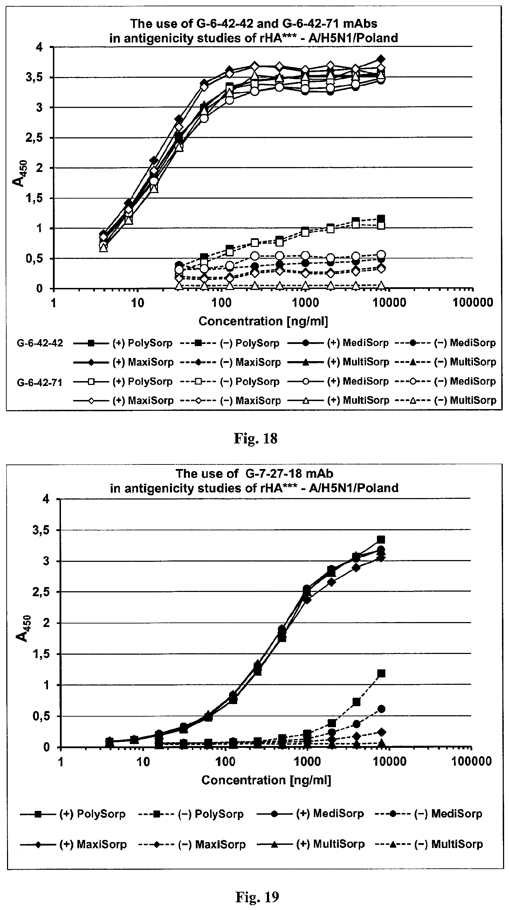

**Fig. 18** shows the titration curves of the G-6-42-42 and G-6-42-71 mAbs against (+) conformational and (-) denatured rHA - A/H5N1/Poland from (***) a bacterial expression system. The protein (17-522 aa, ΔRRRKKR) was produced based on the HA sequence from the A/swan/Poland/305-135V08/2006(H5N1) AIV strain (IBA). The purified and refolded H5 HA protein was subjected to denaturation under reducing conditions. The analysis was performed by ELISA on PolySorp, MediSorp, MaxiSorp and MultiSorp plates (NUNC) coated with rHA preparation, ~80% pure, which contained hemagglutinin at a concentration of ~1 µg/mL.
**Fig. 19** shows the titration curves of the G-7-27-18 mAb against (+) conformational and (-) denatured rHA - A/H5N1/Poland protein from (***) a bacterial expression system. The protein (17-522 aa, ΔRRRKKR) was produced based on the HA sequence from the A/swan/Poland/305-135V08/2006(H5N1) AIV strain (IBA). The purified and refolded H5 HA protein was subjected to denaturation under reducing conditions. The assay was performed by ELISA on PolySorp, MediSorp, MaxiSorp and MultiSorp plates (NUNC) coated with rHA preparation, ~80% pure, which contained hemagglutinin at a concentration of ~1 µg/mL.

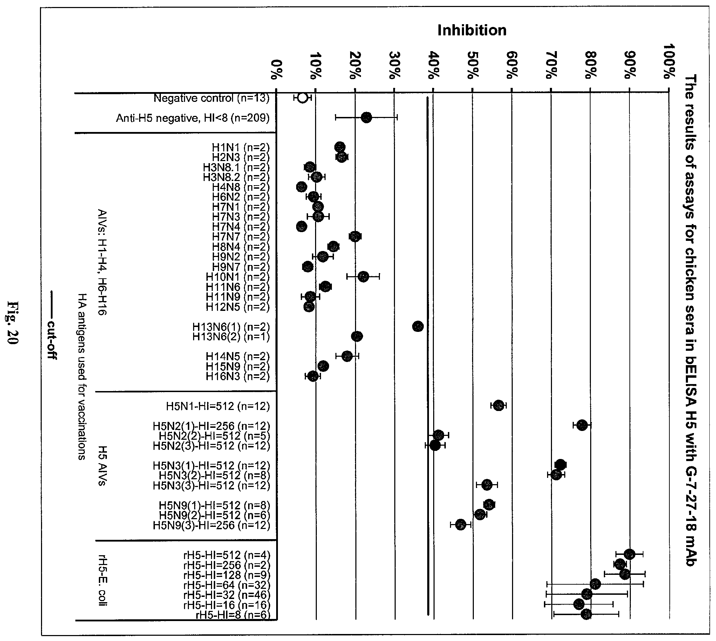

**Fig. 20** shows the results of chicken serum assays using a blocking ELISA for the detection of antibodies against H5 HA (bELISA H5), which was developed and optimized at the IBA. In the bELISA H5 test (IBA), the G-7-27-18 mAb was used. The analyzed samples were previously classified as anti-H5 positive or negative based on the results of the HI test. The cut-off value was determined based on the assay results for anti-H5-negative serum samples (mean value of % inhibition + 2xSD). Data from analyses of the negative control and anti-H1 (H2-H16)-positive sera was presented as the mean values of the % inhibition from a specified number of independent determinations (n) of particular samples. The results of the remaining analyses were presented as the mean values of the % inhibition from a specified number of samples (n) in individual groups of sera. In the Figure, the assay results for the negative control, and the strong and weak positive controls, are highlighted.

The embodiment of the invention is presented below. The embodiments presented do not limit protection of the subject patent application.

### Example 1

### Procedure for the production of mAbs against hemagglutinin of H5-serotype influenza viruses.

Monoclonal antibodies were obtained using the classic hybridoma method, developed in the mouse model, by using *in vivo* immunization. In this method, the animals are immunized several times with the antigen. Splenocytes isolated from spleens of immunized animals are fused with myeloma cells in the presence of 50% PEG and 5% DMSO. Cell culturing in HAT differentiation medium causes that the splenocytes and non-fused myeloma cells die, and only the hybridomas survive. During cloning, the individual hybridomas are isolated, and the culture supernatants of the hybridomas, derived from a single hybridoma, are tested for the presence of particular-class antibodies (isotyping) specific against immunogen. Hybridoma clones can be cultured *in vitro* or in an ascite.

In the first stage of the procedure for mAb production, the mouse immunization was conducted. The purified H5 HA protein (17-530 aa, ΔRRRKKR, 6x His), produced in a mammalian expression system based on the HA sequence from the A/Bar-headed Goose/Qinghai/12/05(H5N1) AIV strain by Immune Technology Corporation (ITC), was used as the immunogen. The rHA - A/H5N1/Qinghai protein was previously subjected to analyses by mass spectrometry, ELISAs for antigenicity and HA protein oligomerization, and a hemagglutination test. The method of carrying out the tests and their results are presented in Example 2. In Example 2, the properties of rHA - A/H5N1/Qinghai immunogen are also described as indicating its usefulness in the procedure for the production of mAbs against H5 HA. According to the standard method, obtaining the antigen-specific mAbs comprises as follows: mouse immunization, fusion of splenocytes with mouse myeloma cells and hybridoma culturing, cloning and selection.

### Mouse immunization

Five, 6-week-old female Balb/c mice were immunized subcutaneously with rHA (10 µg/mouse) at two sites in the neck with Complete Freund's Adjuvant (CFA). An emulsion of antigen with CFA in a 1:1 ratio (v/v) was prepared using two 1-mL syringes connected by an emulsifying needle (Popper & Sons). Immunologically beneficial consequences of using the 2-syringe method for the preparation of the vaccine in the emulsion form and the advantage of this method over the other emulsification techniques are described in the literature (Koh Y.T. et al., 2006). Immunization was conducted under anaesthesia. Another two doses of antigen, 10 µg each, were given intraperitoneally in the form of the PBS solution. The second immunization was conducted 2 weeks after the 1st dose, and the third immunization was performed 3 weeks after the 2_{nd} dose. The fourth immunization was performed 17 days after the 3_{rd} dose by intravenous administration of rHA (10 µg/mouse) in PBS.

### Control of a titer of anti-H5 HA antibodies in plasma

Eleven days after the administration of the 3_{rd} dose and nine days before the fusion, blood was collected from the retro-orbital sinus of all of the immunized mice using heparinized capillary tubes. Plasma obtained after blood centrifugation was aliquoted and frozen at -20oC. To determine the titer of antibodies against H5 HA, the plasma samples were analyzed by ELISA using the HA protein used for animal immunization (rHA - A/H5N1/Qinghai). In the tests, the rHA - A/H5N1/Poland protein with the HA sequence from the A/swan/Poland/305-135V08/2006(H5N1) AIV strain, produced by Oxford Expression Technologies (OET) in a baculovirus expression system, was also used. The protein characterized by the high homology to the immunogen was described in detail in Example 3. To develop the plates, the anti-mouse IgG antibodies specific to γ chain were used. The use of the highly specific secondary antibodies in the performed tests enabled the determination of the H5-specific antibodies exclusively of the IgG class, and thereby the correct evaluation of the animal responses to immunization.

ELISAs were performed with the use of microtitration plates (NUNC). The rHA - A/H5N1/Qinghai (ITC), diluted in PBS to 5 µg/mL, was applied to MaxiSorp plates, whereas the rHA - A/H5N1/Poland (OET, batch 1), diluted in PBS to 7 µg/mL, to MediSorp plates. The plates with antigens were incubated overnight at 2-8oC. The non-specific binding sites on the plates were blocked with 10% FBS/PBS. Plasma samples from immunized mice were tested in 2-fold serial dilutions in PBS. The negative controls were the plasma samples from non-immunized mice. The dilution buffer was the control for non-specific binding of the secondary antibodies (BLK sample). The tested and control samples were incubated on the coated plates for 2 hours at room temperature. The plates were developed by 1-hour incubation at 37oC with HRP-labelled anti-mouse IgG antibodies specific for γ chain (Sigma-Aldrich). The secondary antibodies were diluted 1:1000 in 2% BSA/PBS. TMB (Sigma-Aldrich) was used as a substrate for horseradish peroxidase. The reaction was stopped with 0.5 M H₂SO₄ solution. The absorptions of the samples were read at λ=450 nm. The titer of IgG-class antibodies against H5 HA was defined as the plasma dilution, at which absorption (A₄₅₀) reaches a value of ~1.0.

### Hybridoma generation, culturing and cloning

The immunized mice were sacrificed by cervical dislocation 3 days after intravenous administration of the last boosting dose. The spleens were collected in an aseptic manner and the splenocytes were isolated. Following cell collection and centrifugation (10 min, 1000 rpm, 5°C), the pellet was resuspended in complete RPMI-1640, the cells were counted and evaluated for viability in a Thoma's camera in the presence on trypan blue. The cells of the mouse myeloma cell line SP2/0 (ATCC) were cultured in HT medium to the logarithmic growth phase, centrifugated at 1000 rpm, resuspended in RPMI-1640 and the live cells were counted.

For the fusion, the splenocytes isolated from the spleen of the mouse with the highest titer of the IgG antibodies against H5 HA in plasma were chosen. The spleen and myeloma cells in RPMI-1640 were mixed in the 2:1 ratio. Following centrifugation and precise removing of the supernatant, about 1 mL of the following solution was added for 1 min at 37°C: 50% PEG 1500, 5% DMSO in PBS without calcium and magnesium ions with gentle mixing of the pellet. The fusion was stopped by adding heated RPMI-1640 medium with the addition of antibiotics (streptomycin - 50 µg/mL, penicillin - 50 U/mL) - the first 4 mL slowly, dropwise, and the next 16 mL - quickly. The cell suspension was centrifugated for 10 min at 1000 rpm, at 5°C. The pellet was resuspended in HAT medium and transferred into eight 96-well plates containing a layer of mouse peritoneal macrophages. After a week of incubation at 37°C in an atmosphere of 5% CO₂, the cell cultures were feed with HAT medium, and in the following week - with HT.

Cloning of hybridomas was performed by further dilutions. Cell suspensions were prepared to contain 10 and 5 cells/mL, respectively. Each of the suspensions was aliquoted, 100 µL/well, into 96-well plate with a macrophage layer. The wells were observed and these with clones originating from 1 cell were marked.

After the fusion, the hybridomas were cultured in RPMI-1640 medium supplemented with 20% FBS, containing 2 mM glutamine, 2 mM sodium pyruvate, antibiotics (streptomycin - 50 µg/mL, penicillin - 50 U/mL) and 100 µM hypoxanthine, 2,5 µM aminopterin and 16 µM thymidine (HAT differentiation medium) or 100 µM hypoxanthine and 16 µM thymidine (HT medium), as required. During cloning, the RPMI-1640 medium with the addition of 20% FBS, containing 2 mM glutamine, 2 mM sodium pyruvate and antibiotics (streptomycin - 50 µg/mL, penicillin - 50 U/mL) was used. Further hybridoma culture was carried out in the medium having the same composition as the cloning medium, with the exception of FBS concentration (10%). For freezing the obtained clones in liquid nitrogen, the complete RPMI-1640 medium containing 30% FBS and 10% DMSO was used. Cell number and viability were determined using trypan blue solution in physiological saline. For the isolation of peritoneal macrophages, the Hank's salt solution without calcium and magnesium was used. All of the used reagents were company tested for the absence of toxicity for cell cultures and originated from Sigma-Aldrich or Gibco.

### Hybridoma selection

Hybridoma culture supernatants were analyzed for the presence of antibodies specific for H5 HA. The analyses were performed by the ELISA method. The preliminary hybridoma selection was performed using the rHA - A/H5N1/Qinghai (ITC) as well as the rHA - A/H5N1/Poland (OET) with the sequence very similar to the immunogen's sequence. Further hybridoma selection was carried out by determining the specificity of the generated antibodies against recombinant H5 HA proteins based on the hemagglutinin ectodomain (rHA) or the HA1 subunit (rHA1) with a varied homology to the immunogen, which were produced in a mammalian expression system (ITC). In ELISAs performed for culture supernatants from the non-cloned hybridomas, the proteins with the HA sequences from six H5-serotype AIV strains were used. After cloning, the selection of mAbs was performed using the rHA or rHA1 proteins based on the HA sequences of nine H5-serotype AIV strains. For positive selection of cloned hybridomas, the H5N1, H5N2, H5N3 and H5N9 IVs (x-OvO) were also used. At each step of the procedure, the antibody isotyping was performed.

Clones of mAbs with the highest diagnostic value, chosen as a result of selection, were purified from the hybridoma culture supernatants using affinity chromatography. The antibodies were characterized by mass spectrometry and ELISAs with recombinant H5 HA proteins (rHA, rHA1) and H5-serotype IVs. The tests using IVs of serotypes other than H5, i.e., H1-H4 and H6-H16, were also performed. To evaluate the ability of the generated mAbs to inhibit hemagglutination, the HI testes were carried out.

The antigens and the tests used for the selection of hybridomas generating antibodies against H5 HA, as well as the method of carrying out antibody isotyping are described in detail in Example 3. The antigens and the tests used for further selection of antibodies for serotypic specificity, as well as of hybridomas generating these antibodies are described in Example 4. The results of mAb selection at particular stages of the procedure are described in Example 5. Properties of the selected mAbs are described in Examples 6-10.

### Example 2

### Properties of the immunogen

Commercially available mAbs against H5 HA are usually obtained by the hybridoma method using IVs for animal immunization (ABR/Thermo Scientific, Acris Antibodies, USBiological). In the mAb production, the recombinant HA proteins are also used (USBiological). Prerequisite for the generation of antibodies with a high application value in prevention, therapy and diagnostics of IV infections is the use of the antigen with viral HA properties. In the case of production of mAbs for the use in influenza diagnostics, it is desirable for the antigen to contain primarily well-preserved epitopes specific for the serotype. Therefore, in the production procedure of mAbs specific against H5 HA serotype by the hybridoma method, it was of a key importance to use an immunogen fulfilling above requirements.

Before starting the production procedure for mAbs against H5 HA, information on commercially available H5 HA antigens was collected. The products from Immune Technology Corporation were selected, wherein the company specializes in the production of viral antigens and anti-viral antibodies for diagnostics, monitoring and therapy of viral infectious diseases. At the ITC, recombinant H5 HA proteins from several strains of the H5N1 virus, as well as the HAs of serotypes other than H5, are available. The antigens are produced in a mammalian expression system. The usefulness of purified, recombinant H5 HA proteins for the selection of antibodies and hybridomas producing them was verified using ELISAs for antigenicity and oligomerization studies, which are described in Example 4. In the present Example, the results of testing the properties of recombinant H5 HA protein used as an immunogen are shown.

For the production of mAbs against H5 HA by the hybridoma method, recombinant protein with the HA sequence from the A/Bar-headed Goose/Qinghai/12/05(H5N1) AIV strain (ITC) was selected. The protein, which was expressed in mammalian cells without a signal sequence, transmembrane and cytoplasmic domains, as well as a cleavage site (ΔRRRKKR), was the HA fragment (17-530 aa) containing the HA1 subunit, part of the HA2 subunit and a histidine tag - 6x His at the C-terminus. Due to the eukaryotic origin, the produced antigen was a glycoprotein, just as native HA. According to the specification, the HA protein with molecular weight of ~75 kDA (SDS-PAGE) and at least 95% purity, exists mainly in a form of trimers/oligomers. To evaluate its usefulness in the production procedure of mAbs against H5 HA, the protein, hereinafter described as rHA - A/H5N1/Qinghai, was subjected to analysis by mass spectrometry, ELISAs for antigenicity and oligomerization studies and the hemagglutination test.

### The mass spectrum of rHA - A/H5N1/Qinghai

The mass spectrum of rHA - A/H5N1/Qinghai was obtained using a MALDI TOF/TOF (4800 Plus, AB SCIEX) mass spectrometer. Before performing the mass spectra, the samples were purified using ZipTip®c₄ (Millipore) according to the procedures included in the manufacturer's instruction: "User Guide for Reversed-Phase ZipTip". The matrix was the α-cyano-4-hydroxycinnamic acid (Fluka) at a concentration of 5 mg/mL dissolved in 0.1% trifluoroacetic acid containing 50% of acetonitrile. The mass spectra were measured in the linear mode (MS Linear Positive Ion), in the range of 20 - 100 kDa. External calibration was achieved with ProMix3 mixture (LaserBio Labs). The data acquisition method was established using the MALDI TOF/TOF - 4000 Series Explorer software. The mass spectra of rHA - A/H5N1/Qinghai were processed with a Gaussian filter and the signal detection procedures using the Data Explorer Software (V4.9).

Fig. 1 shows the mass spectrum of rHA - A/H5N1/Qinghai. The molecular weight signals of 76 kDa and 38 kDa originate from the tested HA protein and correspond sequentially to the singly- and doubly-ionized samples. The rHA - A/H5N1/Qinghai molecular weight, determined using a MALDI TOF/TOF mass spectrometer (MS), is of 76 kDa and due to glycosylation, it is higher by ~18 kDa than the weight calculated from the amino acid composition using the GPMAW 8.2 software (Lighthouse).

### Antigenicity of rHA - A/H5N1/Qinghai

The antigenicity studies of rHA - A/H5N1/Qinghai were conducted using commercially available mAbs and pAbs against H5 HA of influenza virus. Based on the specifications, a list of the used antibodies was prepared, as presented below.

**List A Monoclonal and polyclonal antibodies used in the antigenicity studies of HA proteins.**

| **Name** | | **Origin Cat. no.** | **Immunogen** | **Type Isotype** | **Application** |
|---|---|---|---|---|---|
| **Monoclonal antibodies (mAbs) against H5 HA** | | | | | |
| Recognize H5 HA in the HI test, react with H5N1, H5N2 and H5N9 influenza viruses, do not cross-react with HAs of other serotypes (H1, H2, H3, H4, H6, H7, H8, H9, H10, H11, H12, H13, H14 and H15). | | | | | |
| **mAb 1** | Influenza A Hemagglutinin H5 (Avian H5N1) | USBiological, 17649-41 B | Purified avian influenza virus type A (H5N1) | mAb IgG2a* | HI, iELISA DB |
| Specific for H5 HA of influenza A virus [A/Vietnam/1203/04 (H5N1) strain], do not cross-react with HAs of other serotypes, react with H5N1 influenza viruses representatives of different clades and subclades. | | | | | |
| **mAb 2** | Influenza A, Avian, H5N1A/Vietnam/1203/04 Hemagglutinin (Bird Flu) | USBiological, 17649-42C | HA of influenza virus A/Vietnam/1203/04(H5N1) | mAb IgG2a* | HI, VN, ELISA, IP, IHC, WB |
| Recognize H5 HA in the HI test. | | | | | |
| **mAb 3** | Influenza A Hemagglutinin H5 (Avian H5N1) | USBiological 17649-42D | Purified avian influenza virus type A (H5N1) | mAb IgG2a* | HI ELISA |
| Detect influenza A H5 antigen in viral samples. | | | | | |
| **mAb 4** | Anti-Influenza A H5 Antigen Monoclonal Antibody | ABR/Thermo Scientific MA 1-81927 | Purified avian influenza virus type A (H5N1) | mAb IgG2a* | ELISA |
| React with H5 HA in the HI test, react with H5N1, H5N2 and H5N9 influenza viruses, do not cross-react with HAs of other serotypes (H1, H2, H3, H4, H6, H7, H8, H9, H10, H11, H12, H13, H14 and H15). | | | | | |
| **mAb 5** | Monoclonal Antibody to Influenza A (Hemagglutinin H5) H5N1 ― Purified | Acris Antibodies AM00942PU-N | Purified avian influenza virus type A (H5N1) | mAb IgG2a* | HI, iELISA, DB |
| **mAb 6** | Monoclonal Antibody to Influenza A (Hemagglutinin H5) H5N1 ― Purified | Acris Antibodies AM00944PU-N | Purified avian influenza virus type A (H5N1) | mAb IgG2a* | HI, iELISA, DB |
| **mAb 7** | Monoclonal Antibody to Influenza A (Hemagglutinin H5) H5N1 ― Purified | Acris Antibodies AM00945PU-N | Purified avian influenza virus type A (H5N1) | mAb IgG2a* | HI, iELISA, DB |
| **mAb 8** | Monoclonal Antibody to Influenza A (Hemagglutinin H5) H5N1 ― Purified | Acris Antibodies AM00941 PU-N | Purified avian influenza virus type A (H5N1) | mAb IgG2a* | HI, iELISA, DB |

| **Polyclonal antibodies (pAbs) against H5 HA** | | | | | |
|---|---|---|---|---|---|
| React with H5 HA of human and avian H5N1 influenza viruses. Cross-reactivity to other HAs not tested. | | | | | |
| **pAb 1** | Anti-HA (H5N1) (Bar headed goose/Qinghai/ 1 A/05) | ITC IT -003-005G | Hemagglutinin of influenza A virus (H5N1) (1-345 aa) (A/Bar-headed Goose/Qinghai/ 12/05(H5N1)) | pAb rabbit IgG* | WB itd. |
| React with HA and HA2 subunit (H5N1). Cross-reactivity aqainst other serotypes not tested. | | | | | |
| **pAb 2** | Anti-HA2 (H5N1) | ITC IT -003-010 | HA2 protein (H5N1) (347-523 aa) (A/VietNam/1203/2004(H5N1)) | pAb rabbit IgG* | WB itd. |
| *purified, HI - hemagglutination inhibition test, iELISA - indirect ELISA (enzyme-linked immunosorbent assay), DB - dot blot, VN - virus neutralization test, IP - immunoprecipitation, IHC - immunohistochemistry, WB - Western blot, ITC - Immune Technology Corporation | | | | | |

Monoclonal antibodies were from USBiological (3 clones), ABR/Thermo Scientific (1 clone) and Acris Antibodies (4 clones). The mAbs were obtained using the purified H5N1 AIVs (7 clones) or recombinant protein with the HA sequence from the A/Vietnam/1203/04(H5N1) strain (1 clone) as the immunogens. According to the specifications, the antibodies, described as mAb 1 and mAb 5 - 8, recognize H5 HA in the HI test, bind to the H5N1, H5N2 and H5N9 IVs and do not cross-react with HAs of the H1-H4 and H6-H15 serotypes. Specificity of the remaining mAb clones is described as the ability to recognize H5 HA in viral samples (mAb 4) or in the HI test (mAb 3). According to the manufacturer's information, the antibodies, designated as mAb 2, are specific against HA of the A/Vietnam/1203/04 (H5N1) strain, react with H5N1 IVs from various clades and subclades and do not cross-react with HAs of serotypes other than H5. The specifications for all of the used mAbs indicate that the antibodies recognize conformational epitopes.

In the immunoreactivity studies, the polyclonal antibodies against the HA1 and HA2 subunits of H5 hemagglutinin were also used. The antibodies, described hereafter as pAb 1 and pAb 2, were obtained using the HA1 protein (1-345 aa) of the A/Bar-headed Goose/Qinghai/12/05(H5N1) strain and the HA2 subunit fragment (347-523 aa) of the A/VietNam/1203/2004(H5N1) strain (ITC) as the immunogens, respectively. According to the specifications, the pAb 1 and pAb 2 can be used in the Western blot-type analyses. It can be expected that pAbs against HA will recognize both the conformational and linear epitopes of the proteins.

The studies on the recognition of immunogen, i.e., the rHA - A/H5N1/Qinghai, by commercial antibodies were carried out by ELISA using polystyrene PolySorp, MaxiSorp, MediSorp and MultiSorp plates (NUNC), as well as Ni-NTA plates (Qiagen). Polystyrene plates were coated with rHAs in PBS at 5 µg/mL overnight at 2-8oC. The non-specific binding sites on the plates were blocked with 10% FBS/PBS. The HA protein diluted in 1% BSA/PBS to a concentration of 1 µg/mL was applied to a Ni-NTA plate. To control the level of non-specific antibody binding, some of the wells were filled with 1% BSA/PBS and incubated overnight at 2-8oC, in parallel with the antigen-containing wells. Because Ni-NTA plates from Qiagen are pre-blocked, the testing was carried out without the blocking step. The plate-bound antigen was tested using mAbs and pAbs against H5 HA, depicted in List A. The antibodies diluted to 1 µg/mL in 2% BSA/PBS, were incubated on the coated plates overnight at 2-8oC. For the detection of antigen-bound mAbs, the HRP-labelled pAbs against mouse IgG, whole-molecule specific (Sigma-Aldrich), were used. For the detection of antigen-bound pAbs, the HRP-labelled mAbs against rabbit IgG, γ-chain specific (Sigma-Aldrich), were used. The secondary antibodies were diluted 1:1000 with 1% BSA/PBST and incubated on the plates for 30 min (polystyrene plates) or for 45 min (Ni-NTA plates) at room temperature. TMB (Sigma-Aldrich) was used as a substrate for HRP. The reaction was stopped with 0.5 M (polystyrene plates) or 1.25 M (Ni-NTA plates) solution of H₂SO₄. The absorptions of the samples were read at λ=450 nm.

The results from antigenicity studies of rHA - A/H5N1/Qinghai, performed on MediSorp, MaxiSorp and Ni-NTA plates using commercial anti-H5 HA antibodies, are shown in Fig. 2. The HA was recognized by all of the used antibodies, which leads to the conclusion that the antigen comprises well-preserved conformational epitopes of the H5 hemagglutinin HA1 subunit as well as the fragment of the HA2 subunit. Moreover, the obtained results show that the HA1 subunit of rHA - A/H5N1/Qinghai contain the properly folded epitopes recognized by HI antibodies. This indicates the protein potential to induce hemagglutination-inhibiting antibodies.

### Oligomerization of rHA - A/H5N1/Qinghai

The test for the presence of oligomeric forms in the rHA - A/H5N1/Qinghai preparation was performed by the sandwich-type ELISA method, using the same clone of mAb (B513M), designated as mAb 8 (Acris Antibodies), for coating and developing the microtiter plates. Detecting antibodies were labeled with HRP by a service company (Acris Antibodies).

To perform the test, the MaxiSorp plates (NUNC) were coated with mAb 8 (Acris Antibodies, Cat. No. AM00941PU-N) in PBS (1 µg/mL) overnight at 2-8oC. The non-specific binding sites on the plate were blocked with 2% BSA/PBS. Next, the samples of rHA - A/H5N1/Qinghai protein, prepared as a series of 2-fold dilutions in 2% BSA/PBS ranging from 10 µg/mL to 0.009 ng/mL, and the BLK samples (2% BSA/PBS) to control the non-specific binding level, were applied to the plate. The plates were incubated overnight at 2-8oC. To detect oligomeric forms of HA protein, the HRP-labelled mAb 8 (Acris Antibodies, Cat. No. AM00941HR-Cus) was used. The antibodies were diluted 1:500 in 2% BSA/PBS and incubated on the plate for 1 h at 37oC. TMB (Sigma-Aldrich) was used as a substrate for HRP. The reaction was stpopped with 0.5 M solution of H₂SO₄. The absorptions of the samples were read at λ=450 nm.

The results of rHA - A/H5N1/Qinghai study for the presence of oligomers are presented in Fig. 3. The performed test showed that the protein preparation contains oligomeric forms, and this is consistent with specification.

### Hemagglutination activity of rHA - A/H5N1/Qinghai

The rHA - A/H5N1/Qinghai protein, for which the oligomeric forms were found by ELISA, was subjected to an additional study for the presence of functional oligomers using the hemagglutination test. The test utilizes the ability of the viral protein to agglutinate erythrocytes by binding with their surface receptors. Evaluation of the erythrocyte agglutination ability is commonly used in the quality examinations of vaccine antigens.

In the HA test, the preparation of fresh erythrocytes from the SPF chicken blood, obtained from the sterile culture at the Department of Poultry Diseases (DPD) of the National Veterinary Research Institute (NVRI) in Pulawy (Poland), was used. The A/turk/It/80(H5N2) LPAIV (x-OvO), certified by the Istituto Zooprofilattico Sperimentale delle Venezie (IZSVe) in Legnaro (Italy), was used as a positive control. In the test, the erythrocyte control (internal control for the assay), which did not contain the viral antigen was included. The test was performed using 96-well conical bottom (V) plates (CellStar/Greiner bio-one).

To evaluate the hemagglutination activity of rHA - A/H5N1/Qinghai, 50 µl of PBS-Dulbecco (Sigma-Aldrich) was pipetted into each well of the plate followed by the addition of the adequate amount of the antigen and a buffer to a final volume of 100 µL. In this way, the samples containing the rHA amounts from 0.1 µg to 10 µg were prepared. The positive control was prepared by serial dilutions of the suspension containing 4 hemagglutination units (HAU) of the H5N2 LPAIV in PBS-Dulbecco. A buffer only (PBS-Dulbecco) was added into the wells intended for the use as an erythrocyte control. Next, 50 µL of 1% erythrocyte suspension in PBS-Dulbecco was added into each well and the contents of the wells were gently mixed by pipetting. The plate was incubated for 45 min at room temperature and then the results were read. According to the principle of the test, the hemagglutination effect was visually evaluated by a comparison of the samples with erythrocyte control. In contrary to the control erythrocytes, the agglutinated erythrocytes do not settle on the well bottoms but form a unitary suspension.

The hemagglutination test, performed using chicken erythrocytes, showed that the rHA - A/H5N1/Qinghai has the ability to evoke hemagglutination, similarly as the H5N2 LPAIV. The hemagglutination effect was observed within the whole tested range of antigen concentrations, also in the samples containing 0.1 µg of the protein. The results indicate that the rHA - A/H5N1/Qinghai forms functional oligomers, which is the desired feature of the vaccine HA. This also applies to the HA proteins used as immunogens in the mAb production procedure by the hybridoma method.

### Summary

The results of the performed studies indicate that the rHA - A/H5N1/Qinghai protein shares native-antigen characteristics. It preserves conformational epitopes recognized by anti-H5 HA antibodies, including the HI antibodies, forms oligomeric structures, as well as has the cability of binding to cell-surface receptors and agglutinate erythrocytes. This leads to the conclusion that the rHA - A/H5N1/Qinghai protein is a valuable immunogen, thus justifying its use in the production procedure of the anti-H5 HA mAbs by the hybridoma method.

### Example 3

### Specificity and isotype determinations

Besides the use of valuable immunogen, another condition for generating mAbs of high diagnostic value is the use of an adequate procedure for the selection of the serotype-specific antibodies. For this purpose, the tests using the HA proteins preserving the native antigen conformation were developed. Two antigens were chosen for a preliminary selection of hybridomas producing anti-H5 HA antibodies. One of them was the rHA - A/H5N1/Qinghai protein intended for animal immunization. The properties of rHA - A/H5N1/Qinghai protein, indicating its usefulness as the immunogen, but also as the antigen for mAb selection, were described in detail in Example 2. Another antigen was the 17-530-aa (ΔRRRKKR, 6x His) protein with the HA sequence from the A/swan/Poland/305-135V08/2006(H5N1) AIV strain, produced in a baculovirus expression system (OET). Similarly as the rHA - A/H5N1/Qinghai, recombinant HA protein from a baculovirus expression system, described hereinafter as rHA - A/H5N1/Poland, was a glycosylated protein.

The amino acid sequence of rHA - A/H5N1/Poland protein was very similar to the immunogen's sequence. Using the BLAST program, it was shown that the homology between hemagglutinins of the A/swan/Poland/305-135V08/2006(H5N1) and A/Bar-headed Goose/ Qinghai/ 12/05(H5N1) strains, measured as a percentage of amino acid sequence identity, is 99%. One conserved change of amino acid is found in the signal sequence, and the other three changes are semi-conserved and are found in the HA1 and HA2 subunits of the protein. The usefulness of rHA - A/H5N1/Poland for verification of antibody specificity against H5 HA was evaluated by performing studies on the antigenicity and oligomerization degree of the protein and its hemagglutination ability.

### Properties of rHA - A/H5N1/Poland

The rHA - A/H5N1/Poland (OET) protein was analyzed using a MALDI TOF/TOF mass spectrometer (4800 Plus, AB SCIEX) according to the procedure described in Example 2 for the rHA - A/H5N1/Qinghai protein. The determined molecular weight of the antigen (OET, batches 2-8) was 64 kDa (Fig. 4). Due to glycosylation, it was by ~6 kDa higher than the mass calculated from the amino acid composition using the GPMAW 8.2 software (Lighthouse). The difference between experimentally and theoretically determined molecular weights of the rHA - A/H5N1/Poland was lower than in the case of rHA - A/H5N1/Qinghai (~6 kDA *vs* ~18 kDa), which indicates the difference in the glycosylation levels of rHAs produced in baculovirus and mammalian expression systems.

In the antigenicity studies of rHA - A/H5N1/Poland, the panel of commercially available mAbs and pAbs against H5 HA (List A), described in Example 2, was used. The study was performed by ELISA using polystyrene plates of various hydrophilicity (NUNC) and Ni-NTA plates (Qiagen) in the same way as the antigenicity studies of rHA - A/H5N1/Qinghai (Example 2). Fig. 5 shows the results of rHA - A/H5N1/Poland antigenicity studies (OET, batch 2) conducted using MediSorp, MaxiSorp and Ni-NTA plates. Reactivities of mAb 2, pAb 1 and pAb 2 with rHA - A/H5N1/Poland bound to the Ni-NTA plate were significantly lower than with rHA - A/H5N1/Qinghai (Fig. 2). This was probably the result of the difference in the binding and/or the presentation of the antigens on the plate. Considering reactivities of mAbs and pAbs with HA antigen from a baculovirus expression system bound to the various-type plates (Fig. 5), it can be concluded that the protein contains well preserved conformational epitopes of the HA1 subunit, including the epitopes recognized by HI antibodies. Antibody reactivity studies were performed for each of the used antigen batch. The results of these studies each time showed the proper conformation of the HA1 subunit in the rHA - A/H5N1/Poland antigen.

The oligomerization status of rHA - A/H5N1/Poland (OET, batches 5, 8) was examined using a sandwich ELISA with non-labelled and HRP-labelled comercial antibodies against H5 HA, desribed in List A as mAb 8 (Acris Antibodies, Cat.No. AM00941PU-N, AM00941HR-Cus). Recombinant HA was tested as a series of 2-fold dilution in the range of 10 µg/mL - 0.009 ng/mL. The test method was described in Example 2. The results shown in Fig. 3 indicate that the rHA - A/H5N1/Poland protein contains significantly less oligomeric forms than the rHA - A/H5N1/Qinghai antigen. Moreover, the inter-batches differences in the oligomerization state were observed for the protein of baculovirus-expression system origin.

The rHA - A/H5N1/Poland protein (OET, batch 5) was also subjected to the studies aimed at assessment the protein's capability of erythrocyte agglutination. The HA protein produced in a baculovirus expression system was tested simultaneously with the rHA from a mammalian expression system (rHA - A/H5N1/Qinghai). The hemagglutination test method was described in Example 2. The test, performed using chicken erythrocytes, showed that the rHA - A/H5N1/Poland has the hemagglutination ability. The full hemagglutination effect was observed in the samples containing from 10 µg to 2.5 µg of the antigen. The partial hemagglutination was observed in the samples containing 1 µg of the antigen. For erythrocyte agglutination, the significantly higher concentration of rHA - A/H5N1/Poland than of rHA - A/H5N1/Qinghai was needed (Example 2). This is consistent with the results of ELISA for testing the oligomerization state of HA proteins (Fig. 3). The results of the hemagglutination test indicate that the rHA - A/H5N1/Poland exists partially in the form of oligomers responsible for erythrocyte agglutination. However, the content of functional oligomers in the rHA - A/H5N1/Poland antigen is significantly lower than in the rHA - A/H5N1/Qinghai antigen (Example 2).

In summary, the rHA - A/H5N1/Poland from a baculovirus expression system preserves conformational epitopes recognized by anti-H5 HA antibodies, including the HI antibodies. In comparison to the rHA - A/H5N1/Qinghai, this protein is characterized by the lower glycosylation level and a small degree of oligomerization, which is an important feature of the viral HA. The conformation accuracy of the HA1 subunit of the antigen from a baculovirus expression system justifies its use in testing the specificity of the generated antibodies against the H5 HAs in parallel with the mammalian expression system-derived antigen. Different presentation of the HA1 subunit epitopes in the rHA - A/H5N1/Poland than in the rHA - A/H5N1/Qinghai was found to be beneficial for the effectiveness of the anti-HA mAb selection.

### ELISA tests for determining the specificity of the obtained mAbs

The specificity of the produced antibodies was determined by the ELISA method. For this purpose, the tests were developed using the previously characterized rHA - A/H5N1/Qinghai and rHA - A/H5N1/Poland proteins. The test optimization relied mainly on the selection of plates for the binding of particular antigens. The primary criterion for the test plate selection was the availability of important conformational epitopes in the plate-bound protein, assessed using commercially available anti-H5 HA mAbs and pAbs (List A). At the stage of test optimization, all of the types of protein immobilization surfaces offered by NUNC (PolySorp, MediSorp, MaxiSorp, MultiSorp), as well as the Ni-NTA plates (Qiagen) were used. The antigen adsorbtion on the polystyrene plates takes place by different protein fragments, mainly in a random manner, whereas the antigen immobilization on the Ni-NTA plates occurs in an oriented manner. The tests for optimization of the antibody specificity assays were performed under the same conditions as those used in the antigenicity studies of rHA - A/H5N1/Qinghai (Example 2) and rHA - A/H5N1/Poland (Example 3).

Using polystyrene plates of different hydrophilicity, it was shown that the use of MediSorp and MaxiSorp plates for antigen binding, especially the rHA - A/H5N1/Poland, provides better sensitivity of antibody determination than the use of the other types of polystyrene plates (data not shown). It was also shown that reactivities of commercial antibodies with rHA - A/H5N1/Qinghai are the highest after binding to the Ni-NTA plate (Fig. 2), while with rHA - A/H5N1/Poland after binding to the MediSorp plate (Fig. 5). The differences in the antigen presentations, which depend on the plate type being used, were found using mainly pAb 1 and pAb in the case of rHA from a mammalian expression system and mAb 2 in the case of rHA from a baculovirus expression system. In ELISAs for the determinations of the generated antibody specificities, the antibodies against mouse IgG, γ-chain specific, were used. The use of highly specific secondary antibodies in the developed tests limited the selection of the generated anti-H5 HA antibodies to the H5-specific antibodies of the IgG class. In diagnostic tests, they are the most useful among the various-isotype antibodies, due to their usually high affinity for the antigen.

Eventually, the specificity of antibodies was determined by ELISA using Ni-NTA plates (Qiagen) for coating with H5 rHAs from a mammalian expression system and MediSorp plates (NUNC) for coating with H5 rHA from a baculovirus expression system. Ni-NTA plates were coated with rHA - A/H5N1/Qinghai protein diluted in 1% BSA/PBS to a concentration of 1 µg/mL. MediSorp plates were coated with rHA - A/H5N1/Poland protein in PBS at concentrations of 6.2 µg/mL or 5 µg/mL. To control the level of non-specific antibody binding, some of the wells were filled with 1% BSA/PBS (Ni-NTA plates) or PBS (MediSorp plates) and incubated overnight at 2-8oC, in parallel with the antigen-containing wells. Because the Ni-NTA strips from Qiagen are pre-blocked, the testing was carried out without the blocking stage. The non-specific binding sites on the MediSorp plates were blocked with 10% FBS/PBS. The culture supernatants from the non-cloned hybridomas were analyzed at a dilution of 1:20 (Ni-NTA plates coated with rHA - A/H5N1/Qinghai) or 1:10 (MediSorp plates coated with rHA - A/H5N1/Poland). After hybridoma cloning, the culture supernatants were analyzed without dilution. Commercial anti-H5 HA antibodies diluted to a concentration of 0.01 µg/mL in PBS, were used as positive controls (mAb 8, List A). RPMI buffer was the control for non-specific binding of secondary antibodies (BLK sample). The plates with the tested and control samples were incubated overnight at 2-8oC. For the detection of antigen-bound mAbs, the HRP-labelled anti-mouse IgG antibodies, γ-chain specific (Sigma-Aldrich), were used. The secondary antibodies, pre-diluted 1:1000 in 2% BSA/PBS, were incubated on the plates for 1 h at 37oC. TMB (Sigma-Aldrich) was used as a substrate for HRP. The reaction was stopped with 1.25 M (Ni-NTA strips) or 0.5 M (MediSorp plates) solution of H₂SO₄. The absorptions of the samples were read at λ=450 nm.

### Isotyping of the obtained antibodies

Classes and subclasses of the obtained mAbs were determined using a "Mouse Monoclonal Antibody Isotyping Reagents" - ISO-2 isotyping kit (Sigma-Aldrich). MaxiSorp plates (NUNC) were coated with antibodies against the mouse antibodies of various classes and subclasses (IgG1, IgG2a, IgG2b, IgG3, IgA, IgM) at 1:1000 dilution in PBS, wherein the incubation was carried out for 1 h at 37oC or overnight at 2-8oC. On the coated plates, the non-diluted or diluted samples from hybridoma culture supernatants were incubated (1 h, 37oC). The control sample was PBS or RPMI (BLK sample). For the detection of antibodies captured by the isotype-specific antibodies, the HRP-labelled antibodies against mouse IgG antibodies, specific against the whole molecule (1:1000) or the Fab fragment (1:50 000), diluted in 1% BSA/PBST, were used. The plates with the diluted antibodies were incubated for 30 min at room temperature. TMB (Sigma-Aldrich) was used as a substrate for HRP. The reaction was stopped with 0.5 M H₂SO₄ solution. The absorptions of the samples were read at λ=450 nm. The described procedure is a modified version of manufacturer's instruction for ISO-2 kit. The introduced changes were related to the conditions of sample incubation on the plate, as well as to the antibodies and substrate used to develop the plates.

### Example 4 Analysis of serotype specificity

Development of the immunological tests, which can be used to detect infection with H5-serotype IVs, including the H5N1 AIVs, in animals and humans, requires the use of mAbs having a broad range of specificities against H5 HAs. Therefore, the antibodies specific against HAs with amino acid sequences identical or very similar to the immunogen's sequence, were further selected for serotype specificity. For this purpose, the tests were developed using different forms of the H5 HA antigens (positive selection) and HAs of serotypes other than H5 (negative selection). For the positive selection, recombinant H5 HA proteins based on the ectodomain (rHA) or HA1 subunit (rHA1) of hemagglutinin, having a varied homology to the immunogen, and the H5-serotype AIVs (H5N1, H5N2, H5N3 and H5N9) were used. The negative selection was performed using AIVs of fifteen serotypes other than H5 (H1-H4 and H6-H16).

### Recombinant H5 HA proteins

The antigens used in the serotype specificity studies, described as the ectodomain-based HA proteins, corresponded to the H5 HA, from which a signal sequence, transmembrane and cytoplasmic domains were removed. In the panel of HA proteins, there were included also the antigen fragments corresponding to the HA1 subunit, which is characterized by the greater sequence variability than the HA2 subunit and contains serotype-specific epitopes. The use of antigens, described as the HA1 subunit-based HA proteins, increased the probability of finding clones specific against H5-serotype HAs among the selected antibodies. Recombinant HA proteins based on the ectodomain (rHA) and HA1 subunit (rHA1) of the antigen, were produced in a mammalian expression system (ITC). The selection criterion of the proteins for the serotype specificity studies of the produced antibodies was diversity of the homology to the immunogen (rHA - A/H5N1/Qinghai). Studies on the specificity of antibodies against H5 HAs with varied homology were carried out simultaneously to the studies on the specificity towards antigens having the amino acid sequences identical or significantly similar to the immunogen's sequence, which were generated in mammalian (rHA - A/H5N1/Qinghai) and baculovirus (rHA - A/H5N1/Poland) expression systems. In total, recombinant proteins with HA sequences from nine IV strains of the H5 serotype, including the H5N1 (8 strains) and H5N2 (1 strain) IVs, were used in the serotype specificity analysis. Information regarding the antigens used in the specificity studies of the generated antibodies, including the serotype specificities, is presented below, in the List.
**List B** Recombinant hemagglutinin proteins used in the serotype specificity analysis of the generated antibodies.

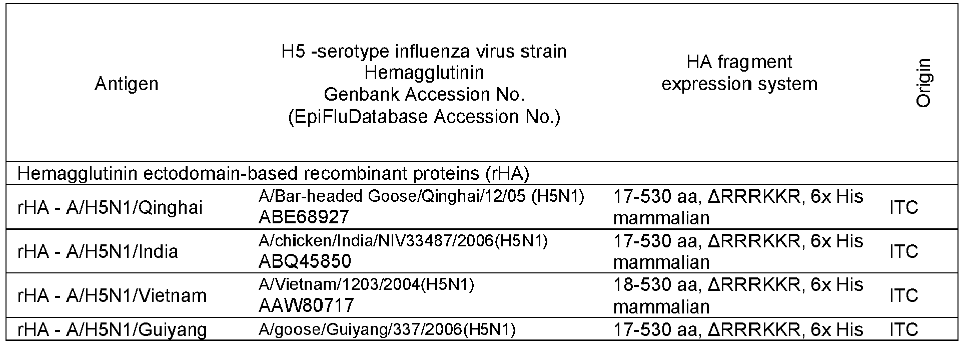

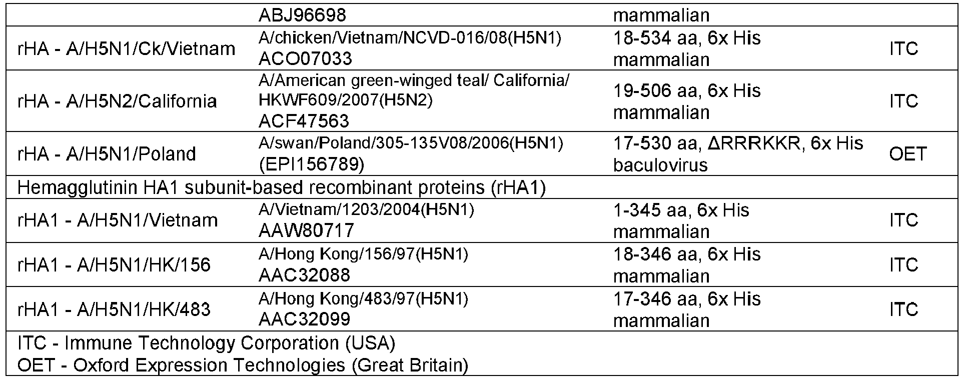

The ectodomain-based HA proteins (rHA) from a mammalian expression system were the antigen fragments containing the HA1 subunit and a part of the HA2 subunit, expressed without a signal sequence (17-530 aa, 18-530 aa, 18-534 aa, 19-506 aa). In four out of six HA antigens, the cleavage sites (ΔRRRKKR) were removed and, according to the specifications, these proteins existed largely in the form of trimers/oligomers. The HA1 subunit-based recombinant HA proteins (rHA1) were expressed in mammalian cells with (1-345 aa) or without (18-346 aa, 17-346 aa) a signal sequence. Conformation of the rHA1 proteins was not specified. All of the recombinant HA proteins contained 6x His tags. The purities of the antigens were at least 95%. In electrophoregrams of the purified antigens, the rHA and rHA1 proteins from a mammalian expression system are present as the single bands having the molecular weights of ~75 kDa and ~50 kDa, respectively.

The usefulness of the rHA and rHA1 antigens, depicted in List B, for testing the serotype specificity of the obtained antibodies was evaluated by performing the tests for antigenicity and oligomerization state. The method for testing the antigenicity of rHA - A/H5N1/Qinghai and rHA - A/H5N1/Poland was presented in Examples 2 and 3, respectively. As in the case of rHA - A/H5N1/Qinghai and rHA - A/H5N1/Poland, the antigenicity studies of the rHA and rHA1 were performed using commercially available antibodies against H5 HA. The panel of antibodies included the mAbs recognizing the native HA, the mAbs displaying properties of serotype-specific antibodies as well as the mAbs active in the HI test. Commercial mAbs and pAbs used for the HA protein characterization are depicted in List A and described in detail in Example 2.

The antigenicity studies of the rHA proteins with the sequences of the A/H5N1/lndia, A/H5N1/Vietnam, A/H5N1/Guiyang, A/H5N1/Ck/Vietnam, A/H5N2/California strains, as well as of the rHA1 proteins with the sequences of the A/H5N1/Vietnam, A/H5N1/HK/156 and A/H5N1/HK/483 strains were performed by ELISA using Ni-NTA plates (Qiagen). The HA proteins, diluted in 1% BSA/PBS to a concentration of 1 µg/mL were applied to the plates. To control the level of non-specific antibody binding, some of the wells were filled with 1% BSA/PBS and incubated overnight at 2-8oC, in parallel with the antigen-containing wells. In the antigenicity studies of seven out of eight recombinant H5 HA proteins, mentioned above, all of the anti-H5 HA mAbs and pAbs from the List A were used. The antibodies diluted to a concentration of 1 µg/mL in 2% BSA/PBS, were incubated on the coated plates overnight at 2-8oC. To develop the plates, the HRP-Iabelled, pAbs against mouse IgG, whole molecule-specific (Sigma-Aldrich), or the HRP-labelled monoclonal antibodies against rabbit IgG, γ-chain specific (Sigma-Aldrich), were used. The secondary antibodies were diluted 1:1000 in 1% BSA/PBST and incubated on the plates for 45 min at room temperature. Antigenicity study of rHA1 - A/H5N1/HK/156 was performed using antibodies, described as mAb 8, diluted to a concentration of 0.1 µg/mL in 2% BSA/PBS. The antigen-bound mAbs were detected using the HRP-labelled pAbs against mouse IgG, whole molecule-specific (Sigma-Aldrich), which were diluted 1:1000 in 2% BSA/PBS and incubated on the plate for 60 min at 37oC. TMB (Sigma-Aldrich) was used as a substrate for horseradish peroxidase. The reaction was stopped with 1.25 M H₂SO₄ solution. The absorptions of samples was read at λ=450 nm. The ELISA test was not optimized for the individual antigens and antibodies.

The results of antigenicity studies of rHA proteins with the sequences of the A/H5N1/India, A/H5N1/Vietnam, A/H5N1/Guiyang, A/H5N1/Ck/Vietnam, A/H5N2/California strains, as well as of rHA1 - A/H5N1/Vietnam and rHA1 - A/H5N1/HK/483 proteins are presented in Fig. 6. The rHA and rHA1 proteins are included in order of decreasing homology to the immunogen (rHA - A/H5N1/Qinghai). Amino acid sequence identities of the HA1 subunits in the rHA antigens were from 99% to 88%, and of the rHA1 antigens 95% and 94%. As shown in Fig. 6, six among the seven recombinant HA proteins formed detectable immunological complexes with monoclonal antibodies. Individual proteins were recognized by a specified number of commercial mAb clones with similar or varied affinity. The rHA - A/H5N1/lndia protein was recognized by all of the monoclonal antibodies (8/8) with high affinity, similarly as the rHA - A/H5N1/Qinghai (Fig. 2). The rHA - A/H5N1/Vietnam and rHA- A/H5N2/California proteins were recognized with high affinity by six, the rHA1- A/H5N1/Vietnam protein by five, and the rHA - A/H5N1/Guiyang and rHA1 - A/H5N1/HK/483 proteins by four out of eight commercial monoclonal antibodies used. Although with lowered affinity, the rHA - A/H5N1/Guiyang and rHA1 - A/H5N1/HK/483 proteins were also recognized by antibodies, referred to as mAb3. The above-described H5 HA ectodomain-based antigens (rHAs) were detected by polyclonal antibodies. The observed diversity of the HA protein antigenicity profiles could be related to the HA1 subunit variability and a different specificity range of the used commercial monoclonal antibodies. Additionally, the different presentation of the rHA and rHA1 antigens and the lower sensitivity of the test using the rHA1 than rHA proteins could affect the results obtained for the ectodomain- and HA1 subunit-based proteins.

Antigenicity studies of rHA1 - A/H5N1/HK/156 protein with the use of mAb 8 having a broad-range specificity against H5 HAs (Fig. 6) showed that the protein is recognized by these antibodies with high affinity (data not shown). In turn, the antigenicity studies of rHA - A/H5N1/Ck/Vietnam protein with the use of all of the mAbs and pAbs against H5 HA presented in List A showed that the protein is recognized only by polyclonal antibodies: pAb 1 and pAb 2 (Fig. 6). None of the eight monoclonal antibodies bound to this antigen. Considering the specificity profiles of monoclonal antibodies used in the studies, it can be concluded that the rHA - A/H5N1/Ck/Vietnam does not have a proper conformation in contrast to the other recombinant HA proteins. The results from antigenicity studies of rHA - A/H5N1/Qinghai and rHA - A/H5N1/Poland, indicating that these proteins preserve the features of the native HA conformation, are described in Examples 2 and 3, respectively.

The results from antigenicity studies of recombinant HA proteins, presented in Fig. 2, 5 and 6, indicate that nine out of ten recombinant HA proteins (List B) with varied homology to the immunogen, including the ectodomain- (6 proteins) and HA1 subunit- (3 proteins) based hemagglutinin antigens, have features of the viral HA structure. The performed studies justify the use of all of the tested HA proteins in the selection procedure for the generated antibodies. The use of the HA1 subunit-based proteins enabled the selection of mAbs against conformational epitopes in the HA1 subunit, whereas the use of the rHA - A/H5N1/Ck/Vietnam allow to distinguish between antibodies recognizing conformational and non-conformational epitopes of the H5 HA.

The studies for the presence of oligomeric forms in the rHA and rHA1 antigens were performed by a sandwich ELISA, identically as in the oligomerization studies of rHA - A/H5N1/Qinghai and rHA - A/H5N1/Poland proteins. The testing conditions were described in Example 2. The results for ten recombinant H5 HA proteins from the List B are presented in Fig. 3. The oligomerization degrees of rHA - A/H5N1/lndia and rHA - A/H5N1/Vietnam proteins were comparable to the one observed for the rHA - A/H5N1/Qinghai. A slightly lower degree of oligomerization was shown for the rHA - A/H5N2/California protein. The rHA - A/H5N1/Guiyang antigen contained significantly less oligomeric forms than the other rHA proteins from a mammalian expression system, but more than both batches of rHA - A/H5N1/Poland from a baculovirus expression system. No oligomeric forms were found in the non-conformational rHA - A/H5N1/Ck/Vietnam antigen. This confirms the results from antigenicity studies of this protein with antibodies, referred to as mAb 8 (Fig. 6), which found use in the oligomerization degree testing. ELISA for testing the oligomerization degree of HA proteins confirmed that the rHA1 antigens exist in the monomeric forms. A slight degree of oligomerization was found only for the rHA1 - A/H5N1/Vietnam protein, which contained a signal sequence, in contrast to the rHA1 - A/H5N1/HK/156 and rHA1 - A/H5N1/HK/483 proteins.

### Influenza viruses of H1-H16 serotypes

AIVs were used in the analysis of serotype specificity of the generated antibodies. The viruses (x-OvO) had certificates from IZSVe. The used AIVs are presented in the List below.

**List C Avian influenza viruses used in the serotype specificity studies of the generated antibodies.**

| Hemagglutinin serotype | Avian influenza virus serotype | Strain | Origin |
|---|---|---|---|
| H1 | H1N1 | A/duck/lt/1447/05(H1N1) | x-OvO |
| H2 | H2N3 | A/duck/Germ/1215/73(H2N3) | x-OvO |
| H3 | H3N8 | A/pass/It/6000/V00(H3N8) | x-OvO |
| | | A/psitt/It/2873/00(H3N8) | x-OvO |
| H4 | H4N8 | A/cockatoo/Eng/72(H4N8) | x-OvO |
| H5 | H5N1 | A/mallard/lt/3401/05(H5N1) | x-OvO |
| | H5N2 | A/turk/lt/80(H5N2) | x-OvO |
| | H5N3 | A/duck/lt/775/04(H5N3) | x-OvO |
| | H5N9 | A/ck/lt/22A/98(H5N9) | x-OvO |
| H6 | H6N2 | A/turkev/Canada/65 (H6N2) | x-OvO |
| H7 | H7N1 | A/ck/lt/1067/V99(H7N1) | x-OvO |
| | H7N3 | A/ty/lt/9289/V02(H7N3) | x-OvO |
| | H7N4 | A/mallard/It/4810-79/04(H7N4) | x-OvO |
| | H7N7 | A/macaw/626/80(H7N7) | x-OvO |
| H8 | H8N4 | A/turk/Ont/6118/68(H8N4) | x-OvO |
| H9 | H9N2 | A/ty/Wis/66(H9N2) | x-OvO |
| | H9N7 | A/turk/Scotland/1/70(H9N7) | x-OvO |
| H10 | H10N1 | A/ostrich/SA/01(H10N1) | x-OvO |
| H11 | H11N6 | A/duck/Eng/56(H11N6) | x-OvO |
| | H11N9 | A/duck/Memphis/546/174(H11N9) | x-OvO |
| H12 | H12N5 | A/duck/Alberta/60/76(H12N5) | x-OvO |
| H13 | H13N6 | A/gull/Maryland/704/77(H13N6) | x-OvO |
| H14 | H14N5 | A/mallard/Gurjev/263/82(H14N5) | x-OvO |
| H15 | H15N9 | A/shearwater/2576/79(H15N9) | x-OvO |
| H16 | H16N3 | A/gull/Denmark/68110/02(H16N3) | x-OvO |
| x-OvO Limited (Great Britain), Istituto Zooprofilattico Sperimentale delle Venezie (IZSVe, Italy) | | | |

Twenty-five AIV strains were used in the serotype specificity analysis of the antibodies, including four of the H5 serotype, and the remaining of the H1, H2, H3, H4, H6, H7, H8, H9, H10, H11, H12, H13, H14, H15 and H16 serotypes.

### Homology of the H5 HA antigens

In the serotype specificity analysis of the generated antibodies, hemagglutinins or their fragments having the sequences of thirteen IV strains of the H5 serotype (Lists B and C) were used. This number included the H5N3 (1 strain), H5N9 (1 strain), H5N2 (2 strains) IVs and, above all, the H5N1 viruses (9 strains), classified into six different clades, presented in the list below.

**List D Classification of the H5N1 influenza viruses, from which the sequences of the H5 HA antigens were derived.**

| H5-serotype influenza virus strain | Hemagglutinin Genbank Accession No. (EpiFluDatabase Accession No.) | Clade H5N1 IVs |
|---|---|---|
| A/Hong Konq/156/97(H5N1) | AAC32088 | 0 |
| A/Hong Kong/483/97(H5N1) | AAC32099 | 0 |
| A/Vietnam/1203/2004(H5N1) | AAW80717 | 1 |
| A/Bar-headed Goose/Qinqhai/12/05(H5N1) | ABE68927 | 2.2 |
| A/chicken/lndia/NIV33487/2006(H5N1) | ABQ45850 | 2.2 |
| A/swan/Poland/305-135V08/2006(H5N1) | (EPI156789) | 2.2 |
| A/goose/Guivang/337/2006(H5N1) | ABJ96698 | 4 |
| A/chicken/Vietnam/NCVD-016/08(H5N1) | ACO07033 | 7 |
| A/mallard/ltaly/3401/2005(H5N1) | ABG57086.1 | EA-nonGsGD |
| EA-nonGsGD - H5-serotype virus, more similar to the low pathogenic viruses of Eurasian origin | | |

The homology of the hemagglutinins, from which the sequences of the H5 HA antigens were derived, was determined against the 1-567-aa (full-length protein) and 17-338-aa (HA1 subunit) fragments of the HA from the A/Bar-headed Goose/Qinghai/60/05(H5N1) strain, which was the sequence source for the immunogen (rHA - A/H5N1/Qinghai). The sequences were analyzed using the BLAST program. The relative homologies of the full-length hemagglutinins are shown in List E, while of the HA1 subunits of the proteins are shown in List F. Hemagglutinins of different IV strains are included in decreasing Max Score order.

**List E The homology of the full-length hemagglutinins, from which the sequences of the H5 HA antigens were derived, against the hemagglutinin of the A/H5N1/Qinghai strain, determined using the BLAST program.**

| Sequences producing significant alignments: | | | | | | |
|---|---|---|---|---|---|---|
| Description | Max score | Total score | Query cover | E value | Ident | Accessionx |
| hemagglutinin [Influenza A virus (A/Bar-headed Goose/Qinghai/60/05(H5N1))] | 1186 | 1186 | 100% | 0.0 | 100% | ABE68927.1 |
| hemagglutinin [Influenza A virus (A/chicken/India/NIV33487/06(H5N1))] | 1181 | 1181 | 99% | 0.0 | 99% | ABQ45850.1 |
| hemagglutinin [Influenza A virus (A/swan/Poland/305-135V08/2006(H5N1))] | 1177 | 1177 | 99% | 0.0 | 99% | (EPI156789) |
| hemagglutinin HA [Influenza A virus (A/Viet Nam/1203/2004(H5N1))] | 1150 | 1150 | 99% | 0.0 | 97% | AAW80717.1 |
| hemagglutinin subtype H5 [Influenza A virus (A/Hong Kong/156/97(H5N1))] | 1142 | 1142 | 99% | 0.0 | 96% | AAC32088.1 |
| hemagglutinin subtype H5 [Influenza A virus (A/Hong Konq/483/1997(H5N1))] | 1135 | 1135 | 99% | 0.0 | 95% | AAC32099.1 |
| hemagglutinin [Influenza A virus (A/duck/ltaly/775/2004(H5N3))] | 1112 | 1112 | 99% | 0.0 | 93% | ABS89310.1 |
| hemagglutinin [Influenza A virus (A/goose/Guiyang/337/2006(H5N1))] | 1100 | 1100 | 98% | 0.0 | 94% | ABJ96698.1 |
| hemagglutinin [Influenza A virus (A/chicken/ltaly/22A/1998(H5N9))] | 1089 | 1089 | 99% | 0,0 | 91 % | ABR37720.1 |
| hemagglutinin [Influenza A virus (A/American green-winged teal/California/HKWF609/2007(H5N2))] | 1082 | 1082 | 99% | 0.0 | 89% | ACF47563.1 |
| hemagglutinin [Influenza A virus (A/turkey/ltaly/1980(H5N2))] | 1067 | 1067 | 98% | 0.0 | 91% | ACS93985.1 |
| hemagglutinin [Influenza A virus (A/chicken/Vietnam/NCVD-016/2008(H5N1))] | 1066 | 1066 | 99% | 0.0 | 91% | ACO07033.1 |
| hemagglutinin [Influenza A virus (A/mallard/Italy/3401/2005(H5N1))] | 1058 | 1073 | 96% | 0.0 | 93% | ABG57086.1 |
| xGenbank (EpiFluDatabase) Accession No. | | | | | | |

**List F The homology of the sequences forming the HA1 subunit of the H5 HA antigens, against the HA1 subunit of hemagglutinin from the A/H5N1/Qinghai strain, determined using the BLAST program.**

| Sequences producing significant alignments: | | | | | | |
|---|---|---|---|---|---|---|
| Description | Max score | Total score | Query cover | E value | Ident | Accessionx |
| hemagglutinin [Influenza A virus (A/Bar-headed Goose/Qinghai/60/05(H5N1))] | 679 | 696 | 100% | 0.0 | 100% | ABE68927.1 |
| hemagglutinin [Influenza A virus (A/chicken/India/NIV33487/06(H5N1))] | 677 | 694 | 100% | 0.0 | 99% | ABQ45850.1 |
| hemagglutinin [Influenza A virus ⁽A/swan/Poland/305-135V08/2006(H5N1))] | 677 | 693 | 100% | 0.0 | 99% | (EPI156789) |
| hemagglutinin HA [Influenza A virus (A/Viet Nam/1203/2004(H5N1))] | 654 | 685 | 100% | 0.0 | 95% | AAW80717.1 |
| hemagglutinin subtype H5 [Influenza A virus (A/Hong Kong/156/97(H5N1))] | 652 | 668 | 100% | 0.0 | 95% | AAC32088.1 |
| hemagglutinin subtype H5 [Influenza A virus (A/Hong Kong/483/1997(H5N1))] | 646 | 664 | 100% | 0.0 | 94% | AAC32099.1 |
| hemagglutinin [Influenza A virus (A/duck/Italy/775/2004(H5N3))] | 636 | 652 | 100% | 0.0 | 93% | ABS89310.1 |
| hemagglutinin [Influenza A virus (A/goose/Guiyang/337/2006(H5N1))] | 635 | 650 | 100% | 0.0 | 93% | ABJ96698.1 |
| hemagglutinin [Influenza A virus (A/mallard/Italy/3401/2005(H5N1))] | 633 | 649. | 100% | 0.0 | 93% | ABG57086.1 |
| hemagglutinin [Influenza A virus (A/chicken/Italy/22A/1998(H5N9))] | 619 | 636 | 100% | 0.0 | 90% | ABR37720.1 |
| hemagglutinin [Influenza A virus (A/American green-winged teal/California/HKWF609/2007(H5N2))] | 616 | 633 | 100% | 0.0 | 88% | ACF47563.1 |
| hemagglutinin [Influenza A virus (A/turkey/Italy/1980(H5N2))] | 615 | 631 | 100% | 0.0 | 90% | ACS93985.1 |
| hemagglutinin [Influenza A virus (A/chicken/Vietnam/NCVD-016/2008(H5N1))] | 605 | 623 | 100% | 0.0 | 89% | ACO07033.1 |
| xGenbank (EpiFluDatabase) Accession No. | | | | | | |

The full-length hemagglutinin homology search for twelve, H5-serotype IV strains against the HA from the A/H5N1/Qinghai strain showed significant variability of the proteins (List E), from which the sequences were derived to produce HA antigens that were used in the serotype specificity analysis of the generated antibodies. Because the serotype-specific epitopes are within the HA1 subunit of HA, the high variability of the sequences forming this subunit in the HA antigens, shown in the homology search (List F), is especially important for the effective selection of the monoclonal antibodies, desired in diagnostics. The antigens having the HA1 subunit of very high and relatively low homologies against the HA1 subunit of HA from the A/H5N1/Qinghai strain were included in the HA protein panel. For the proteins, which are the most similar to the immunogen, i.e., the rHA - A/H5N1/lndia and the rHA - A/H5N1/Poland, the homology scores of their HA1 subunit: Max Score, Total Score, Identities reached the values of 677, 694 and 693, and 99%, respectively. The same scores for the HA1 subunit in the A/H5N9/Ck/Italy AIV, rHA - A/H5N2/California, A/H5N2/Tk/Italy AIV and rHA-A/H5N1/Ck/Vietnam antigens reached the values of 619-605, 636-623 and 88-90%, respectively.

### ELISA tests for determining serotype specificity of the obtained mAbs

The clones specific for HAs having the amino acid sequences identical or very similar to the immunogen's sequence, i.e., for the rHA - A/H5N1/Qinghai and rHA - A/H5N1/Poland, were analyzed using the previously characterized rHA and rHA1 proteins of various homology against the immunogen, which were produced in a mammalian expression system (ITC). Because of the presence of histidine tags in the recombinant antigens, ELISAs were performed using Ni-NTA plates. Preferably for antibody detection, the oriented binding of the antigens to Ni-NTA plates provides availability of different antigen epitopes. The determinations of antibody specificities against AIVs were performed by ELISA using MaxiSorp plates (NUNC).The first supernatant analyses for the presence of antibodies against AIVs of the H5 serotype (x-OvO) were performed only after hybridoma cloning (Example 5). Full characterization of antibody specificities against AIVs of the H1, H2, H3, H4, H6, H7, H8, H9, H10, H11, H12, H13, H14, H15 and H16 serotypes (x-OvO), was performed only for the selected and purified clones of mAbs (Example 7). Similarly as in the tests for determining the antibody specificities against H5 HAs (Example 3), the highly specific anti-mouse antibodies were used as detecting antibodies in testing the antibody serotype specificities with the use of the rHA and rHA1 proteins and various AIV strains. Thus, the antibody selection was limited to the serotype-specific antibodies of the desired class, i.e., IgG.

### ELISAs using recombinant HA proteins

To perform ELISA using recombinant HA proteins, the rHA and rHA1 proteins, including the rHA-A/H5N1/Qinghai (ITC), were diluted in 1% BSA/PBS and then applied to Ni-NTA plates (Qiagen). Some of the plate wells were filled with 1% BSA/PBS and incubated overnight at 2-8oC, in parallel with the antigen-containing wells. The supernatants from the non-cloned hybridomas were analyzed without dilution on the plates coated with the rHA and rHA1 at a concentration of 1 µg/mL. After hybridoma cloning, the culture supernatants were analyzed without dilution and/or after dilution in PBS on the plates coated with the rHA and rHA1 at concentrations of 1 and 5 µg/mL, respectively. The samples for reactivity studies with rHAs having the sequences from the A/H5N1/Qinghai, A/H5N1/lndia, A/H5N1/Vietnam and A/H5N1/Guiyang strains were diluted 1:3000 or 1:3000 and 1:5000. For testing on the rHA - A/H5N2/California-coated plates, the supernatants were analyzed without dilution or, additionally, at 1:1500 and 1:3000 dilutions. The supernatant samples for reactivity studies with the remaining HA antigens were not diluted. To control the level of non-specific antibody binding, the samples prepared from hybridoma culture supernatants were additionally analyzed in non-coated wells of the plate. The assay was carried out in the presence of control samples. Commercial anti-H5 HA antibodies (mAb 8, List A) were the positive controls, whereas RPMI buffer was the negative control (BLK sample). The plates with tested and control samples were incubated overnight at 2-8oC. For the detection of antigen-bound mAbs, the HRP-labelled antibodies against mouse IgG, γ-chain specific (Sigma-Aldrich), were used. The secondary antibodies were diluted 1:1000 in 1% BSA/PBST or 2% BSA/PBS and incubated on the plates for 45 min at room temperature or for 60 min at 37oC. TMB (Sigma-Aldrich) was used as a substrate for HRP. The reaction was stopped with 1.25 M H₂SO₄ solution. The absorptions of the samples were read at λ=450 nm.

The results from reactivity studies of commercial mAbs and pAbs on the Ni-NTA plates coated with the rHA and rHA1 proteins, having the HA sequence from the A/H5N1/Vietnam strain, at concentrations of 1 µg/mL (Fig. 6) and 0.25-4 µg/mL (data not shown) indicated that the detectability of antibodies against the HA1 subunit is lower when the rHA1 is used for plate coating than in the case of the rHA usage and depends on the concentration of antibodies and their affinity for the antigen. The positive signals for the samples of hybridoma culture supernatants tested against the rHA and rHA1 clearly indicates that mAbs are specific against the HA1 subunit, whereas the positive signal for the rHA and the lack of a signal for the rHA1 does not necessary mean that mAbs target the HA2 subunit of the antigen. By increasing the rHA1 concentration for coating of the Ni-NTA plates from 1 µg/mL to 5 µg/mL, the sensitivity of the test for serotype specificity studies was increased (data not shown). The test with an increased sensitivity was used to analyze the samples of culture supernatants from hybridomas, after they were cloned.

### ELISAs using influenza viruses of H1-16 serotypes

To perform ELISAs, the AIV preparations (x-OvO) were diluted in PBS to 4000 HAU/mL based on the values specified by the manufacturer, and then applied to MaxiSorp plates (NUNC). The plates with wells containing viral antigens were incubated overnight at 2-8oC, in parallel with the PBS-filled wells. The efficiency of coating with individual viruses could vary due to the observed differences in the preparation compositions. After incubation, the plates were blocked with 2% BSA/PBS. The hybridoma culture supernatants were analyzed without dilution on the plates coated with H5-serotype AIVs (positive selection). The purified mAb clones were diluted in 2% BSA/PBS to 20 µg/mL and analyzed on the plates coated with AIVs of the H5 serotype (positive selection) and of the H1-H4 and H5-H16 serotypes (negative selection). To confirm the correctness of the testing with IVs of serotypes other than H5, a part of each plate for negative selection was coated with H5N3 and H5N9 viruses. The controls for non-specific antibody binding were the samples containing mAbs and the BLK samples (RPMI buffer and/or 2% BSA/PBS) applied to the antigen-coated and non-coated wells of the plate, respectively. The controls were commercial anti-H5 HA antibodies (mAb 8, List A), diluted in 2% BSA/PBS to concentrations of 0.05, 0.01, 0.005 and 0.001 µg/mL during testing of the culture supernatants and to 20 µg/mL during testing of the purified mAbs. The plates with the obtained mAbs and control samples were incubated overnight at 2-8oC. For the detection of antigen-bound mAbs, the HRP-labelled antibodies against mouse IgG, γ-chain specific (Sigma-Aldrich), were used. The secondary antibodies were diluted 1:1000 in 2% BSA/PBS and incubated on the plates for 1 h at 37oC. TMB (Sigma-Aldrich) was used as a substrate for HRP. The reaction was stopped with 0.5 M H₂SO₄ solution. The absorptions of samples were read at λ=450 nm.

### Summary

The screening of monoclonal antibodies for serotype specificity was performed by the ELISA method using the previously characterized H5 HA antigens (positive selection) and IVs of serotypes other than H5 (negative selection). For positive selection, recombinant H5 HA proteins based on the ectodomain (rHA) or HA1 subunit (rHA1) of hemagglutinin, described in List B, were used. Recombinant antigens differed in the amino acid sequences and properties. The individual rHA and rHA1 proteins showed characteristic profiles of recognition by commercial anti-HA antibodies (Fig. 2, 5, 6). Out of ten recombinant proteins, for which the antigenicity studies were conducted, the nine antigens, including six rHA and three rHA1 proteins, had features of the viral HA structure. The rHA1 antigens were in the form of monomers, whereas the rHA antigens were, at least partially, in the form of oligomers (Fig. 3), what makes them similar to the trimeric HA in the IV's envelope. The content of oligomeric forms in the rHA antigens was varied. Significantly different oligomerization and glycosylation levels were shown for the H5 HAs (17-530 aa, ΔRRRKKR, 6x His) with very similar amino acid sequences: the rHA - A/H5N1/Qinghai from mammalian and the rHA - A/H5N1/Poland from baculovirus expression systems (Examples: 2, 3). For the positive antibody selection, four certified AIV strains of the H5 serotypes: H5N1, H5N2, H5N3 and H5N9, were also used (List C). Thus, the antibody screening was performed using different forms of the H5 HA antigen with beneficially varied properties that were expected to affect the presentation of epitopes to antibodies, desired in diagnostics. The use of conformational rHA1 proteins enabled the identification of mAbs binding to the hemagglutinin HA1 subunit, while the use of a protein, which does not show native HA antigenicity (rHA - A/H5N1/Ck/Vietnam), allows for distinguishing between antibodies recognizing conformational and linear epitopes of the H5 HAs.

In the serotype specificity analysis of the generated antibodies, the hemagglutinins or their fragments showing native-antigen characteristics were used. They contained the HA sequences from twelve IV strains of the H5 serotype (Lists B and C). This number included the H5N3 (1 strain), H5N9 (1 strain), H5N2 (2 strains) IVs and the H5N1 viruses (8 strains), classified into five clades: 0, 1, 2.2, 4 and EA-nonGsGD (List D). The homologies of the HA1 subunit of the conformational HA antigens to the HA1 subunit of the immunogen (rHA - A/H5N1/Qinghai), measured as a percentage of identical amino acids in the sequence, were from 99% to 88% (List F). It could be assumed that the use of diverse forms of the H5 HA antigens with varied homology will enable evaluation of the serotype specificity of the novel mAb clones, similarly as the reactivity studies of commercial antibodies with recombinant H5 HA proteins revealed the specificity range of the individual clones against H5 hemagglutinins (Fig. 2, 5, 6). The negative selection was performed using twenty-one AIV strains representing HA serotypes other than H5. The H1-H4 and H6-H16 AIVs (List C) were certified, just as AIVs of the H5 serotype.

In summary, the strategy used for mAb selection provided a high probability of identification of antibody clones recognizing different epitopes specific for the H5 HAs, which would react with H5-serotype IVs belonging to different clades and subclades, and at the same time, would not cross-react with viruses of serotypes other than H5.

### Example 5 The results of antibody selection

As a result of using the procedure for mAb obtaining with the rHA - A/H5N1/Qinghai for mouse immunization, described in Example 1, 440 hybridomas were obtained, including 58 ones, which produced antibodies against H5 HA. Specificity determination was performed by ELISA using two antigens: rHA - A/H5N1/Qinghai from a mammalian expression system (ITC) and rHA - A/H5N1/Poland from a baculovirus expression system (OET). The testing conditions were described in Example 3. The hybridoma culture supernatants containing antibodies recognizing rHA - A/H5N1/Qinghai were subjected to further analysis using recombinant HA proteins based on the ectodomain (rHA) and HA1 subunit (rHA1) of hemagglutinin, which were produced in mammalian cells (ITC). The assay was performed as described in Example 4. The results indicating the conformation accuracy of the used HA proteins were presented in Examples 2, 3 and 4. The antigens contained the HA sequences of five H5N1 virus strains and one H5N2 virus strain. The antigen sequences were analyzed using the BLAST program by a comparison with the HA sequence from the A/Bar-headed Goose/Qinghai/60/05(H5N1) strain. The homologies of the full-length, source sequences for the HA antigens, expressed as a percentage of identical amino acids, were from 99% to 89%, and of the HA1 subunit-forming sequences from 99% to 88%. The results from the serotype specificity determinations of the obtained antibodies are presented in Table 1 included below.

In particular, Table 1 shows the results from immunoreactivity studies of antibodies produced by the non-cloned hybridomas. The samples collected from the hybridoma supernatants were tested by ELISA using recombinant proteins based on the ectodomain (rHA) and HA1 subunit (rHA1) of hemagglutinin. The rHA - A/H5N1/Poland protein was produced in a baculovirus expression system (OET, batch 1). The remaining rHA and rHA1 proteins originated from a mammalian expression system (ITC). For coating with HA antigens, MediSorp plates (rHA - A/H5N1/Poland) from NUNC and Ni-NTA plates (rHA and rHA1 from a mammalian expression system) from Qiagen were used. The homologies of hemagglutinins, from which the sequences of the H5 HA antigens were derived, were determined against the 1-567-aa (full-length protein) and 17-338-aa (HA1 subunit) fragments of the HA from the A/Bar-headed Goose/Qinghai/60/05(H5N1) strain, which was the sequence source for the immunogen (rHA - A/H5N1/Qinghai). The sequence comparison was performed using the BLAST program and was expressed as a percentage of identical amino acids.

**Table 1**

| HA antigens source sequence | | | | Homology HA Ident | | 58 hybridomas before cloning | | | | | | |
|---|---|---|---|---|---|---|---|---|---|---|---|---|
| (IV strain, H5N1 AIV clade) | [%] | | | | | | | | | | | |
| | HA 1-567 aa | HA1 17-338 aa | 25 | 3 | 3 | 9 | 4 | 7 | 3 | 2 | 1 | 1 |
| Hemagglutinin ectodomain-based proteins from a mammalian expression system (rHA) | | | | | | | | | | | | |
| rHA - A/H5N1/Qinghai A/Bar-headed Goose/Qinghai/12/05 clade 2.2 | **100** | **100** | + | + | + | + | + | + | + | + | + | + |
| rHA - A/H5N1/lndia A/chicken/India/NIV33487/2006 clade 2.2 | 99 | 99 | + | + | + | + | + | + | + | + | + | + |
| rHA - A/H5N1/Vietnam A/Vietnam/1203/2004 clade 1 | 97 | 95 | + | + | + | + | + | + | + | + | + | + |
| rHA - A/H5N1/Guiyang A/goose/Guiyang/337/2006 clade 4 | 94 | 93 | + | + | - | + | + | + | + | - | - | - |
| rHA - A/H5N2/California A/American green-winged teal/ California/HKWF609/2007 | 89 | 88 | + | - | + | + | + | - | - | + | - | - |

| Hemagglutinin ectodomain-based protein from a baculovirus expression system (rHA) | | | | | | | | | | | | |
|---|---|---|---|---|---|---|---|---|---|---|---|---|
| rHA - A/H5N1/Poland A/swan/Poland/305-135V08/2006 clade 2.2 | 99 | 99 | + | + | + | + | - | + | - | + | + | - |

| Hemagglutinin HA1 subunit-based protein from a mammalian expression system (rHA1) | | | | | | | | | | | | |
|---|---|---|---|---|---|---|---|---|---|---|---|---|
| rHA1 -A/H5N1/Vietnam A/Vietnam/1203/2004 clade 1 | - | 95 | + | + | + | - | - | - | - | - | - | - |
| ↓ | | | | | | | | | | | | |
| 6 hybridomas were selected for cloning | | | | | | | | | | | | |

Antibodies produced by most of the non-cloned hybridomas (31/58) recognized recombinant proteins generated in mammalian and baculovirus expression systems, including both the rHA antigens and the rHA1 - A/H5N1/Vietnam protein. Few hybridomas (6/31) were found to produce antibodies, which do not detect proteins with hemagglutinin sequences of the lowest homology to the immunogen, i.e., rHA - A/H5N2/California or rHA - A/H5N1/Guiyang. The tests of the culture supernatants from the remaining primary hybridomas (27/58) did not show the presence of antibodies recognizing rHA1 - A/H5N1/Vietnam. A positive signal with rHA - A/H5N1/Vietnam and the lack of a signal with rHA1 - A/H5N1/Vietnam, observed in the tests of all of the culture supernatants from this hybridoma group, not necessarily mean that the mAbs are directed against the HA2 subunit of the antigen. Rationale behind this interpretation was presented in Example 4. Among the 27 hybridomas, for which the production of antibodies against rHA1 - A/H5N1/Vietnam was not shown, 13 hybridomas produced antibodies recognizing all of the used rHA proteins from a mammalian expression system. The antibodies produced by the remaining 14 hybridomas did not show reactivity against rHA - A/H5N2/California and/or rHA - A/H5N1/Guiyang with the lowest homologies to the immunogen. In the culture supernatants of some hybridomas (8/27), no antibodies recognizing baculovirus expression system-derived rHA antigen having the HA sequence from the A/H5N1/Poland strain were found, despite high homology of the protein to the immunogen.

Taking into account that the serotype-specific epitopes are localized in the HA1 subunit of HA, and the reactivity with antigens of varied homology indicates the specificity range of the obtained antibodies, the most promising material for further screening was the group of the 25 primary hybridomas, which produced antibodies recognizing all of the antigens used in the serotype specificity test. From this group, six hybridomas were chosen for cloning: G-1-31, G-2-14, G-5-32, G-6-42, G-7-24 and G-7-27. The immunoreactivity profiles obtained in the tests of culture supernatants from the selected hybridomas are included below in Table 2. The results are shown as the signal (A₄₅₀) values obtained in ELISAs using recombinant HA proteins.

In particular, Table 2 shows the results of immunoreactivity studies of antibodies produced by hybridomas selected for cloning. The studies were performed by ELISA using recombinant proteins based on the ectodomain (rHA) and HA1 subunit (rHA1) of hemagglutinin. The rHA - A/H5N1/Poland protein was produced in a baculovirus expression system (OET, batch 1). The remaining rHA and rHA1 proteins originated from a mammalian expression system (ITC). For coating with HA antigens, MediSorp (rHA - A/H5N1/Poland) plates from NUNC and Ni-NTA plates (rHA, rHA1 from a mammalian expression system) from Qiagen were used. The results are shown as the signal (A₄₅₀) values obtained from the analyses of hybridoma culture supernatants. The homologies of hemagglutinins, from which the sequences of the H5 HA antigens were derived, were determined against the 1-567-aa (full-length protein) and 17-338-aa (HA1 subunit) fragments of the HA from the A/Bar-headed Goose/Qinghai/60/05(H5N1) strain, which was the sequence source for the immunogen (rHA - A/H5N1/Qinghai). The sequence comparison was performed using the BLAST program and expressed as a percentage of identical amino acids.

**Table 2**

| HA antigens source sequence (IV strain, H5N1 AIV clade) | Homology HA Ident [%] | | Hybridomas selected for cloning (no.) | | | | | |
|---|---|---|---|---|---|---|---|---|
| | HA 1-567 aa | HA1 17-338 aa | G--1--31 | G--2--14 | G--5--32 | G--6--42 | G--7--24 | G--7--27 |
| Hemagglutinin ectodomain-based proteins from a mammalian expression system (rHA) | | | | | | | | |
| rHA - A/H5N1/Qinghai A/Bar-headed Goose/Qinghai/12/05 clade 2.2 | 100 | 100 | > 4.0 | > 4.0 | > 4.0 | > 4.0 | > 4.0 | > 4.0 |
| rHA - A/H5N1/lndia A/chicken/India/NIV33487/2006 clade 2.2 | 99 | 99 | > 4.0 | > 4.0 | > 4.0 | > 4.0 | > 4.0 | > 4.0 |
| rHA - A/H5N1/Vietnam A/Vietnam/1203/2004 clade 1 | 97 | 95 | > 4.0 | > 4.0 | > 4.0 | > 4.0 | > 4.0 | > 4.0 |
| rHA - A/H5N1/Guiyang A/goose/Guiyang/337/2006 clade 4 | 94 | 93 | > 4.0 | > 4.0 | > 4.0 | > 4.0 | > 4.0 | > 4.0 |
| rHA - A/H5N2/California A/American green-winged teal/ California/ HKWF609/2007 | 89 | 88 | > 4.0 | > 4.0 | > 4.0 | > 4.0 | > 4.0 | 3.784 |

| Hemagglutinin ectodomain-based protein from a baculovirus expression system (rHA) | | | | | | | | |
|---|---|---|---|---|---|---|---|---|
| rHA - A/H5N1/Poland A/swan/Poland/305-135V08/2006 clade 2.2 | 99 | 99 | 3.143 | 2.594 | 3.220 | 3.343 | 3.104 | 3.335 |

| Hemagglutinin HA1 subunit-based protein from a mammalian expression system (rHA1) | | | | | | | | |
|---|---|---|---|---|---|---|---|---|
| rHA1 -A/H5N1/Vietnam A/Vietnam/1203/2004 clade 1 | - | 95 | 1.532 | 1.244 | 1.861 | 3.638 | 1.670 | 1.975 |

In the studies of serotype specificity of antibodies produced by the selected hybridomas using the rHA antigens from a mammalian expression system and the rHA - A/H5N1/Poland from a baculovirus expression system, the very high (A₄₅₀> 4) and high (~2.6 - ~3.3) values of signals (A₄₅₀) were obtained, respectively. Using the rHA1 - A/H5N1/Vietnam in the tests, the lower A₄₅₀ values were read than in the case of the rHA1 - A/H5N1/Vietnam usage (~1.2 - ~3.6 vs > 4). This confirms the earlier observations on the lower detection sensitivity of the anti-HA1 subunit antibodies with the use of short protein fragments (1-345 aa) under the applied test conditions.

After cloning of the selected G-1-31, G-2-14, G-5-32, G-6-42, G-7-24 and G-7-27 hybridomas, 64 clones producing anti-H5 HA antibodies were obtained. Specificity determinations of the obtained mAbs against rHA - A/H5N1/Qinghai and rHA - A/H5N1/Poland and their isotyping were performed by ELISAs, as described in Example 3. The hybridoma culture supernatants were subjected to further analysis for the presence of serotype-specific antibodies. In comparison to the examinations performed for culture supernatants from the non-cloned hybridomas, the studies for serotype specificity of antibodies produced by the cloned hybridomas were performed using the HA antigen panel, which was extended to include two recombinant, HA1 subunit-based proteins from a mammalian expression system: rHA1 - A/H5N1/HK/156 and rHA1 - A/H5N1/HK/483 (ITC), as well as the H5N1, H5N2, H5N3 and H5N9 AIVs (x-OvO). The results of antigenicity studies indicating the conformation accuracy of the used rHA1 antigens are shown in Fig. 6 and described in Example 4. The AI viruses are shown in List C and described in Example 4. The rHA1 - A/H5N1/HK/156 and rHA1 - A/H5N1/HK/483 antigens shared 95% and 94%, amino acid sequence identity with immunogen's HA1 subunit, respectively, whereas the HA1 subunit of hemagglutinins in viral particles: 93-90%. The panel of antigens was completed with rHA - A/H5N1/Ck/Vietnam, which did not display any features of native HA structure (Fig. 6) in the antigenicity and oligomerization studies of recombinant proteins, described in Example 4. The serotype specificity determinations of the obtained mAbs were performed by ELISAs under conditions described in Example 4. The results from the serotype specificity analysis of the obtained mAb clones are presented in Table 3 included below.

In particular, Table 3 shows the results from immunoreactivity studies of antibodies produced by the cloned hybridomas. The studies were performed by ELISAs using recombinant proteins based on the ectodomain (rHA) and HA1 subunit (rHA1) of hemagglutinin, as well as the H5-serotype AIVs. The rHA - A/H5N1/Poland protein was produced in a baculovirus expression system (OET). The remaining rHA and rHA1 proteins were produced in a mammalian expression system (ITC). Influenza viruses of the H5 serotype (x-OvO) originated from the IZSVe. For coating with HA antigens, MediSorp (rHA - A/H5N1/Poland, batch 3), MaxiSorp (AIV) plates from NUNC and Ni-NTA plates (rHA, rHA1 from a mammalian expression system) from Qiagen were used. The homologies of hemagglutinins, from which the sequences of the H5 HA antigens were derived, was determined against the 1-567-aa (full-length protein) and 17-338-aa (HA1 subunit) HA fragments of the A/Bar-headed Goose/Qinghai/60/05(H5N1) strain, which iwas the sequence source for the immunogen (rHA - A/H5N1/Qinghai). The sequence comparison was performed using the BLAST program and expressed as a percentage of amino acid sequence identity.

**Table 3**

| HA antigens | Homology HA Ident [%] | | Hybridomas before cloning (no.) | | | | | |
|---|---|---|---|---|---|---|---|---|
| Recombinant HA protein source sequence (IV strains, H5N1 AIV clade) | | | G-1 -31 | G-2 -14 | G-5 -32 | G-6 -42 | G-7 -24 | G-7 -27 |
| | HA 1-567 aa | HA1 17-338 aa | 64 hybridomas after cloning | | | | | |
| Influenza virus (serotype, IV strain, H5N1 AIV clade) | | | 22 | 1 | 10 | 6 | 14 | 11 |
| Hemagglutinin ectodomain-based proteins from a mammalian expression system (rHA) | | | | | | | | |
| rHA - A/H5N1/Qinghai A/Bar-headed Goose/Qinghai/12/05, clade 2.2 | 100 | 100 | + | + | + | + | + | + |
| rHA - A/H5N1/lndia A/chicken/lndia/NIV33487/2006, clade 2.2 | 99 | 99 | + | + | + | + | + | + |
| rHA - A/H5N1/Vietnam A/Vietnam/1203/2004. clade 1 | 97 | 95 | + | + | + | + | + | + |
| rHA - A/H5N1/Guiyang A/goose/Guiyang/337/2006, clade 4 | 94 | 93 | + | + | + | + | + | + |
| rHA - A/H5N1/Ck/Vietnam ! A/chicken/Vietnam/NCVD-016/08, clade 7 | 91 | 89 | - | - | - | - | - | - |
| rHA - A/H5N2/California A/American green-winged teal/ California/ HKWF609/2007 | 89 | 88 | + | + | + | + | + | + |

| Hemagglutinin ectodomain-based protein from a baculovirus ex pression system (rHA) | | | | | | | | |
|---|---|---|---|---|---|---|---|---|
| rHA - A/H5N1/Poland A/swan/Poland/305-135V08/2006, clade 2.2 | 99 | 99 | + | + | + | + | + | + |

| Hemagglutinin HA1 subunit-based proteins from a mammalian expression system (rHA1) | | | | | | | | |
|---|---|---|---|---|---|---|---|---|
| rHA1 - A/H5N1/Vietnam A/Vietnam/1203/2004, clade 1 | - | 95 | + | + | + | + | + | + |
| rHA1 -A/H5N1/HK/156 A/Hong Konq/156/97, clade 0 | - | 95 | + | + | + | + | + | + |
| rHA1 - A/H5N1/HK/483 A/Hong Kong/483/1997, clade 0 | - | 94 | + | + | + | + | + | + |

| **H5-serotype influenza viruses** | | | | | | | | |
|---|---|---|---|---|---|---|---|---|
| IV - H5N3 A/duck/Italy/775/2004 | 93 | 93 | + | + | + | + | + | + |
| IV- H5N1 A/mallard/Italy/3401/2005. clade EA-nonGsGD | 93 | 93 | + | + | + | + | + | + |
| IV- H5N9 A/chicken/Italy/22A/1998 | 91 | 90 | + | + | + | + | + | + |
| IV - H5N2 A/turkey/Italy/1980 | 91 | 90 | + | + | + | + | + | + |
| mAb isotype | | | IgG1 | IgG1 | IgG1 | IgG1 | IgG1 | IgG1 |
| Selected hybridomas: G-1-31-22, G-2-14-10, G-5-32-5, G-6-42-42, G-6-42-71, G-7-24-17, G-7-27-18 | | | ↓ 1 clone | ↓ 1 clone | ↓ 1 clone | 1 2 clone | ↓ 1 clone | ↓ 1 clone |
| ! Antigen not showing native hemagglutinin conformation | | | | | | | | |

The obtained mAb clones, all of the IgG1 isotype, recognized conformational HA antigens: rHA (5/5) and rHA1 (3/3) from a mammalian expression system, rHA - A/H5N1/Poland from a baculovirus expression system, and H5 HA in viral particles (4/4). None of the obtained mAb clones bound to the rHA - A/H5N1/Ck/Vietnam, which did not show native HA features. As all of the obtained mAb clones showed the desired, broad range specificity against H5 HAs (Table 3), a few mAbs from cloning of each of the six primary hybridomas were selected for further studies. Such an approach enhanced the probability of finding the clones recognizing different epitopes specific for the H5 serotype of HA. One clone was selected from each of the mAb groups originated from the cloning of the G-1-31, G-5-32, G-7-24 and G-7-27 hybridomas, and two clones were selected from mAbs derived from the G-6-42 hybridoma cloning. In addition, the G-2-14-10 clone, obtained by the G-2-14 hybridoma cloning was chosen for further characterization.

The selected G-1-31-22, G-2-14-10, G-5-32-5, G-6-42-42, G-6-42-71, G-7-24-17 and G-7-27-18 antibody clones were purified from the hybridoma culture supernatants using HiTrap Protein G HP affinity columns (GE Healthcare). The preparation was performed according to the manufacturer's instructions. For mAb elution, 1 M glycine-HCI buffer, pH 2.7, was used. Protein fractions were neutralized during elution using 1 M Tris-HCI, pH 9.0. Buffer exchange and antibody concentrations were performed by centrifugation using Vivaspin® 6 Centrifugal Concentrator, 10 000 MWCO units (Sartorius Stedim Biotech). The purified G-1-31-22, G-2-14-10, G-5-32-5, G-6-42-42, G-6-42-71, G-7-24-17 and G-7-27-18 mAbs were examined by mass spectrometry and ELISAs for serotype specificity testing. The differentiation of the above-mentioned antibody clones was performed based on the immunoreactivity profiles and peptide maps of Fab fragments. To evaluate the ability of the generated mAbs to inhibit hemagglutination, the Hl testes were carried out. The results from the performed studies are presented in the subsequent Examples 6-10. The possible applications of the obtained antibodies are described in Examples 11 and 12. The object of the present patent application are monoclonal antibodies specific to HA of the H5-serotype IVs: G-1-31-22, G-2-14-10, G-5-32-5, G-6-42-42, G-6-42-71, G-7-24-17 and G-7-27-18 and their use.

### Example 6 Mass spectra of the selected antibody clones

The purified G-1-31-22, G-2-14-10, G-5-32-5, G-6-42-42, G-6-42-71, G-7-24-17 and G-7-27-18 antibody clones, chosen as a result of the selection for serotype specificity (Table 3) and described in Example 5, were analyzed using a MALDI TOF/TOF mass spectrometer (4800 Plus, AB SCIEX). Before performing mass spectra, the samples were purified using ZipTip®_{C4} (Millipore) according to the procedures included in the manufacturer's instruction: "User Guide for Reversed-Phase ZipTip". The matrix was the sinapinic acid (Fluka) at a concentration of 5 mg/mL, dissolved in 0.1% trifluoroacetic acid containing 50% of acetonitrile. Mass spectra were measured in the linear mode (MS Linear Positive Ion), in the range of 20 - 170 kDa. External calibration was achieved with IgG standard (AB SCIEX). All mass spectra were processed with a Gaussian filter and the signal detection procedures using the Data Explorer Software (V4.9).

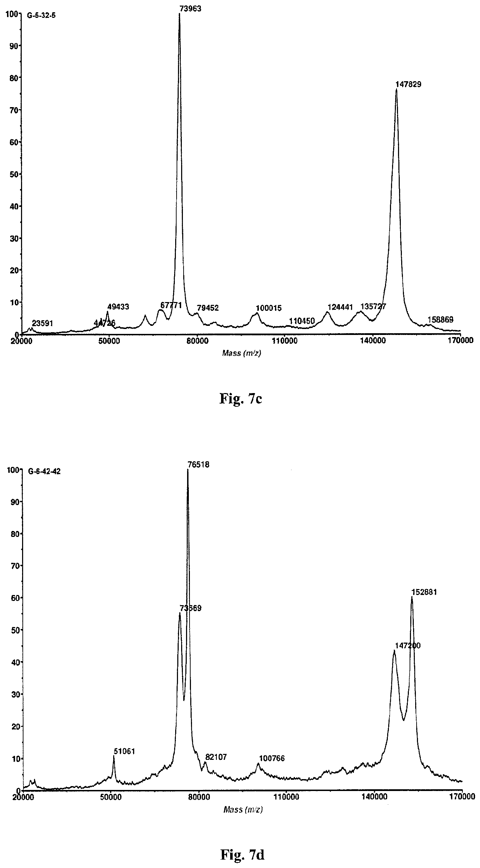

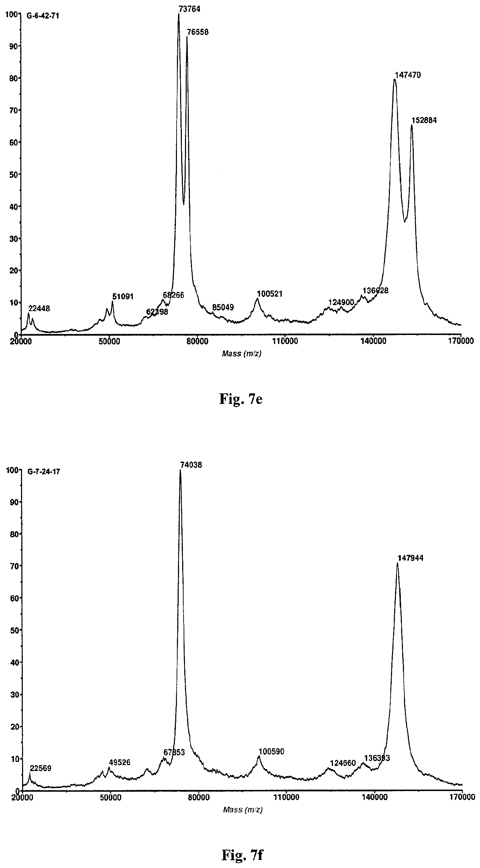

Fig. 7a-g shows the mass spectra of the G-1-31-22, G-2-14-10, G-5-32-5, G-6-42-42, G-6-42-71, G-7-24-17 and G-7-27-18 mAbs against H5 HA. The molecular weight signals in the ranges of 147 - 153 kDa and 74 - 77 kDa represent the tested antibodies and correspond to the singly- and doubly-ionized samples, respectively. The determined molecular weights are in line with values expected for the mouse IgG antibodies. The differences between spectra of the individual clones may result from a varied protein glycosylation. The molecular weight of the G-1-31-22 mAb is 147 kDa, while of the G-2-14-10, G-5-32-5, G-7-24-17 and G-7-27-18 mAbs is 148 kDa. The mass spectra of the G-6-42-42 and G-6-42-71 mAbs, which were obtained by cloning of the primary G-6-42 hybridoma (Table 3), indicate the presence of 2 antibody forms having molecular weights of 147 kDa and 153 kDa (Fig. 7d,e). Preasumably, these 2 antibody forms differ in the glycosylation level, and the higher molecular weight proteins were generated as a result of additional, non-enzymatic glycosylation.

The relationship between the increase in molecular weights of antibodies and the increased level of non-enzymatic glycosylation was well documented in the studies of immunoglobulin fractions from diabetic patients (Lapolla A. et al., 1997, 2000a, 2000b). For example, the molecular weights of IgG antibodies from healthy individuals and the antibody standard, determined with the use of a mass spectrometer, were 149 kDa and 148 kDa, respectively, and 152 kDa for antibodies in the protein fractions from patients with poorly-controlled diabetes (Lapolla A. et al., 1997, 2000a). The effect of the enhanced non-enzymatic glycosylation was also obtained *in vitro,* by incubating the protein fraction from plasma of a healthy individual or the IgG antibody standard in the presence of high glucose concentrations, when an increase in the antibody molecular weight from 149 kDa and 148 kDa to 153 kDa, respectively, was observed (Lapolla A. et al., 2000a).

### Example 7 Reactivity of the selected antibody clones with hemagglutinin antigens.

The G-1-31-22, G-2-14-10, G-5-32-5, G-6-42-42, G-6-42-71, G-7-24-17 and G-7-27-18 monoclonal antibodies, chosen as a result of the selection for serotype specificity (Table 3), were purified by affinity chromatography, as described in Example 5. Further studies included the reactivity determination of the selected mAb clones with HAs of the H1-H16 serotypes in order to evaluate their specificity and affinity for the H5 HA of different IV strains, and their ability to cross-react with HAs of serotypes other than H5. The studies were carried out using the H5 HA antigens with diverse properties. Among these antigens, there were recombinant proteins based on the ectodomain (rHA) or HA1 subunit (rHA1) of hemagglutinin and the H5-serotype AIVs (Lists B and C). Recombinant HA proteins, originated from a mammalian expression system and, exceptionally, from a baculovirus expression system, contained the HA1 subunit with, usually, proper conformation. They existed as monomers (rHA1) or, mostly or partially, formed oligomers (rHA). Conformational antigens contained HA sequences from twelve IV strains of the H5 serotype with varied homology to the immunogen (rHA - A/H5N1/Qinghai) - amino acid sequence identities of the HA1 subunit of the proteins used in the studies were from 99% to 88% (List F). The antigens: rHA - A/H5N1/Qinghai from a mammalian expression system and rHA - A/H5N1/Poland from a baculovirus expression system, were described in detail in Examples 2 and 3, respectively, whereas the remaining H5 HA antigens, produced in mammalian cells, were described in Example 4.

In comparison to the tests performed for the supernatants from the G-1-31-22, G-2-14-10, G-5-32-5, G-6-42-42, G-6-42-71, G-7-24-17 and G-7-27-18 hybridoma cultures (Example 5), the studies for serotype specificity of antibodies purified from the culture supernatants of these hybridomas were performed using the panel of antigens, which was extended to include the HA ectodomain-based recombinant protein from a bacterial expression system and AIVs of H1-H4 and H6-H16 serotypes. The protein (17-522 aa, ΔRRRKKR) with the HA sequence from the A/swan/Poland/305-135V08/2006(H5N1) AIV strain, was over-expressed in *Escherichia coli* at the Institute of Biotechnology and Antibiotics (IBA) in Warsaw (Poland). Following purification and refolding, the H5 HA protein was subjected to the analyses by mass spectrometry, ELISAs for HA protein antigenicity and oligomerization studies, similarly as the other recombinant H5 HA antigens. In the immunoreactivity studies, the antigen preparation of ~80% purity was used. The molecular weight of the rHA - A/H5N1/Poland protein from a bacterial expression system, determined using a MALDI TOF/TOF mass spectrometer (4800 Plus, AB SCIEX), was 57 kDa. Contrary to the results obtained for the rHA - A/H5N1/Qinghai and rHA - A/H5N1/Poland proteins from mammalian and baculovirus expression systems, respectively (Examples 2, 3), the experimentally determined molecular weight of bacterial HA was consistent with the weight calculated from the amino acid composition using the GPMAW 8.2 program (Lighthouse). This is due to the fact that the proteins produced in bacteria do not undergo glycosylation. The conducted analyses showed that the produced protein has the native antigen features. It preseves conformational epitopes recognized by commercial anti-H5 HA antibodies (List A), including also the HI antibodies, as well as forms oligomeric structures. Two antigenic variants of the protein were prepared at the IBA. In the tests described in the present Example and in Examples 8 and 11, the second antigenic variant was used. In the studies of the purified mAbs, twenty-one certified AIV strains representing the H1-H4 and H6-H16 serotypes (List C) were used.

### Specificity for the H5 HAs

Specificity of the purified G-1-31-22, G-2-14-10, G-5-32-5, G-6-42-42, G-6-42-71, G-7-24-17 and G-7-27-18 mAbs against H5 HAs was tested by titrating them against the rHA - A/H5N1/Qinghai protein from a mammalian expression system (ITC), which was used as the immunogen in the antibody production procedure (Examples 1, 2). Also, titrations against the rHA - A/H5N1/Poland proteins from baculovirus (OET) and bacterial (IBA) expression systems with high homologies to the immunogen (Lists E and F), were performed.

To perform the assay, the rHA - A/H5N1/Qinghai (ITC) and the rHA - A/H5N1/Poland (OET, batch 8), diluted in PBS to a concentration of 1 µg/mL were applied to MediSorp plates (NUNC), and the rHA - A/H5N1/Poland preparation (IBA) of ~80%purity, containing the refolded hemagglutinin in PBS at a concentration of ~1 µg/mL, was applied to PolySorp, MediSorp, MaxiSorp and MultiSorp plates (NUNC). Some of the plate wells were filled with PBS instead of the antigen solution. Following the overnight incubation at 2-8ₒC, the plates were blocked with 10% FBS/PBS. The purified mAb clones were analyzed as a series of 2-fold dilutions in 2% BSA/PBS. The antibodies diluted in a range of 8 µg/mL - 0.015 ng/mL were applied to the plates coated with antigens from mammalian and baculovirus expression systems, whereas the antibodies diluted in a range of 8 µg/mL - 7.8 (3.9) ng/mL were applied to the plates coated with the protein from a bacterial expression system. The controls for a level of the non-specific antibody binding were samples containing antibodies at concentrations of 4 µg/mL and/or 8 µg/mL and 2% BSA/PBS (BLK sample) applied to the non-coated and antigen-coated wells, respectively. The plates with the obtained mAbs and control samples were incubated overnight at 2-8ₒC. For the detection of antigen-bound mAbs, the HRP-labelled antibodies against mouse IgG, γ-chain specific (Sigma-Aldrich), were used. The secondary antibodies were diluted 1:1000 in 2% BSA/PBS and incubated on the plates for 1 h at 37ₒC. TMB (Sigma-Aldrich) was used as a substrate for HRP. The reaction was was stopped with 0.5 M H₂SO₄ solution. The absorptions of the samples were read at λ=450 nm.

Titration curves of the G-1-31-22, G-2-14-10, G-5-32-5, G-6-42-42, G-6-42-71, G-7-24-17 and G-7-27-18 mAbs against rHA - A/H5N1/Qinghai from a mammalian expression system are shown in Fig. 8, and against rHA - A/H5N1/Poland from baculovirus and bacterial expression systems in Fig. 9 and Fig.10, respectively. The obtained results indicate that all of the tested mAb clones have a high affinity for the antigens from an eukaryotic expression system - high levels of signals (A₄₅₀) were obtained for antibody concentrations in the nanogram range (Fig. 8, 9). No significant differences were observed between the titration curves of the individual antibodies against the antigens from mammalian and baculovirus expression systems. The results from immunoreactivity studies of particular mAb clones with bacterial rHA - A/H5N1/Poland protein bound to the PolySorp, MediSorp, MaxiSorp and MultiSorp plated (NUNC), were similar, therefore they are presented as the mean A₄₅₀ values ± SD, obtained using particular types of plates (Fig. 10). The reactivity with this antigen was shown for the G-2-14-10, G-6-42-42, G-6-42-71 and G-7-27-18 antibodies, wherein the high A₄₅₀ values were obtained with nanogram (G-6-42-42, G-6-42-71, G-7-27-18) or microgram (G-2-14-10) ranges of antibody concentrations. Under the applied assay conditions, a very weak reactivity of the G-5-32-5 mAb and no reactivities of the G-1-31-22 and G-7-24-17 mAbs with bacterial H5 HA protein were found.

Antibody concentrations giving the signal (A₄₅₀) levels of 1.5 and/or 1.0 were determined by interpolation from the linear parts of the 4-parametric titration curves using the Gene5 program (Bio-Tek). The Interpolated values obtained for the individual mAb clones are presented in Table 4 included below.

In particular, Table 4 shows concentration of the G-1-31-22, G-2-14-10, G-5-32-5, G-6-42-42, G-6-42-71, G-7-24-17, G-7-27-18 mAbs, determined by interpolation from the linear ranges of the 4-parametric titration curves against recombinant HA proteins with high homology to the immunogen. The rHA* - A/H5N1/Qinghai protein (17-530 aa, ΔRRRKKR, 6x His) with the HA sequence derived from the A/Bar-headed Goose/Qinghai/12/05(H5N1) AIV strain was produced in (*) a mammalian expression system (ITC). The rHA** - A/H5N1/Poland (17-530 aa, ΔRRRKKR, 6x His) and rHA*** - A/H5N1/Poland (17-522 aa, ΔRRRKKR) proteins were produced in (**) baculovirus (OET, batch 8) and (***) bacterial (IBA) expression systems, respectively, based on the HA sequence derived from the A/swan/Poland/305-135V08/2006(H5N1) AIV strain. The determination was performed by ELISA. Titrations against the HA proteins from mammalian and baculovirus expression systems were performed using MediSorp plates (NUNC) coated with the antigens at a concentration of 1 µg/mL, and the results are shown as the concentration values interpolated for the signal (A₄₅₀) level of 1.5. Titration against the HA protein from a bacterial expression system was performed on PolySorp, MediSorp, MaxiSorp and MultiSorp plates (NUNC) coated with rHA preparation, ~80%pure, which contained the refolded hemagglutinin at a concentration of ~1 µg/mL. The results are shown as means from the concentration values (± SD) that were interpolated for the A₄₅₀ levels of 1.0 and/or 1.5, obtained in assays performed using particular types of polystyrene plates. Titration curves and interpolated values were determined using the Gene5 program (Bio-Tek).

**Table 4**

| **mAb clones** | **mAb concentration for A₄₅₀ = 1.5 [ng/mL]** | | | **mAb concentration for A₄₅₀ = 1.0 [ng/mL]** |
|---|---|---|---|---|
| | **rHA* A/H5N1/Qinghai** | **rHA** A/H5N1/Poland** | **rHA*** A/H5N1/Poland** | **rHA*** A/H5N1/Poland** |
| G-1-31-22 | 39 | 38 | - | - |
| G-2-14-10 | 11 | 15 | - | ∼6368 ± 157 |
| G-5-32-5 | 13 | 14 | - | - |
| G-6-42-42 | 9 | 10 | ∼10 ± 1 | ~5 ± 1 |
| G-6-42-71 | 14 | 14 | ~12 ± 2 | ~6 ± 1 |
| G-7-24-17 | 39 | 43 | - | - |
| G-7-27-18 | 7 | 9 | ∼22 ± 28 | ∼161 ± 12 |
| * mammalian, ** baculovirus, ***bacterial expression systems | | | | |

The values interpolated from the titration curves of the G-2-14-10, G-5-32-5, G-6-42-42, G-6-42-71, G-7-27-18 mAbs against rHA - A/H5N1/Qinghai from a mammalian expression system and rHA - A/H5N1/Poland from a baculovirus expression system for the A₄₅₀ = 1.5 reached the values in the ranges of 7-14 ng/mL and 9-15 ng/mL, respectively, and in the case of G-1-31-22 and G-7-24-17 mAbs, they were 39 ng/mL and 38 ng/mL, and 43 ng/mL, respectively. The values interpolated from the titration curves of the G-6-42-42, G-6-42-71 mAbs against rHA - A/H5N1/Poland from a bacterial expression system for the A₄₅₀ = 1.5 reached the values of ∼10 ng/mL ± 1 and ∼12 ng/mL ± 2, respectively. They were comparable to the values obtained by titration of these antibodies against the antigens from an eukaryotic expression system. In the case of G-7-27-18 mAb titration, the concentration interpolated for the A₄₅₀ = 1.5 from the titration curve against bacterial HA protein was ∼322 ng/mL ± 28 and was, in average, 41-fold higher than against the antigens originated from mammalian and baculovirus expression systems. Within the applied range of the G-2-14-10 mAb dilutions, the concentration interpolated for the A₄₅₀ = 1.0 from the titration curve against bacterial HA protein was ∼6368 ng/mL ± 157. It was over 1000-fold higher than the concentration interpolated for the same signal level obtained for the G-6-42-42 and G-6-42-71 mAbs, and ~40-fold higher than the one obtained for the G-7-27-18 mAb.

The data shown in Table 4 indicate similarity of the results obtained for particular mAb clones in the studies that used the rHA proteins (17-530 aa, ΔRRRKKR, 6x His) from mammalian and baculovirus expression systems with high homology, differing in the levels of glycosylation (Examples 2, 3) and oligomerization (Fig. 3). In the case of four out of the seven obtained mAb clones, for which reactivity with non-glycosylated, bacterial H5 HA protein (17-522 aa, ΔRRRKKR) was shown under the applied conditions, the interpolated concentration values indicate similarity of the epitope presentation for the G-6-42-42 and G-6-42-71 mAbs in recombinant, different-origin HA proteins, as well as large or very large differences in the epitope presentation for the G-7-27-18 and G-2-14-10 mAbs, respectively, in the antigens from prokaryotic and eukaryotic expression systems.

### Reactivity with recombinant H5 HA proteins

Before performing reactivity studies of the generated mAbs with recombinant H5 HA proteins, the analysis of the serially diluted antibodies on Ni-NTA plates coated with rHA - A/H5N1/Vietnam from a mammalian expression system (ITC) (List B) was conducted. The assay was performed under conditions described in Example 4. Concentrations of mAbs from the linear range of the titration curves giving an A₄₅₀ of~2.5 are presented in Table 5, below.

In particular, Table 5 shows the concentrations of the G-1-31-22, G-2-14-10, G-5-32-5, G-6-42-42, G-6-42-71, G-7-24-17, G-7-27-18 mAbs from the linear range of the titration curves against rHA - A/H5N1/Vietnam at which the signal (A₄₅₀) level was ∼2.5. The protein (18-530 aa, ΔRRRKKR, 6x His) with the HA sequence derived from the A/Vietnam/1203/2004(H5N1) strain was produced in (*) a mammalian expression system (ITC). The assay was performed by ELISA on Ni-NTA plates (Qiagen) coated with antigen at a concentration of 1 µg/mL.

**Table 5**

| **mAb clones** | **mAb concentration for A₄₅₀ ∼2.5 [ng/mL]** |
|---|---|
| | **rHA* A/H5N1/Vietnam** |
| | |
| G-1-31-22 | 50.0 |
| G-2-14-10 | 22.5 |
| G-5-32-5 | 20.0 |
| G-6-42-42 | 8.75 |
| G-6-42-71 | 12.5 |
| G-7-24-17 | 50.0 |
| G-7-27-18 | 12.5 |
| | |
| * mammalian expression system | |

For immunoreactivity studies, Ni-NTA plates (Qiagen) were coated with H5 HA proteins: the rHA and rHA1 from a mammalian expression system (ITC) and the rHA protein from a baculovirus expression system (OET, batch 8). The antigens were diluted in 1% BSA/PBS to a concentration of 1 µg/mL. Some of the plate wells were filled with 1% BSA/PBS and incubated overnight at 2-8ₒC, in parallel with the antigen-containing wells. The tested mAb clones were diluted in 2% BSA/PBS to concentrations given in Table 5, and then applied to the coated and non-coated wells of the plate. The assay was carried out in the presence of control samples. Commercial antibodies against H5 HA (mAb 8, List A) were the positive control, whereas 2% BSA/PBS was the negative control (BLK sample). Plates with the tested and control samples were incubated overnight at 2-8ₒC. For the detection of antigen-bound mAbs, the HRP-labelled antibodies against mouse IgG, γ-chain specific, (Sigma-Aldrich), were used. The secondary antibodies were diluted 1:1000 in 2% BSA/PBS and incubated on the plates for 1 h at 37ₒC. TMB (Sigma-Aldrich) was used as a substrate for HRP. The reaction was stopped using 1.25 M H₂SO₄ solution. The absorptions of the samples were read at λ=450 nm.

The results from reactivity studies of the G-1-31-22, G-2-14-10, G-5-32-5, G-6-42-42, G-6-42-71, G-7-24-17 and G-7-27-18 mAbs with recombinant proteins based on the ectodomain (rHA) and HA1 subunit (rHA1) of hemagglutinin, are shown in Fig. 11. The obtained mAb clones recognized all of the conformational HA antigens: the rHA (5/5) and rHA1 (3/3) proteins from a mammalian expression system, the rHA - A/H5N1/Poland from a baculovirus expression system. None of the obtained mAb clones bound to the rHA - A/H5N1/Ck/Vietnam, which did not display native-HA characteristics. The results obtained for the purified antibodies are consistent with the results of the tests for the presence of serotype-specific antibodies in culture supernatants from the cloned hybridomas, which are shown in Table 3 and described in Example 5. Immunoreactivity studies proved that the G-1-31-22, G-2-14-10, G-5-32-5, G-6-42-42, G-6-42-71, G-7-24-17 and G-7-27-18 mAbs recognize conformational epitopes of the H5 HAs and are directed against the HA1 subunit of the antigen. In contrast to some of the commercially available anti-H5 HA antibodies (Fig. 6), described in List A, all of the obtained mAbs showed reactivity with H5 HAs of antigenically distant IV strains.

To provide better data comparability, the signal (A₄₅₀) values obtained in the reactivity studies of the generated antibodies with recombinant H5 HA proteins having the sequences from different viral strains, were expressed as % of the A₄₅₀ values read in the tests, in which the immunogen (rHA - A/H5N1/Qinghai) was used. The results thus obtained, further described as a relative reactivity of the obtained antibodies against recombinant HA proteins, are shown in Fig. 12. The obtained mAb clones retained high reactivity, shown with rHA - A/H5N1/Qinghai, against the rHA proteins (17/18-530 aa, ΔRRRKKR, 6x His) from a mammalian expression system having the HA sequences from the A/H5N1/lndia, A/H5N1/Vietnam and A/H5N1/Guiyang influenza viruses ― the relative reactivities against individual antigens were 96-113%, 100-111% and 96-121%, respectively. The HA1 subunit of the above-mentioned antigens shared from 99% to 93% amino acid sequence identities with immunogen's HA1 subunit (List F).

For most of the obtained clones (5/7), the decrease in relative reactivities against rHA - A/H5N2/California (19-506 aa, 6x His) from a mammalian expression system to values in a range of 58-85% was found. The HA1 subunit of this antigen shared 88% amino acid sequence identity with immunogen's HA1 subunit (List F). The reduced relative reactivities were also observed in the studies using the rHA - A/H5N1/Poland (17-530 aa, ΔRRRKKR, 6x His) from a baculovirus expression system with high homology to the immunogen (99% amino acid sequence identity between the HA1 subunits, List F). This was the case for all of the obtained mAbs (7/7), and the relative reactivities were from 46% to 87%.

In the tests using the HA1 subunit-based proteins from a mammalian expression system: rHA1 - A/H5N1/Vietnam (1-345 aa, 6x His), rHA1 - A/H5N1/HK/156 (18-346 aa, 6x His) and rHA1 - A/H5N1/HK/483 (17-346 aa, 6x His), the lower reactivities were found in comparison to the reactivities against the rHA proteins from a mammalian expression system with the HA sequences from the A/H5N1/Qinghai, A/H5N1/lndia, A/H5N1/Vietnam and A/H5N1/Guiyang influenza viruses. The relative reactivities against individual rHA1 antigens were 60-89%, 43-81% and 26-55%, respectively. The differences in relative reactivities of the obtained antibody clones against rHA - A/H5N1/Vietnam and rHA1 - A/H5N1/Vietnam, were from 21% to 42%. This is consistent with previous observations that the detection sensitivity of the anti-HA1 subunit antibodies is lower when the rHA1 proteins and not rHA proteins are used for coating of the Ni-NTA plates (Examples 4, 5). The amino acid sequence identities between the rHA1 proteins with the HA sequences from the A/H5N1/Vietnam, A/H5N1/HK/156, A/H5N1/HK/483 influenza viruses and the immunogen's HA1 subunit were 95%, 95% and 94%, respectively (List F).

The reduced relative reactivities against the rHA - A/H5N2/California antigen with a relatively high oligomerization degree (Fig. 3), which was observed for most of the obtained mAbs (5/7), could result from a lower homology of the protein to the immunogen (88% *vs* 99%-93% amino acid sequence identities between the HA1 subunits, List F), but also from the difference in the antigen presentation related to the length of the protein fragment (19-506 aa *vs* 17/18-530 aa). The significantly lower reactivities of all of the obtained mAbs against rHA - A/H5N1/Poland from a baculovirus expression system than against rHA - A/H5N1/Qinghai of a similar sequence, as well as with other rHA proteins of the same length (17/18-530 aa) from a mammalian expression system, showed in the studies using the Ni-NTA plates, resulted probably from a relatively low protein oligomerization., This could influence the coating efficiency and/or presentation of the antigen bound to the plate by a 6x His tag. The justification for such an interpretation are the results from the work on the optimization of ELISAs for determining the specificity of the generated mAbs (Example 3), as well as from the specificity studies of the obtained mAb clones against rHA - A/H5N1/Poland and rHA - A/H5N1/Qinghai, which were performed using MediSorp plates and were described in the first part of the present Example. These studies did not show significant differences in the reactivity level of the individual mAb clones against the antigens from mammalian and baculovirus expression systems, despite the differences in the glycosylation (Examples 2, 3) and oligomerization levels (Fig. 3). As in the case of rHA - A/H5N1/Poland from a baculovirus expression system, the very low oligomerization or its absence influencing the antigen coating and presentation, was probable the cause of the reduced relative reactivities of the obtained mAbs with the rHA1 proteins bound to the Ni-NTA plate. Analysis of the obtained results leads to the conclusion that the observed diversity of the relative reactivities of the obtained mAbs against recombinant H5 HA antigens results from different properties of the proteins used in the studies rather than from variability of the sequences forming the HA1 subunit of these proteins.

### Reactivity with influenza viruses of the H1-H16 serotypes

The studies on the the ability of the G-1-31-22, G-2-14-10, G-5-32-5, G-6-42-42, G-6-42-71, G-7-24-17 and G-7-27-18 mAbs to recognize HA in viral particles were performed by ELISA using twenty-five AIV strains, representing the H1-H16 serotypes (List C). The assay was performed under conditions described in Example 4. The results from reactivity studies of the obtained mAb clones are presented in Table 6 included below.

In particular, Table 6 shows the results from reactivity studies of the G-1-31-22, G-2-14-10, G-5-32-5, G-6-42-42, G-6-42-71, G-7-24-17 and G-7-27-18 mAbs against LPAIVs of the H1, H2, H3, H4, H5, H6, H7, H8, H9, H10, H11, H12, H13, H14, H15 and H16 serotypes (List C). The determination was performed with ELISA by coating of MaxiSorp plates (NUNC) with viruses diluted to 4000 HAU/mL based on the values specified by the manufacturer. Influenza viruses (x-OvO) originated from the IZSVe. The purified antibodies were tested at a concentration of 20 µg/mL.

**Table 6**

| **HA serotype** | **Avian influenza viruses (AIVs)** | **mAb clones** | | | | | | |
|---|---|---|---|---|---|---|---|---|
| | | **G-1 -31 -22** | **G-2 -14 -10** | **G-5 -32 -5** | **G-6 -42 -42** | **G-6 -42 -71** | **G-7 -24 -17** | **G-7 -27 -18** |
| **H1** | A/duck/It/1447/05(H1N1) | - | - | - | - | - | - | - |
| **H2** | A/duck/Germ/1215/73(H2N3) | - | - | - | - | - | - | - |
| **H3** | A/pass/It/6000/V00(H3N8) | - | - | - | - | - | - | - |
| | A/psitt/It/2873/00(H3N8) | - | - | - | - | - | - | - |
| **H4** | A/cockatoo/Eng/72(H4N8) | - | - | - | - | - | - | - |
| **H5** | A/mallard/It/3401/05(H5N1) | + | + | + | + | + | + | + |
| | A/turk/It/80(H5N2) | + | + | + | + | + | + | + |
| | A/duck/It/775/04(H5N3) | + | + | + | + | + | + | + |
| | A/ck/It/22A/98(H5N9) | + | + | + | + | + | + | + |
| **H6** | A/turkey/Canada/65 (H6N2) | - | - | - | - | - | - | - |
| **H7** | A/ck/It/1067/V99(H7N1) | - | - | - | - | - | - | - |
| | A/ty/It/9289/V02(H7N3) | - | - | - | - | - | - | - |
| | A/mallard/It/4810-79/04(H7N4) | - | - | - | - | - | - | - |
| | A/macaw/626/80(H7N7) | - | - | - | - | - | - | - |
| **H8** | A/turk/Ont/6118/68(H8N4) | - | - | - | - | - | - | - |
| **H9** | A/ty/Wis/66(H9N2) | - | - | - | - | - | - | - |
| | A/turk/Scotland/1/70(H9N7) | - | - | - | - | - | - | - |
| **H10** | A/ostrich/S A/01 (H10 N1) | - | - | - | - | - | - | - |
| **H11** | A/duck/Eng/56(H11N6) | - | - | - | - | - | - | - |
| | A/duck/Memphis/546/174(H11N9) | - | - | - | - | - | - | - |
| **H12** | A/duck/Alberta/60/76(H12N5) | - | - | - | - | - | - | - |
| **H13** | A/gull/Maryland/704/77(H13N6) | - | - | - | - | - | - | - |
| **H14** | A/mallard/Gurjev/263/82(H14N5) | - | - | - | - | - | - | - |
| **H15** | A/shearwater/2576/79(H15N9) | - | - | - | - | - | - | - |
| **H16** | A/gull/Denmark/68110/02(H16N3) | - | - | - | - | - | - | - |

All of the obtained mAb clones, i.e., G-1-31-22, G-2-14-10, G-5-32-5, G-6-42-42, G-6-42-71, G- 7-24-17 and G-7-27-18, recognized HA of the H5N1, H5N2, H5N3 and H5N9 AIVs. The results obtained for the purified antibodies are consistent with the results of the tests for the presence of antibodies specific against H5-serotype AIVs in the culture supernatants from the cloned hybridomas, which are shown in Table 3 and described in Example 5. The tests with the use of AIVs of the H1-H4 and H6-H16 serotypes showed that the generated antibodies do not cross-react with HAs of serotypes other than H5.

The results from reactivity studies of the G-1-31-22, G-2-14-10, G-5-32-5, G-6-42-42, G-6-42-71, G-7-24-17 and G-7-27-18 mAbs with H5N3, H5N1, H5N9 and H5N2 AIVs are presented in Fig. 13. The reactivities of all of the obtained antibody clones were the highest against the H5N3 AIV. In the tests using this virus, the signal (A₄₅₀) values were in the range of 1.4 - 3.0. According to the results from the sequence analysis of the H5 HA antigens against the source sequence of the immunogen (rHA - A/H5N1/Qinghai), which are included in Lists E and F, the full-length protein as well as the HA1 subunit of the H5N3 virus's HA showed the highest homology scores (Max Score, Total Score, Identities) among the hemagglutinins of the AIV strains used in the immunoreactivity studies of the obtained mAbs.

To provide better data comparability, the signal (A₄₅₀) values obtained in the studies of the generated antibody reactivities against the H5N1, H5N9 and H5N2 viruses, were expressed as % of the A₄₅₀ values read in the tests, in which the H5N3 AIV was used. The results thus obtained, further described as a relative reactivity of the obtained antibodies against the H5- serotype AIVs, are shown in Fig. 14. The reactivities of the G-1-31-22, G-2-14-10, G-5-32-5, G-6-42-42, G-6-42-71, G-7-24-17 and G-7-27-18 mAbs with H5N1 AIV were 45-80% of the reactivities determined with H5N3 virus. Comparing to the hemagglutinin of the H5N3 AIV, the HA1 subunit of the H5N1 virus's HA is characterized by the same amino acid sequence identity determined against the immunogen (93%) and the lower homology scores: Max Score and Total Score (List F). Further lowering of the obtained mAb reactivities was observed in assays using the H5N9 and H5N2 AIVs. The relative reactivities of antibodies with these viruses were in the range of 10-39% and 24-35%, respectively. According to the data in List F, the HA1 subunit of the H5N9 and H5N2 viruses shares 90% amino acid sequence identity with immunogen's HA1 subunit, and the homology scores: Max Score and Total Score, are higher in the case of the antigen of the H5N9 virus than the H5N2 virus.

Analysis of the obtained results leads to the conclusion that the observed diversity of the individual mAb clone reactivities against the H5N1, H5N2, H5N3 and H5N9 AIVs may be related to the variation of the sequences forming the HA1 subunit of HAs in viruses used for testing and/or to the effectiveness of the ELISA plate coating with viral antigens. Information regarding the differences in the composition of the viral preparations, which could influence the conditions of assays for antibody immunoreactivity studies, is included in the description of ELISA procedure using influenza viruses (Example **4**).

### Summary

Further studies of the G-1-31-22, G-2-14-10, G-5-32-5, G-6-42-42, G-6-42-71, G-7-24-17 and G-7-27-18 mAbs, after their purification from the hybridoma culture supernatants, were performed using the H5 HA antigens with diverse properties. Among the antigens, there were the HA ectodomain-based recombinant proteins (rHA) from mammalian, baculovirus and bacterial expression systems, the proteins based on the HA1 subunit (rHA1), obtained in mammalian cells, as well as twenty-five AIV strains of the H1-H16 serotypes (Lists B and C). The vast majority of the H5 HA antigens (13/14) displayed native-hemagglutinin characteristics (Fig. 2, 5, 6).

The obtained mAb clones recognized all conformational, glycosylated H5 HA antigens: the rHA (6/6) and rHA1 (3/3) proteins from an eukaryotic expression system (Fig. 11), as well as the H5-serotype AIVs (4/4) (Fig. 13) with diverse homology to the rHA - A/H5N1/Qinghai immunogen (99% to 88% amino acid sequence identities between the HA1 subunits, List F). Under the applied testing conditions, for some clones (4/7) a significant reactivity towards the non-glycosylated bacterial H5 HA protein was also shown. None of the obtained mAb clones bound to the non-conformational rHA - A/H5N1/Ck/Vietnam protein (Fig. 11) or to the AIVs of the H1-H4 and H6-H16 serotypes (Table 6).

The obtained results lead to the conclusion that the G-1-31-22, G-2-14-10, G-5-32-5, G-6-42-42, G-6-42-71, G-7-24-17 and G-7-27-18 mAbs are directed against conformational epitopes in the hemagglutinin HA1 subunit, show a broad range of specificities to the H5-serotype HAs and at the same time, do not cross-react with HAs of the H1-H4 and H6-H16 serotypes.

### Example 8 Clone differentiation based on the immunoreactivity profiles

Based on the results from reactivity studies of the G-1-31-22, G-2-14-10, G-5-32-5, G-6-42-42, G-6-42-71, G-7-24-17 and G-7-27-18 mAbs with the H5 HA antigens (Example 7), the reactivity profiles were created for the use in a comparative analysis of the obtained clones. Fig. 15 shows the reactivity profiles of antibodies with rHA - A/H5N1/Qinghai from a mammalian expression system, rHA - A/H5N1/Poland from a baculovirus expression system and rHA - A/H5N1/Poland from a bacterial expression system, as well as with the H5N3 AIV, which are characterized by the highest homology to the immunogen among the H5-serotype antigens used for testing: recombinant HA proteins and AIVs, respectively. Fig. 16 shows the relative reactivity profiles of the obtained clones against recombinant proteins with the H5 HA sequences from various IV strains, whereas Fig. 17 shows the profiles against the H5N1, H5N9 and H5N2 AIVs. The term "relative reactivity" was introduced and described in Example 7. In the case of recombinant antigens, this term means the reactivity level relative to the reactivity with the immunogen (rHA - A/H5N1/Qinghai), and in the case of AIVs - relative to the reactivity with the H5N3 virus. The relative reactivities against recombinant H5 HA proteins and the H5-serotype AIVs are expressed as % of the A₄₅₀ values determined in the tests using the rHA - A/H5N1/Qinghai and the H5N3 AIV, respectively. Information regarding antigens used in the immunoreactivity studies is included in Lists B and C. Recombinant H5 HA proteins are described in detail in Examples 2, 3, 4 and 7. The test methods are described in Example 7.

The G-6-42-42 and G-6-42-71 antibodies differ clearly from the other obtained clones by characteristic reactivity profiles (Fig. 15, 16, 17). In the tests using MediSorp plates (Fig. 15), these antibodies showed a high affinity for the rHA - A/H5N1/Qinghai from a mammalian expression system and the rHA - A/H5N1/Poland from a baculovirus expression system. In comparison to the remaining five clones, the G-6-42-42 and G-6-42-71 mAbs are characterized by the highest reactivities with rHA - A/H5N1/Poland produced in *E. coli,* and also with the H5N3 virus (Fig. 15), the equalized at the level of ~100% relative reactivities against the rHA - A/H5N1/lndia, rHA - A/H5N1/Vietnam and rHA - A/H5N1/Guiyang proteins, and the lowest relative reactivities against the other rHA and rHA1 proteins (Fig. 16). According to the results shown in Fig. 17, the G-6-42-42 and G-6-42-71 antibodies are also distinguished by the significantly lower relative reactivities against the H5N9 AIV than against the H5N2 virus (11%, 10% *vs* 30%, 29%). Similarity of all immunoreactivity profiles for the G-6-42-42 and G-6-42-71 mAbs (Fig. 15, 16, 17) indicates that these antibodies, originated from the cloning of the same primary hybridoma, designated G-6-42 (Table 3), represent one clone. Additionally, the mass spectra of both the G-6-42-42 and the G-6-42-71 antibodies showed the presence of two protein forms (Fig. 7d, e), which was not found for the other clones (Fig. 7a, b, c, f, g).

An important criterion differentiating the remaining of the obtained mAbs: G-1-31-22, G-2-14-10, G-5-32-5, G-7-24-17 and G-7-27-18, are the signal (A₄₅₀) levels reached at the specified antibody concentrations, treated as an indicative measure of the affinity for the antigens and presented in Fig. 15. The profiles of relative reactivities of the G-1-31-22, G-2-14-10, G-5-32-5, G-7-24-17 and G-7-27-18 antibodies against recombinant H5 HA proteins and the H5-serotype AIVs, shown in Fig. 16 and 17, indicate a significant similarity, but also the differences, which became the basis for differentiation of these clones. The key discriminatory antigens were the rHA - A/H5N2/California protein (Fig. 16) and the H5N1 AIV (Fig. 17).

The G-1-31-22 and G-7-24-17 antibodies differ from the G-2-14-10, G-5-32-5 and G-7-27-18 antibodies, *inter alia,* because they show a lower binding affinity for the rHA - A/H5N1/Qinghai protein from a mammalian expression system and the rHA - A/H5N1/Poland protein from a baculovirus expression system adsorbed on MediSorp plates (Fig. 15), as well as for the rHA - A/H5N1/Vietnam protein bound to the Ni-NTA plate (Table 5). Contrary to the other of the obtained clones, the G-1-31-22 and G-7-24-17 antibodies did not show reactivities with bacterial rHA - A/H5N1/Poland protein under the testing conditions, and their reactivities with the H5N3 AIV were the lowest and comparable only to the G-5-32-5 mAb reactivity with this virus (Fig. 15). The results presented in Fig. 16 showed the significantly lower levels of relative reactivities of the G-1-31-22 and G-7-24-17 clones with rHA - A/H5N2/California than those of the G-2-14-10, G-5-32-5 and G-7-27-18 clones (70% *vs* 85-102%). While reactivity profiles of the G-1-31-22 and G-7-24-17 clones, presented in Fig. 15 and 16, are similar, the profiles of the relative reactivities of these antibodies with the H5-serotype AIVs, presented in Fig. 17, indicate that the G-1-31-22 and G-7-24-17 antibodies are the different clones. The G-1-31-22 mAb showed a significantly higher relative reactivity with the H5N1 AIV than the G-7-24-17 mAb (80% *vs* 54%) and the other clones of the obtained antibodies (80% *vs* 45-67%).

The immunoreactivity profiles, presented in Fig. 15, 16 and 17, show that the G-5-32-5 antibodies have features distinguishing them from the G-1-31-22 and G-7-24-17 mAbs, as well as from the G-2-14-10 and G-7-27-18 antibodies. The G-5-32-5 mAb is characterized by a high binding affinity for the rHA - A/H5N1/Qinghai protein from a mammalian expression system and the rHA - A/H5N1/Poland protein from a bculovirus expression system adsorbed on MediSorp plates, and at the same time, by a very weak reactivity with bacterial HA protein and one of the lowest reactivities with the H5N3 AIV (Fig. 15). This clone is distinguished from the other antibodies by the highest level of relative reactivities with rHA - A/H5N2/California, rHA1 - A/H5N1/Vietnam and rHA1 - A/H5N1/HK/483 proteins from a mammalian expression system and rHA - A/H5N1/Poland from a baculovirus expression system, bound to the Ni-NTA plates (Fig. 16), as well as by the lowest level of relative reactivity against the H5N1 AIV (Fig. 17). The obtained results indicate that the G-5-32-5 mAb recognizes glycosylated HA proteins produced in an eukaryotic expression system with a higher affinity than the HA in viral particles.

The G-2-14-10 and G-7-27-18 clones were in a group of antibodies with a high binding affinity for the rHA - A/H5N1/Qinghai protein from a mammalian expression system and the rHA - A/H5N1/Poland protein from a baculovirus expression system adsorbed on MediSorp plates (Fig. 15). Comparing to the G-1-31-22, G-7-24-17 and G-5-32-5 clones, the G-2-14-10 and G-7-27-18 antibodies showed a significant reactivity with rHA - A/H5N1/Poland protein produced in a bacterial expression system, and a higher reactivity with the H5N3 AIV (Fig. 15). The reactivity of the G-7-27-18 mAb with both the bacterial H5 HA and the H5N1 AIV was higher than this of the G-2-14-10 mAb, which indicate that these antibodies are the different clones (Fig. 15). Beside the differences shown in Fig. 15, the G-2-14-10 and G-7-27-18 antibodies are characterized by a substantial similarity in the relative reactivity profiles with recombinant HA proteins and the H5N1, H5N9 and H5N2 viruses (Fig. 16, 17). In terms of the relative reactivities, the G-2-14-10 and G-7-27-18 clones are distinguished from the G-1-31-22, G-7-24-17, G-5-32-5 antibodies primarly by the results obtained in the tests using the rHA - A/H5N2/California protein and the H5N1 AIV (Fig. 16, 17). The relative reactivity levels of the G-2-14-10 and G-7-27-18 mAbs with rHA - A/H5N2/California protein were lower than of the G-5-32-5 mAb and higher than of the G-1-31-22 and G-7-24-17 mAbs, whereas with the H5N1 virus they were lower than of the G-1-31-22 mAb and higher than of the G-7-24-17 and G-5-32-5 mAbs.

### Summary

As a result of the applied procedure for mAb generation by the hybridoma method (Example 1), 7 antibody clones, denoted G-1-31-22, G-2-14-10, G-5-32-5, G-6-42-42, G-6-42-71, G-7-24-17 and G-7-27-18, were obtained. The analysis of the immunoreactivity profiles revealed that the G-1-31-22, G-2-14-10, G-5-32-5, G-7-24-17 and G-7-27-18 mAbs are the different clones, whereas the G-6-42-42 and G-6-42-71 mAbs represent one antibody clone, different from the remaining five clones. Thus, six mAb clones were obtained, which recognize different epitopes specific for the H5 serotype of the hemagglutinin.

### Example 9

### Differentiation of clones based on the peptide maps of Fab fragments

The aim of the studies described below was to obtain the Fc and Fab fragments of the G-1-31-22, G-2-14-10, G-5-32-5, G-6-42-42, G-6-42-71, G-7-24-17 and G-7-27-18 antibodies by ficin digestion and the comparison of their peptide maps generated as a result of trypsinolysis. Based on the recorded mass spectra, the peptides characteristic solely for one specified or only for some of the obtained antibody clones ("discriminatory" peptides), as well as those present in all of the analyzed mAbs ("common" peptides) were determined.

The purified mAbs were concentrated with the use of VivaSpin6 centrifugal concentrators, MWCO 10 000 units (Sartorius Stedim Biotech) with simultaneous buffer exchange to PBS, pH 7.4. Ficin digestion of antibodies was performed using a commercial "Pierce_{TM} Mouse IgG₁ Fab and F(ab')₂ Micro Preparation Kit" (Pierce/Thermo Scientific). Sample preparation, digestion and purification were conducted strictly according to the manufacturer's instructions. The protein at a maximum concentration, i.e., 250 µg in 125 µL solution, was loaded into the ficin-containing column. The digestion time was 5 h. After separation by affinity chromatography, the fractions containing the Fab and Fc fragments, as well as undigested antibodies, were concentrated using VivaSpin6 centrifugal concentrators, MWCO 5 000 units (Sartorius Stedim Biotech) with simultaneous buffer exchange to PBS, pH 7.4.

The concentrated fractions were separated using non-reducing electrophoresis (SDS-PAGE) in two polyacrylamide gel system (5% stacking gel, pH 6.8 and 12.5% resolving gel, pH 8.8). The protein bands corresponding to the Fab and Fc fragments and undigested IgG molecules were excised from the gel, reduced with 10 mM dithiothreitol (Sigma-Aldrich), alkylated with 50 mM iodoacetamide (Sigma-Aldrich) and digested with 10 ng/mL trypsin solution (Promega). Simultaneously, the digestion of the above-mentioned fractions with trypsin in a solution was performed. Each time, the peptide-containing samples were concentrated using a concentrator (Concentrator 5301, Eppendorf) and analyzed by MALDI TOF/TOF mass spectrometry.

Molecular weights and fragmentation ions of the peptides were determined using a MALDI TOF/TOF mass spectrometer (4800 Plus, AB SCIEX). The matrix was the alpha-cyano-4-hydroxy-cinnamic acid dissolved in 0.1% trifluoroacetic acid containing 50% of acetonitrile. The peptide masses were recorded in the reflector-positive mode (MS Reflector Positive Ion) in the range of 900-8000 Da. External calibration was achieved with a 4700 proteomics analyzer calibration mixture (AB SCIEX). The masses of molecules, included into calculations and given in the description, are the monoisotopic masses of the ionized single molecule with a hydrogen atom attached. The peptides were identified based on the obtained peptide maps and fragmentation spectra using databases available in the Mascot system. The theoretical masses of peptides, whose sequences were confirmed using the Mascot system, were calculated with the use of the generally available internet service on the ExPASy server. The peptides found in the protein databases were further described in the text and tables as "identified". In the tables, they are designated with "*" symbol. For a distinction, the peptides not assigned to a known protein were considered as the "non-identified" peptides. In the case of these peptides, the given monoisotopic masses are the means from all of the obtained mass spectra. The data in the tables below represent the results from both applied trypsinolysis methods. The "+" and "-" symbols in the tables referred to the presence and absence of a specified peptide in the peptide maps of the particular mAbs, respectively.

### Peptide maps of Fc fragments

The peptide maps of the Fc fragments from the G-1-31-22, G-2-14-10, G-5-32-5, G-6-42-42, G-6-42-71, G-7-24-17 and G-7-27-18 mAbs and the amino acid sequences of the peptides identified in these maps are presented in Table 7(a, b) included below.

More specifically, Table 7 shows **(a)** peptide maps of the Fc fragments from the G-1-31-22, G-2-14-10, G-5-32-5, G-6-42-42, G-6-42-71, G-7-24-17 and G-7-27-18 mAbs and **(b)** amino acid sequences of the peptides identified in these maps. The peptide maps obtained as a result of trypsinolysis were analyzed using MALDI TOF/TOF mass spectrometry. The peptides were identified based on the obtained peptide maps and fragmentation spectra using databases available in the Mascot system.

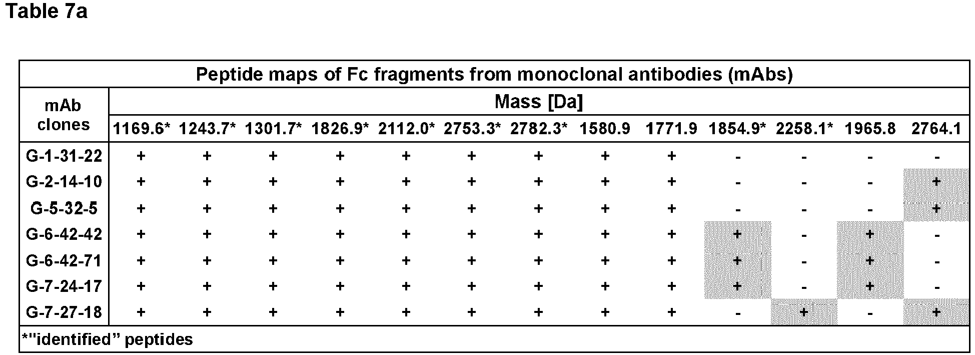

**Table 7b**

| **Amino acid sequences of the peptides identified in the peptide maps of Fc fragments** | |
|---|---|
| **Mass [Da]** | **Amino acid sequence** |
| 1169.6 | IQHQDWTGGK |
| 1243.7 | VNSAAFPAPIEK |
| 1301.7 | VHNEGLPAPIVR |
| 1826.9 | EPQVYVLAPPQEELSK |
| 1854.9 | SVSELPIMHQDWLNGK |
| 2112.0 | FSWFVDDVEVNTATTKPR (1 MC)a |
| 2258.1 | SVSELPIMHQDWLNGKEFK (1 MC)a |
| 2753.3 | STVSLTCMVTSFYPDYIAVEWQR (CYS_CAM)_{b} |
| 2782.3 | DTLTISGTPEVTCVVVDVGHDDPEVK (CYS_CAM)_{b} |
| ₐMC is 1 missed cleavage site | |
| _{b}CYS_CAM is iodoacetamide-modified cysteine (modification was introduced intentionally before trypsin digestion of the protein to avoid reorganization of disulphide bridges during hydrolysis) | |

Analysis of the peptide maps of the Fc fragments from the obtained mAbs allowed to distinguish thirteen peptides (Table 7a). The amino acid sequences could be assigned to nine of these peptides (Table 7b), which were identified as the IgG antibody fragments. The majority of the peptides (9/13) shown in Table 7a are the "common" peptides, among which the seven are the "identified" and the two "non-identified" peptides. The other four peptides are the "discriminatory" fragments (Table 7a). The amino acid sequences of two of them are known (Table 7b). Identical "discriminatory" peptide profiles of the Fc fragments were found in two groups of clones, one of which included the G-6-42-42, G-6-42-71 and G-7-24-17 mAbs, and the other the G-2-14-10 and G-5-32-5 mAbs. Compared to the mAbs from the second group, the G-7-27-18 mAbs contained an additional "discriminatory" peptide. No "discriminatory" fragments were found in the G-1-31-22 clone.

### Peptide maps of Fab fragments

As in the case of Fc fragments, the peptide map analysis for the Fab fragments of the G-1-31-22, G-2-14-10, G-5-32-5, G-6-42-42, G-6-42-71, G-7-24-17 and G-7-27-18 clones allowed to indicate the "common" and "discriminatory" peptides. The "common" peptides from the Fab antibody fragments and the amino acid sequences of the identified "common" peptides are presented in Table 8(a, b) included below.

More specifically, Table 8 shows **(a)** "common" peptides in the peptide maps of the Fab fragments from the G-1-31-22, G-2-14-10, G-5-32-5, G-6-42-42, G-6-42-71, G-7-24-17 and G-7-27-18 mAbs and **(b)** amino acid sequences of the identified "common" peptides. The peptide maps obtained as a result of trypsinolysis were analyzed using MALDI TOF/TOF mass spectrometry. The peptides were identified based on the obtained peptide maps and fragmentation spectra using databases available in the Mascot system.

**Table 8a**

| **Peptide maps of Fab fragments from monoclonal antibodies (mAbs) - "common" peptides** | | | | | | | | | |
|---|---|---|---|---|---|---|---|---|---|
| **mAb clones** | **Mass [Da]** | | | | | | | | |
| | **1696.9*** | **1666.8** | **1678.8** | **2015.0** | **2028.0** | **2037.0** | **2255.2** | **2421.0** | **2439.1** |
| **G-1-31-22** | + | + | + | + | + | + | + | + | + |
| **G-2-14-10** | + | + | + | + | + | + | + | + | + |
| **G-5-32-5** | + | + | + | + | + | + | + | + | + |
| **G-6-42-42** | + | + | + | + | + | + | + | + | + |
| **G-6-42-71** | + | + | + | + | + | + | + | + | + |
| **G-7-24-17** | + | + | + | + | + | + | + | + | + |
| **G-7-27-18** | + | + | + | + | + | + | + | + | + |
| ***"identified" peptides** | | | | | | | | | |

**Table 8b**

| **Amino acid sequences of "common" peptides identified in the peptide maps of Fab fragments** | |
|---|---|
| **Mass [Da]** | **Amino acid sequence** |
| 1696.9 | PAVLNQPSSVSGSLGQR |

Analysis of the peptide maps of the Fab fragments from the obtained antibodies allowed the indication of nine "common" peptides (Table 8a). Among them, only one (1696.9 Da) was identified using the Mascot search engine. It was assigned the amino acid sequence shown in Table 9b. Although for most of the remaining peptides the good quality fragmentation spectra were obtained, they could not be identified using the protein databases.

The "discriminatory" peptides in the peptide maps of the Fab fragments from the G-1-31-22, G-2-14-10, G-5-32-5, G-6-42-42, G-6-42-71, G-7-24-17 and G-7-27-18 antibodies, as well as the amino acid sequences of the identified "discriminatory" peptides are presented in Table 9 (a, b) included below.

More specifically, Table 9 shows **(a)** "discriminatory" peptides in the peptide maps of the Fab fragments from the G-1-31-22, G-2-14-10, G-5-32-5, G-6-42-42, G-6-42-71, G-7-24-17 and G-7-27-18 antibodies and **(b)** amino acid sequences of the identified "discriminatory" peptides. The peptide maps obtained as a result of trypsinolysis were analyzed using MALDI TOF/TOF mass spectrometry. The peptides were identified based on the obtained peptide maps and fragmentation spectra using databases available in the Mascot system.

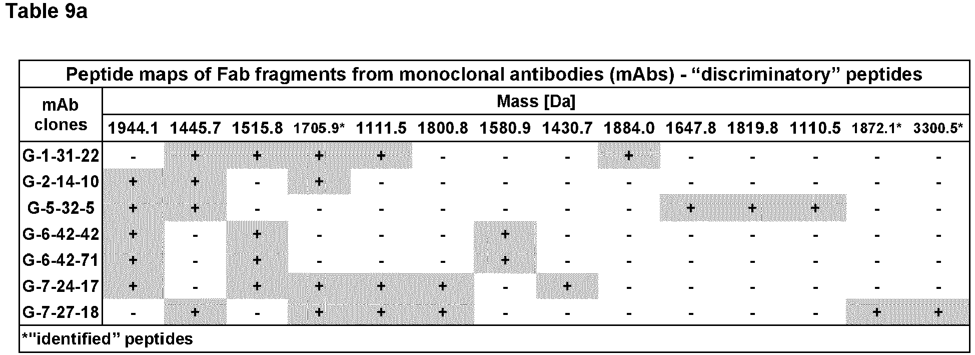

**Table 9b**

| **Amino acid sequences of "discriminatory" peptides identified in the peptide maps of Fab fragments** | |
|---|---|
| **Mass [Da]** | **Amino acid sequence** |
| 1705.9 | LWIYGTSDLASGVPAR |
| 3300.5 | ATLTVDASSSTAYIQLSSLSSEDSAVYFCAR |

Analysis of the peptide maps of the Fab fragments from the obtained antibodies allowed the indication of fourteen "discriminatory" peptides, among which three of the 1705.9 Da, 1872.1 Da and 3300.5 Da masses were identified using the protein databases (Table 9a). To the 1705.9-Da and 3300.5-Da peptides, the sequences were assigned, as shown in Table 9b. Three "discriminatory" peptides were recognized in the Fab fragments of the G-2-14-10, G-6-42-42 and G-6-42-71 clones, five of the G-1-31-22 and G-5-32-5 clones, and six of the G-7-24-17 and G-7-27-18 clones.

### Profiles of "discriminatory" peptides in the Fab fragment peptide maps

The peptide maps of the Fab fragments from the G-1-31-22, G-2-14-10, G-5-32-5, G-6-42-42, G-6-42-71, G-7-24-17 and G-7-27-18 antibodies were subjected to a detailed comparative analysis. In table 10 (a-e), the profiles of the "discriminatory" peptides from the Fab fragments of the individual mAbs were presented and compared with the profiles of the remaining antibodies. Among the "discriminatory" peptides, these which are present in the peptide map of only one, specified antibody clone were indicated. They are further referred to as peptides characteristic for that clone.

More specifically, Table 10 shows the "discriminatory" peptides in the peptide maps of the Fab fragments from the clones: (a) G-6-42-42 and G-6-42-71, (b) G-2-14-10 and G-5-32-5, (c) G-1-31-22, (d) G-7-24-17, (e) G-7-27-18, obtained as a result of trypsinolysis and analyzed using MALDI TOF/TOF mass spectrometry.

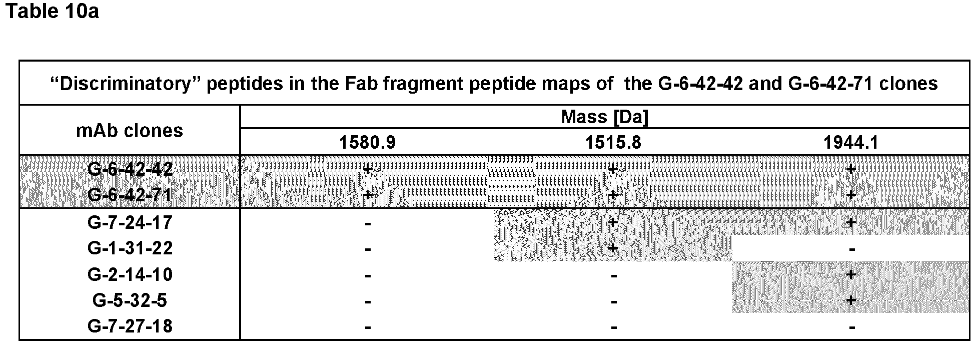

For the G-6-42-42 and G-6-42-71 antibodies, the same "discriminatory" fragments, derived both from the Fab (Table 10a), and Fc (Table 7a) antibody parts, were found. These are the only clones among the generated mAbs, for which identical peptide maps were obtained. It was assumed that they represent one antibody clone. In the Fab part of the G-6-42-42 (G-6-42-71) antibodies, the 1580.9-Da peptide characteristic for this clone was recognized. It was also found that the G-6-42-42 (G-6-42-71) antibodies differ from four out of the five remaining clones in that they contain simultaneously the 1515.8-Da and 1944.1-Da fragments.

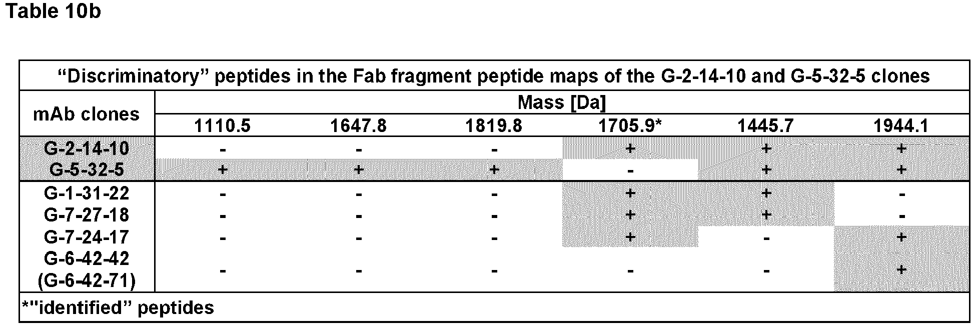

As a result of digestion of the Fab part of the G-2-14-10 clone, three "discriminatory" peptides were obtained (Table 10b), including one peptide with a mass of 1705.9 Da (Table 9b) that was identified. Among the peptides derived from the Fab fragment of the G-2-14-10 antibodies, no peptide characteristic for this clone was found. In the peptide map of the G-5-32-5 antibody Fab fragment, five "non-identified", "discriminatory" peptides were indicated, including three characteristic for this clone: 1110.5 Da, 1647.8 Da and 1819.8 Da (Table 10b). Both the G-2-14-10 mAb and the G-5-32-5 mAb differ from the remaining four clones by the simultaneous presence of the 1445.7-Da and 1944.1-Da peptides. The profiles of the "discriminatory" peptides, presented in Table 10b, indicate that the G-2-14-10 and G-5-32-5 antibodies are the different clones, and at the same time, each of them is different from the remaining antibody clones.

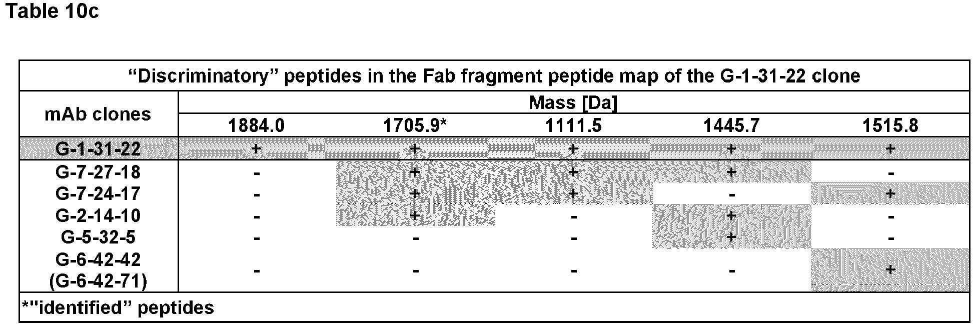

Analysis of the peptide maps of the Fab fragment from the G-1-31-22 clone enabled indication of five "discriminatory" peptides (Table 10c), including one peptide with a mass of 1705.9 Da that was identified (Table 9b). In the G-1-32-22 mAb peptide map, the 1884.0-Da peptide characteristic for this clone was recognized. The G-1-31-22 antibodies differ from the remaining clones also by the simultaneous presence of the "non-identified", 1111.5-Da, 1445.7-Da and 1515.8-Da peptides.

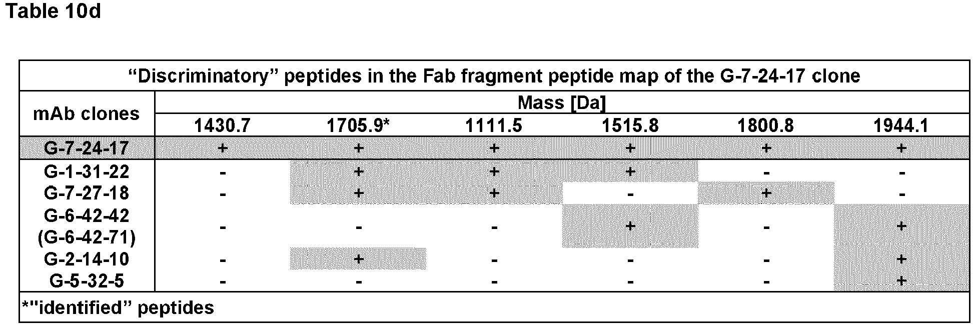

In the peptide map of the Fab fragment of the G-7-24-17 clone, six "discriminatory" peptides were indicated (Table 10d), including one peptide with a mass of 1705.9 Da that was identified (Table 9b). In the Fab part of the G-7-24-17 antibodies, the 1430.7-Da peptide characteristic for this clone was recognized. The G-7-24-17 clone distinguishes also by the simultaneous presence of the 1111.5-Da, 1515.8-Da, 1800.8-Da and 1944.1-Da peptides.

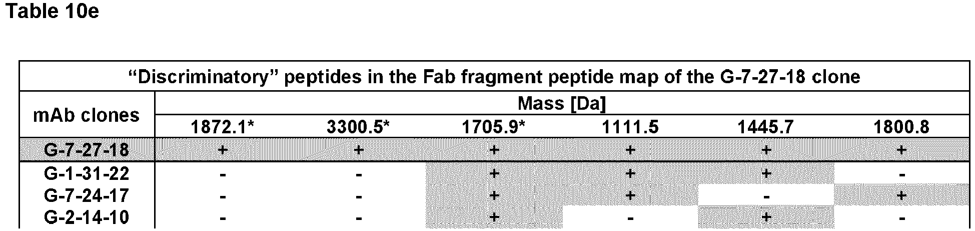

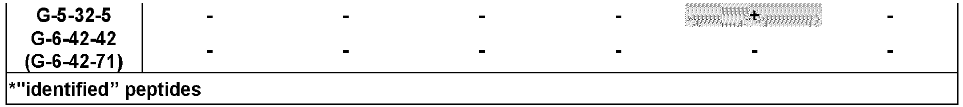

Analysis of the peptide map of the Fab fragment from the G-7-27-18 clone enabled recognition of six "discriminatory" peptides (Table 10e). Among them, there are three peptides with masses of 1705.9 Da, 1872.1 Da and 3300.5 Da that were identified (Table 9b). The 1872.1-Da and 3300.5-Da peptides were found exclusively in the peptide map of the G-7-27-18 mAb. Moreover, the G-7-27-18 clone differs from the remaining antibodies by the simultaneous presence of the 1111.5-Da, 1445.7-Da and 1800.8-Da peptides.

### Summary

As a result of ficin digestion, the Fc and Fab fragments of the G-1-31-22, G-2-14-10, G-5-32-5, G-6-42-42, G-6-42-71, G-7-24-17 and G-7-27-18 antibodies were obtained and then their peptide maps were generated by trypsinolysis. Based on the obtained peptide maps and fragmentation spectra, an attempt was made to identify the peptides using databases available in the Mascot system.

Most of the peptides in the maps of the Fc fragments from the analyzed mAbs (Table 7a) were the "common" peptides (9/13; 69%). The amino acid sequences of most of the peptides derived from the Fc fragments (9/13; 69%) were found in the protein databases (Table 7b). The obtained results are consistent with the current state of knowledge that the Fc fragments are species- and isotype-conserved components of the immunoglobulins, which have no significance for their specificity. Unlike the Fc fragments, in the Fab fragment peptide maps of the examined antibodies (Tables 8a, 9a), the majority were the "discriminatory" peptides (14/23; 61%). Moreover, only a small number of peptides (4/23; 17%) was identified in the Fab fragments (Tables 8b, 9b), substantially less than in the Fc fragments (17% *vs* 69%). The Fab antibody fragments are characterized by a great variability of the specificity-determining sequences, and hence the the databases can be incomplete in these areas.

Of major importance for the identification of the obtained antibodies was a comparative analysis of the peptide maps of their variable fragments responsible for antigen binding, i.e., the Fab fragments. The clone differentiation was based mainly on the presence of peptides characteristic for the individual clones or specified groups of clones ("discriminatory peptides"). The most informative was the presence of peptides having the unique amino acid sequences, which have not been deposited in the protein databases so far ("non-identified discriminatory peptides").

The detailed analysis of the Fab fragment peptide maps showed that the obtained antibodies differ in the number and profile of the "discriminatory" peptides (Table10a-e). Among the seven obtained mAbs, the six distinct antibody clones were recognized and it was shown that the G-6-42-42 and G-6-42-71 antibodies are the same clone. This is consistent with the results from the differentiation of the obtained mAbs based on the immunoreactivity profiles, which were presented in Example 8.

### Example 10 Determination of the obtained mAb activities in the hemagglutination inhibition test

The monoclonal antibodies generated as a result of mouse immunization with rHA - A/H5N1/Qinghai and selected for specificity against H5-serotype HAs were subjected to the HI test. The test uses the ability of antibodies that bind specifically to the specified antigenic sites on the HA molecule, to block binding of the protein to erythrocyte-surface receptors. This is manifested by loss of the antigen ability to agglutinatinate of erythrocytes.

The studies included the purified G-1-31-22, G-2-14-10, G-5-32-5, G-6-42-42, G-6-42-71, G-7-24-17 and G-7-27-18 antibodies. Commercial mAbs against H5 HA (Pierce/Thermo Scientific, Acris Antibodies), denoted mAb 7, mAb 8 and mAb 9, were used as the reference antibodies. According to the specifications, the antibodies recognize H5 HA in the HI test (List A). The HI test was performed using the H5N3 LPAIV as the antigen. Hemagglutinin of this AIV strain is characterized by the highest homology to the immunogen's source sequence among the hemagglutinins of the H5-serotype IVs used in the mAb production procedure (Lists E and F). This concerns the full-length protein and the HA1 subunit, which contains the epitopes for the HI antibodies. The HI test was also performed using the H5N2 LPAIV as the antigen. The comparative analyses of hemagglutinins from the H5N3, H5N1, H5N9 and H5N2 AIVs showed that the HA1 subunit of the H5N2 virus's HA has the lowest homology to the immunogen's HA1 subunit, if all of the homology scores (Max Score, Total Score, Identities) are considered (List F). According to the results of ELISA using four LPAIV strains of the H5 serotype (Example 7), the reactivities of all of the obtained mAb clones against the H5N3 AIV were the highest, whereas against the H5N2 AIV were one of the lower (Fig. 13). They were 24-35% of reactivities determined with the H5N3 virus (Fig. 14). In the HI test, viruses and antisera, certified by the IZSVe, were used. They are described below, in List G.

**List G Influenza viruses and antisera used in the studies of the hemagglutination inhibition activity of the obtained mAbs.**

| **Hl test with H5N3 low-pathogenic avian influenza virus** | | | **Origin** |
|---|---|---|---|
| Antigen | LPAIV | A/duck/It/775/04(H5N3) | x-OvO |
| Positive control | Antiserum | A/duck/It/775/04(H5N3) | x-OvO |
| Negative control | Antiserum | A/macaw/626/80(H7N7) | x-OvO |

| **Hl test with H5N2 low-pathogenic avian influenza virus** | | | **Origin** |
|---|---|---|---|
| Antigen | LPAIV | A/turk/It/80(H5N2) | x-OvO |
| Positive control | Antiserum | A/turk/It/80(H5N2) | x-OvO |
| Negative control | Antiserum | A/macaw/626/80(H7N7) | x-OvO |
| LPAIV - low-pathogenic (LP) avian influenza virus (AIV) | | | |
| x-OvO Limited (Great Britain), Istituto Zooprofilattico Sperimentale delle Venezie (IZSVe, Italy) | | | |

In the test, the fresh preparation of erythrocytes from the SPF chicken blood, obtained from the sterile culture at the DPD, NVRI, was used. Each performed HI test included the positive control, which contained antisera against the H5N3 or H5N2 AIVs incubated with H5N3 or H5N2 viral antigens, respectively. Regardless the antigen used, the negative controls were the samples containing the anti-H7N7 AIV antiserum. For all of the tested antibodies and antisera, the blood cell controls without the viral antigen were performed (internal control for the assay). For each HI test, a hemagglutination unit (HAU) was determined.

To evaluate the HI activities of the obtained mAbs, the series of 2-fold dilutions of the selected antibody clones and their mixtures in PBS-Dulbecco (Sigma-Aldrich) were prepared in the 96-well conical bottom (V) plates (CellStar/Greiner bio-one). In this way, the antibody dilutions in the range from 2x to 4096(8192)x were prepared. The reference anti-H5 HA mAbs were analogically diluted. On each test plate, the serial dilutions of the control antisera against the H5N3 or the H5N2 AIVs (positive control) and the H7N7 AIV (negative control) were prepared, resulting in dilutions from 2× to 4096(8192)x. Next, into each of the wells containing mAbs and antisera diluted 4 and more times in the final volume of 25 µL, 25 µL of the H5N3 or H5N2 AIV suspensions containing 4 HAU were added. Into each of the wells for the blood cell control, containing 2-fold diluted mAbs and antisera, 25 µL of PBS-Dulbecco was added. After a 25-min preliminary incubation at room temperature, 25 µL of 1% erythrocyte suspension was added into each well. The results were observed after a minimum 30-min incubation with blood cells.

According to the test principle, the hemagglutination inhibition was evaluated visually by comparing the samples with blood cell controls. In the HI-positive samples, the erythrocytes are not agglutinated by the viral antigens and therefore they fall freely on the well bottoms, as in the blood cell control samples. The HI titer of monoclonal antibodies was defined as the lowest antibody concentration that causes hemagglutination inhibition. The HI titer of antisera was typically defined as the reciprocal of the highest dilution that inhibits erythrocyte agglutination by the viral antigens. The results of the HI tests for the G-1-31-22, G-2-14-10, G-5-32-5, G-6-42-42, G-6-42-71, G-7-24-17, G-7-27-18 mAbs using the H5N3 and H5N2 AIVs as antigens are presented in Table 11 below together with the results for the reference antibodies and control antisera.

More specifically, Table 11 shows the results from the HI tests for the G-1-31-22, G-2-14-10, G-5-32-5, G-6-42-42, G-6-42-71, G-7-24-17, G-7-27-18 mAbs using the H5N3 and H5N2 LPAIVs (x-OvO) as antigens. Each performed test included the positive control (antiserum against the H5N3 or H5N2 AIVs) and the negative control (anti-H7N7 AIV antiserum), as well as the blood cell controls. In the HI test using the H5N3 AIV, the commercial anti-H5 HA mAbs (Pierce/Thermo Scientific, Acris Antibodies) were used as the reference antibodies.

**Table 11**

| **mAbs and control antisera** | **mAb concentrations [µg/mL] Antiserum dilution** | **Result** | |
|---|---|---|---|
| | | **HI activity** | **HI titer** |
| **HI test with H5N3 low-pathogenic avian influenza virus** | | | |
| G-1-31-22 | 0.3 - 526 | - | - |
| G-2-14-10 | 0.8 - 1011 | - | - |
| G-5-32-5 | 0.9-1008 | - | - |
| G-6-42-42 | 0.7 - 1034 | - | - |
| G-6-42-71 | 0.9 - 973 | - | - |
| G-7-24-17 | 0.5 - 877 | - | - |
| G-7-27-18 | 0.9-1023 | - | - |
| G-1-31-22 + G-2-14-10 + G-5-32-5 + G-6-42-42 + G-6-42-71 + G-7-24-17 + G-7-27-18 | 0.7 - 726 | - | - |
| mAb 9 (Pierce/Thermo Sci., Cat. No. MA1-81928) | 0.1 - 125 | + | 7.8 µg/mL |
| mAb 7 (Acris Antibodies, Cat. No. AM00945PU-N) | 0.1 - 125 | + | 2.0 µg/mL |
| mAb 8 (Acris Antibodies, Cat. No. AM00941PU-N) | 0.1 - 125 | + | 1.0 µg/mL |
| Antiserum against H5N3 LPAIV | 4x - 2048x (4096x) | + | 1:512, 1:1024 |
| Antiserum against H5N7 LPAIV | 4x - 2048x (4096x) | - | - |

| **HI test with H5N2 low-pathogenic avian influenza virus** | | | |
|---|---|---|---|
| G-1-31-22 | 0.5-526 | - | - |
| G-2-14-10 | 1.0-1011 | - | - |
| G-5-32-5 | 1.0 - 1008 | - | - |
| G-6-42-42 | 1.0 - 1034 | - | - |
| G-6-42-71 | 0.9 - 973 | - | - |
| G-7-24-17 | 0.9-877 | - | - |
| G-7-27-18 | 1.0 - 1023 | - | - |
| Antiserum against H5N2 LPAIV | 4× - 4096x | + | 1:512 |
| Antiserum against H5N7 LPAIV | 4× - 4096x | - | - |

The HI tests showed that none of the mAb clones, which were generated as a result of mouse immunization with rHA - A/H5N1/Qinghai and selected for serotype specificity, has the ability to inhibit hemagglutination by the H5N3 and H5N2 AIVs. No HI activity was showed for the G-1-31-22, G-2-14-10, G-5-32-5, G-6-42-42, G-6-42-71, G-7-24-17, G-7-27-18 mAbs, despite using a wide range of antibody concentrations: from sub-micrograms to couple of hundreds of micrograms, wherein some clones were tested at concentrations of up to ~1 mg. Under the same conditions, the HI titers of commercial mAbs, denoted mAb 9, mAb 7 and mAb 8, determined using the H5N3 AIV, were 7.8 µg/mL, 2.0 µg/mL and 1.0 µg/mL, respectively. Correctness of the assay performance was confirmed by the high titers of HI-positive control antisera (anti-H5N3 or anti-H5N2 AIV antisera) and by the lack of the HI activity of negative control antiserum (anti-H7N7 AIV antiserum). Additionally, the observation of blood cell control samples did not showed that the tested antibody samples and antisera affected the quality of erythrocytes.

The G-1-31-22, G-2-14-10, G-5-32-5, G-6-42-42, G-6-42-71, G-7-24-17 and G-7-27-18 antibodies differ from the mAb 7 and mAb 8 having the narrow and broad ranges of specificity against H5 HAs (Fig. 6), respectively, in this that they do not inhibit hemagglutination (Table 11), while maintaining a broad-range serotype specificity (Fig. 11, 13). Negative results of the HI tests for the produced mAbs (Table 11) together with positive results from the studies of their reacivities with H5N3 and H5N2 AIVs by ELISA (Fig. 13) indicate that epitopes recognized by the obtained antibodies may be beyond RBD.

### Example 11

### The use of the produced mAbs in the quality evaluation of H5 hemagglutinin antigens

The main challenge of work on the obtaining subunit vaccines against influenza that are based on the use of hemagglutinin proteins produced with genetic engineering methods, is to obtain the antigens resembling the viral HA. Particularly important for the vaccine HA quality is the structure accuracy the HA1 subunit, where conformational epitopes for neutralizing antibodies are localized. One of the indicators of the HA protein usefulness for vaccine manufacturing is the reactivity of antibodies of known properties with the produced antigens. Antigenicity studies found broad application in the analysis of HA proteins produced in the form of inclusion bodies in bacteria cells. For these proteins, the suitable refolding and purification methods need to be used to obtain a valuable vaccine antigen (S 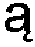 czyńska V., 2014). Because the newly obtained antibody clones recognize exclusively conformational epitopes in the HA1 subunit and show the broad-range specificities against H5 HAs, they can be successfully used in a study the properties of the antigens for production of vaccines against H5-serotype HPIVs regardless of the source sequence for the vaccine antigen. Envisioned is also the use of the generated antibodies in the stability control of the antigens and influenza vaccines. Another possible application for the obtained mAbs is their use to monitor a quality of the H5 HA proteins intended for the use or already used as reagents in diagnostic tests, i.e., for verification whether the conformation of the serotype-specific epitopes in antigens is proper.

To determine applicability of the obtained antibodies in the quality evaluation of the H5 HA antigens, the antigenicity studies of rHA - A/H5N1/Poland from a bacterial expression were performed using the G-6-42-42, G-6-42-71 and G-7-27-18 mAbs. Following purification and refolding, the H5 HA protein (17-522 aa, ΔRRRKKR), produced on the basis of the HA sequence from the A/swan/Poland/305-135V08/2006(H5N1) AIV strain (IBA), was subjected to analyses, which showed the accuracy of its conformation. The test methods and protein properties were described in detail in Example 7. The rHA - A/H5N1/Poland was analyzed before and after denaturation under reducing conditions. Denaturation was conducted by diluting the protein preparation in a 2x concentrated denaturation buffer (4% SDS, 625 mM β-mercaptoethanol, 120 mM Tris-HCI, pH 8.0) in the 1:1 ratio (v/v) and by heating the obtained solution at 99oC for 10 minutes. The antigenicity study was performed by ELISA on PolySorp, MediSorp, MaxiSorp and MultiSorp plates (NUNC) coated with conformational and denatured rHA preparation, ~80%pure, which contained hemagglutinin at a concentration of ~1 µg/mL. The 2-fold serially diluted G-6-42-42, G-6-42-71 and G-7-27-18 mAbs were applied to the plates blocked with 10% FBS/PBS. The assay was performed under conditions described in Example 7. The concentrations interpolated from the 4-parameter titration curves of the antibodies against the bacterial HA protein were determined using the Gene5 program (Bio-Tek).

Fig. 18 and 19 show the results from antigenicity studies of the conformational and denatured rHA - A/H5N1/Poland protein from a bacterial expression system using the G-6-42-42, G-6-42-71 and G-7-27-18 antibodies. The differences in the titration curves of the G-6-42-42, G-6-42-71 and G-7-27-18 mAbs against the conformational and denatured rHA protein, manifested by the high levels of reads (A₄₅₀) from the assays with the protein before denaturation and the very low reads after denaturation, indicate the loss of the proper conformation by the HA protein under denaturing conditions. In contrary to the results obtained with conformational rHA protein, the A₄₅₀ values read in the tests with denatured antigen depended on the plate type used for antigen binding. They were decreasing with an increase in hydrophilicity of the surface being coated. The highest signals from antibody titrations were read with the antigen, which, after denaturation, was adsorbed on the PolySorp plates having a high binding affinity for the hydrophobic molecules. The concentrations interpolated for the A₄₅₀ = 1.0 from the titration curves of the G-6-42-42, G-6-42-71 and G-7-27-18 mAbs against the denatured, bacterial HA protein bound to the plate of this type, were 338-, 389- and 35-fold higher, respectively, than those against the conformational protein. The denatured antigen bound to the MultiSorp plates of the highest hydrophilicity was not recognized by the antibodies used in the studies.

The results described in the present example confirm earlier findings that the obtained G-6-42-42, G-6-42-71 and G-7-27-18 antibodies recognize conformational epitopes of hemagglutinin (Examples 5, 7). A certain reactivity level of these antibodies with the rHA protein subjected to denaturation, observed in the tests using some types of plates, probably resulted from a partial protein refolding after dilution in PBS under plate-coating conditions.

The obtained results confirm usefulness of the produced antibodies for studying the properties of the newly generated H5 HA proteins as well as for the detection of changes of these proteins during storage. This is the case of both the antigens for the production of vaccines against H5-serotype IVs, e.g., H5N1 HPAIV, and the antigens used in diagnostic tests.

### Example 12

### The use of the generated mAbs in diagnostic tests

To show a possible application of the generated mAbs in diagnostic tests, the prototype blocking ELISA was developed for the detection of anti-H5 HA antibodies (bELISA H5). The G-7-27-18 clone was used in the bELISA H5 test. As the antigen, the 17-530-aa protein (ΔRRRKKR, 6x His) having the HA sequence from the A/swan/Poland/305-135V08/2006 strain of the H5N1 virus, produced in a baculovirus expression system (OET), was used. The protein properties were described in detail in Example 2. The bELISA H5 test was optimized. The assays using the polystyrene plates with various polarity enabled selection of the best plate for the antigen binding and epitop presentation to the G-7-27-18 mAb. In the additional experiments, preferable conditions for plate incubation at particular stages of the determination procedure were specified. The optimum concentrations/dilutions of the reagents were determined by titration.

In the optimal variant of the bELISA H5 test, MediSorp plates (NUNC) were coated with the antigen at a concentration of 0.5 µg/mL (50 µL/well) overnight at 2-8ₒC. The non-specific binding sites on the plates were blocked for 1 h at room temperature with Protein-Free T20 (PBS) Blocking Buffer (200 µL/well) from Pierce. Into each well for the mAb control, 100 µL of incubation buffer (1% BSA/PBS) was added, while into each of the remaining wells of the plate - 50 µl of incubation buffer was added. The control and tested serum samples were diluted in the plate wells by adding 50 µL of serum to 50 µL of 1% BSA/PBS. The samples were incubated on the plates for 1h at 37ₒC with shaking (150 rpm). Next, the 7-27-18 mAb, diluted to 1 µg/mL in Antibody Stabilizer PBS (Candor Bioscience), was added to the plate (50 µL/well), which was then incubated again for 1h at 37oC and 150 rpm. For the detection of antigen-bound mAbs, the HRP-labelled antibodies against mouse IgG, γ-chain specific (Sigma-Aldrich), were used. The secondary antibodies were diluted 1:3500 in HRP-Protector (Candor Bioscience) and incubated on the plates (50 µL/well) for 1 h at 37ₒC and 150 rpm. TMB (Sigma-Aldrich) was used as a substrate for HRP (50 µl/well). After 15 min of incubation at room temperature, the reaction was stopped with 0.5 M H₂SO₄ solution (50 µL/well). The absorptions of the samples were read at λ=450 nm.

The assays were carried out in the presence of control samples. The control of maximum binding of the 7-27-18 mAb with H5 HA antigen (mAb control) was obtained in the plate wells, into which the serum was not added. The normal chicken serum (Abcam) was used as the negative control, whereas the antisera obtained by immunization of SPF chickens with inactivated H5N3 and H5N2 AIVs (x-OvO) were used as the positive controls. The antiserum against the H5N3 AIV was the strong positive control and the antiserum against the H5N2 AIV was the weak positive control. According to the above protocol, 13 bELISA H5 tests were performed. The mAb control was determined in 8 repeats on each plate. The remaining control samples and serum test samples were analyzed in duplicates. The level of inhibition for the 7-27-18 mAb-antigen binding by sera, expressed as a percentage, was calculated according to the formula: inhibition% = 100 - [(A₄₅₀ sample/A₄₅₀ mAb control) x100]. In the calculations, the mean A₄₅₀ values for the mAb control and the tested samples were included.

Preliminary evaluation of the diagnostic value of the BELISA H5 test was performed using the commercially available chicken sera (Abcam, x-OvO), the sera from the DPD of the NVRI, as well as the serum samples prepared during the chicken immunization studies conducted at the IBA. The sera intended for the analysis were previously tested for the presence of antibodies against influenza virus HA using the HI test. The HI test enables the classification of the animals as the anti-HA positive or negative, as well as the determination of the antiserum serotype specificities. In the validation procedures of the tests for IV infection diagnostics, the HI test is treated as a so-called "gold standard". The HI titers of the normal chicken serum (Abcam) and sera from the immunization studies (IBA) were determined at the IBA using the H5N2 AIV (x-OvO) as the antigen. Antisera (x-OvO), certified by the IZSVe, were characterized using the specified HI titer values.

The anti-H5-negative sera were from a group of the non-vaccinated chickens differentiated in terms of type, breed, age and rearing conditions. The anti-H5-negative sera, positive against HAs of the H1-H4 and H6-H16 serotypes (x-OvO), were obtained by immunization of SPF chickens with inactivated AIVs, shown in List C. The anti-H5-positive sera were obtained by animal vaccination with H5 HA antigens, in particular, the SPF chickens with inactivated H5N1, H5N2, H5N3, H5N9 AIVs (x-OvO) shown in List C and the non-SPF chickens with recombinant H5 HA protein (rH5-*E*. *coli).* The rH5-*E*. *coli* protein (the first antigenic variant) was produced at the IBA based on the HA sequence from the A/swan/Poland/305-135V08/2006 strain of the H5N1 virus. The protein properties were described in Example 7. To obtain antisera, the H5 HA antigens with varied sequences were used. The relative homology scores of these antigens are shown in Lists E and F. The samples used in the preliminary evaluation of the diagnostic value of the bELISA H5 test, are described in detail in the List below.

**List H Samples of chicken sera analyzed by the bELISA H5 test (IBA) using the G-7-27-18 mAb.**

| **Sera** | **Number of chickens/series** | **Number of samples** | **HI titer** | **Origin** |
|---|---|---|---|---|
| **Controls for bELISA** | | | | |
| Negative control Normal chicken serum | 1 series | 1 | < 1:8₆ | Abcam (Cat. No. ab7477) |
| Positive control, strong anti-H5N3 AIV (1)₁ | 1 series | 1 | 1:512₇ | x-OvO* |
| Positive control, weak anti-H5N2 AIV (3)₁ | 1 series | 1 | 1:512₇ | x-OvO* |

| **Anti-H5-negative (1)** | | | | |
|---|---|---|---|---|
| SPF, layer-type chickens₂ | 10 | 18 | n.d.₈ | DPD, NVRI ** |
| Layer-type chickens₃ | 30 | 130 | <1:8₆ | IBA*** |
| Broiler-type chickens₄ | 42 | 61 | < 1:8₆ | controls in immunization studies |

| **Anti-H5-negative** (2) | | | | |
|---|---|---|---|---|
| Anti-AIVs: H1-H4, H6-H12, H14-H16₁ | 1 series of each | 20 | positive₇ | x-OvO* |
| Anti-H 13 AIV₁ | 2 series | 2 | positive₇ | x-OvO* |

| **Anti-H5-positive (1)** | | | | |
|---|---|---|---|---|
| Anti-H5N1 AIV₁ | 1 series | 1 | 1:512₇ | x-OvO* |
| Anti-H5N2 AIV₁ | 3 series | 3 | 1:256; 1:512₇ | x-OvO* |
| Anti-H5N3 AIV₁ | 3 series | 3 | 1:512₇ | x-OvO* |
| Anti-H5N9 AIV₁ | 3 series | 3 | 1:256; 1:512₇ | x-OvO* |

| **Anti-H5-positive (2)** | | | | |
|---|---|---|---|---|
| Layer-type chickens₅ | 69 | 115 | 1:8 ÷ 1:512₆ | IBA*** IBA*** immunization studies |
| ₁SPF chickens were immunized with inactivated AIVs of H1-H16 serotypes (List C). | | | | |
| ₂Blood was collected on 63 and/or 77 days of life of SPF, layer-type chickens (White Leghorn) (1 or 2 collections from 1 chicken). | | | | |
| ₃Blood was collected from non-SPF, layer-type chickens (Rossa 1) on 49, 56, 63, 70 and 77 days of animal life (3-5 collections from 1 chicken). | | | | |
| ₄Blood was collected from non-SPF, broiler-type chickens (Ross 308) on 21, 35, 38, 42 and 49 days of animal life (1-4 collections from 1 chicken). | | | | |
| ₅Non-SPF, layer-type chickens (Rossa 1) were vaccinated with rH5- *E. coli* protein having the HA sequence from the A/swan/Poland/305-135V08/2006 strain of the H5N1 AIV (EpiFluDatabase Accession No. EPI156789). The blood was collected 1 and/or 2 weeks following administration of the 2_{nd} dose of the antigen, on 56 and/or 63 days of animal life, or on 70 and/or 77 days of animal life (1 or 2 collections from 1 chicken). | | | | |
| ₆determined using the H5N2 AIV (x-OvO) as the antigen and HIU=1:8 (IBA) | | | | |
| ₇according to the specification (x-OvO) | | | | |
| ₈ not determined | | | | |
| *x-OvO Limited (Great Britain), Istituto Zooprofilattico Sperimentale delle Venezie (IZSVe, Italy) | | | | |
| **Department of Poultry Diseases, National Veterinary Research Institute (Pulawy, Poland) | | | | |
| ***Institute of Biotechnology and Antibiotics (Warsaw, Poland) | | | | |

Analyses of the H5-negative sera with bELISA H5 enabled determination of the cut-off value for the developed test (mean value of % inhibition + 2xSD), and thereby the interpretation of the results for the tested samples. The % inhibition values higher than the cut-off value mean that the samples are seropositive, and the lower ones that they are seronegative. The results of sample analyses with the use of the bELISA H5 test in relation to the cut-off value are presented in Fig. 20. The data from the assays of the negative control and anti-H 1 (H2-H16)-positive sera was presented as the mean values of the % inhibition from a specified number of independent determinations (n) of the particular samples. The results of the remaining analyses were presented as the mean values of the % inhibition from a given number of samples (n) in the particular serum groups.

The mean values of the % inhibition for the negative control, anti-H5-negative sera from non-immunized chickens and chickens immunized with AIVs of the serotypes other than H5 (H1-H4, H6-H16) are in Fig. 20 below the cut-off value. In turn, the mean % inhibition values for the anti-H5-positive sera from the chickens immunized with H5-serotype AIVs and rH5-*E*. *coli* protein are above the cut-off value. The obtained results indicate that the prototype bELISA H5 test allows to distinguish the chickens immunized with H5 HA antigens from the anti-H5-negative chickens. Importantly, the antisera against AIVs of the H1-H4 and H6-H16 serotypes do not show cross-reactions in the developed test. Presumably, the bELISA H5 test would enable also unambiguous diagnostics of the animals infected with H5-serotype IVs, such as the H5N1 HPAIVs. In the case of vaccinations against HPAIVs with H5 HA proteins, the bELISA H5 test together with the test for the detection of antibodies against the other IV antigens, e.g., neuraminidase, could be used as a so-called DIVA test designated for the differentiation of infected from vaccinated animals

Characterization of the prototype bELISA H5 test was expanded with the preliminary determination of the key validation parameters of the diagnostic test. The assay repeatability was determined by performing independent determinations for the mAb control, negative control (normal chicken serum) and positive controls (antisera against the H5N3 and H5N2 AIVs). Analytical (Asp) and diagnostic (Dsp) specificities of the test were evaluated by calculating the number of the samples denoted as true negative (TN) among the sera from chickens immunized with AIVs of the H1-H4, H6-H16 serotypes (Asp) and among the anti-H5-negative sera from non-immunized chicken group (Dsp). Diagnostic sensitivity (Dse) of the bELISA H5 test was determined by calculating the number of the samples denoted as true positive (TP) among the sera from chickens immunized with H5-serotype AIVs (H5N1, H5N2, H5N3 and H5N9) or rH5-*E*. *coli* protein. The results from the validation parameter determinations for the bELISA H5 test are presented in Table 12 included below.

More specifically, Table 12 shows the results from the preliminary determination of the validation parameters of the blocking ELISA for the detection of antibodies against H5 HAs (bELISA H5) in chicken sera, which was developed and optimized at the IBA. In the bELISA H5 test, the G-7-27-18 mAb was used. Analyzed samples were previously classified as anti-H5 positive or negative based on the results of the HI test. The assay repeatability was determined by performing independent determinations for the control samples. Analytical (Asp) and diagnostic (Dsp) specificities of the test were evaluated by calculating the number of the samples denoted as true negative (TN) among the anti-H5-negative sera. Diagnostic sensitivity (Dse) was determined by calculating the number of samples denoted as true positive (TP) among the anti-H5-positive sera.

**Table 12**

| **Sera** | **Number of samples** | **Number of assays** | **Result** | | |
|---|---|---|---|---|---|
| **Repeatability of assays [A₄₅₀]** | | | **Mean** | **SD** | **RSD** |
| G-7-27-18 mAb control | 1 | 13 | 1.566 | 0.111 | 7.1% |
| Negative control Normal chicken serum | 1 | 13 | 1.460 | 0.114 | 7.8% |
| Positive control, strong anti-H5N3 AIV (1) | 1 | 12 | 0.439 | 0.044 | 10.0% |
| Positive control, weak anti-H5N2 AIV (3) | 1 | 12 | 0.944 | 0.083 | 8.8% |

| **Analytical specificity (Asp)** | | | **TN** | **FP** | **Asp TN/(TN+FP)** |
|---|---|---|---|---|---|
| anti-H1 (H2-H4, H6-H16) AIV | 22 | 43 | 43 | 0 | 100% |

| **Diagnostic specificity (Dsp) TN FP** | | | | | **Dsp TN/(TN+FP)** |
|---|---|---|---|---|---|
| anti-H5-negative | 209 | 209 | 204 | 5 | 97.6% |

| **Diagnostic sensitivity 1 (Dse 1)** | | | **TP** | **FN** | **Dse** |
|---|---|---|---|---|---|
| | | | | | **TP/(TP+FN)** |
| anti-H5 AIV | 10 | 99 | 97 | 2 | 98.0% |

| **Diagnostic sensitivity 2 (Dse 2)** | | | **TP** | **FN** | **Dse TP/(TP+FN)** |
|---|---|---|---|---|---|
| anti-H5 | 115 | 115 | 114 | 1 | 99.1% |
| **TN - true negative** | **FP - false positive** | | **TP - true positive** | **FN - false negative** | |

The assay results for the control, anti-H5 negative and positive samples indicate that the prototype bELISA H5 test is characterized by a satisfying repeatability (RSD: 7.1% - 10.0%) and the high factors of the analytical specificity (Asp: 100%), as well as the diagnostic specificity and sensitivity (Dsp: 97.6%; Dse 1: 98.0%; Dse 2: 99.1%).

### LITERATURE

Cao Z et al. The epitope and neutralization mechanism of AVFluIgG01, a broad-reactive human monoclonal antibody against H5N1 influenza virus. PLoS One. 2012; 7:e38126.
Chiu FF et al. Immunological study of HA1 domain of hemagglutinin of influenza H5N1 virus. Biochem Biophys Res Commun. 2009; 383:27-31.
Corti D et al. A neutralizing antibody selected from plasma cells that binds to group 1 and group 2 influenza A hemagglutinins. Science. 2011; 333:850-6.
Dlugolenski D et al. Production of H5-specific monoclonal antibodies and the development of a competitive enzyme-linked immunosorbent assay for detection of H5 antibodies in multiple species. Avian Dis. 2010; 54(1 Suppl):644-9.
Du L et al. Identification and structural characterization of a broadly neutralizing antibody targeting a novel conserved epitope on the influenza virus H5N1 hemagglutinin. J Virol. 2013; 87:2215-25.
Ekiert DC et al. Antibody recognition of a highly conserved influenza virus epitope. Science. 2009; 324:246-51.
Grabowska I et al. Electrochemical biosensors for detection of avian influenza virus--current status and future trends. Acta Biochim Pol. 2014; 61:471-8.
Ha Y et al. H5 avian and H9 swine influenza virus haemagglutinin structures: possible origin of influenza subtypes. EMBO J. 2002; 21:865-75.
Hvistendahl M. Avian influenza. Enigmatic bird flu strain races across the U.S. Midwest. Science. 2015; 348:741-2.
**ICTV**, International Committee On Taxonomy Of Viruses, Taxonomy History, ICTV, 2014. [http://www.ictvonline.org/virusTaxonomy.asp?taxnode_id=20142704]
Ip HS et al. Novel Eurasian highly pathogenic avian influenza A H5 viruses in wild birds, Washington, USA, 2014. Emerg Infect Dis. 2015; 21:886-90.
Jarocka U et al. An immunosensor based on antibody binding fragments attached to gold nanoparticles for the detection of peptides derived from avian influenza hemagglutinin H5. Sensors (Basel). 2014; 14:15714-28.
Koh YT et al. Immunological consequences of using three different clinical/laboratory techniques of emulsifying peptide-based vaccines in incomplete Freund's adjuvant. J Transl Med. 2006; 4:42.
Köhler G, Milstein C. Continuous cultures of fused cells secreting antibody of predefined specificity. Nature. 1975; 256:495-7.
Lamb RA, Choppin PW. The gene structure and replication of influenza virus. Annu Rev Biochem. 1983; 52:467-506.
Lapolla A et al. Evaluation of IgG glycation levels by matrix-assisted laser desorption/ionization mass spectrometry. Rapid Commun Mass Spectrom. 1997; 11:1342-6.
Lapolla A et al. (a) Matrix-assisted laser desorption/ionization mass spectrometry, enzymatic digestion, and molecular modeling in the study of nonenzymatic glycation of IgG. J Am Soc Mass Spectrom. 2000; 11:153-9.
Lapolla A et al. (b) The role of mass spectrometry in the study of non-enzymatic protein glycation in diabetes. Mass Spectrom Rev. 2000; 19:279-304.
Lebarbenchon C et al. Evaluation of a commercial enzyme-linked immunosorbent assay for detection of antibodies against the H5 subtype of Influenza A virus in waterfowl. Influenza Other Respir Viruses. 2013; 7:1237-40.
Miyagawa E et al. Development of a novel rapid immunochromatographic test specific for the H5 influenza virus. J Virol Methods. 2011; 173:213-9.
Oh HL et al. An antibody against a novel and conserved epitope in the hemagglutinin 1 subunit neutralizes numerous H5N1 influenza viruses. J Virol. 2010;84: 8275-8286.
Okuno Y et al. A common neutralizing epitope conserved between the hemagglutinins of influenza A virus H1 and H2 strains. J Virol. 1993; 67:2552-8.
Petric M et al. Role of the laboratory in diagnosis of influenza during seasonal epidemics and potential pandemics. J Infect Dis. 2006; 194 Suppl 2:S98-110.
Postel A et al. Broad spectrum reactivity versus subtype specificity-trade-offs in serodiagnosis of influenza A virus infections by competitive ELISA. J Virol Methods. 2011; 173:49-59.
Prabakaran M et al. Development of epitope-blocking ELISA for universal detection of antibodies to human H5N1 influenza viruses. PLoS One.2009; 4:e4566.
Rowe T et al. Detection of antibody to avian influenza A (H5N1) virus in human serum by using a combination of serologic assays. J Clin Microbiol. 1999; 37:937-43.
**S** 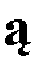 **czyńska V**. Influenza virus hemagglutinin as a vaccine antigen produced in bacteria. Acta Biochim Pol. 2014; 61:561-72.
Skehel JJ, Wiley DC. Receptor binding and membrane fusion in virus entry: the influenza hemagglutinin. Annu Rev Biochem. 2000; 69:531-69.
Steinhauer DA. Role of hemagglutinin cleavage for the pathogenicity of influenza virus. Virology. 1999; 258:1-20.
Stelzer-Braid S et al. A commercial ELISA detects high levels of human H5 antibody but cross-reacts with influenza A antibodies. J Clin Virol. 2008; 43:241-3.
Suarez DL. Overview of avian influenza DIVA test strategies. Biologicals. 2005; 33:221-6.
Sui J et al. Structural and functional bases for broad-spectrum neutralization of avian and human influenza A viruses. Nat Struct Mol Biol. 2009; 16:265-73.
Szewczyk B et al. Introduction to molecular biology of influenza a viruses. Acta Biochim Pol. 2014; 61:397-401.
Verhagen JH et al. Infectious disease. How a virus travels the world. Science. 2015 6; 347:616-7.
Wang SF et al. Generating and characterizing monoclonal and polyclonal antibodies against avian H5N1 hemagglutinin protein. Biochem Biophys Res Commun. 2009; 382:691-6.
Wilson IA et al. Structure of the haemagglutinin membrane glycoprotein of influenza virus at 3 A resolution. Nature. 1981; 289:366-73.
Wu R et al. A novel neutralizing antibody against diverse clades of H5N1 influenza virus and its mutants capable of airborne transmission. Antiviral Res. 2014; 106:13-23.
Xu X et al. Genetic characterization of the pathogenic influenza A/Goose/Guangdong/1/96 (H5N1) virus: similarity of its hemagglutinin gene to those of H5N1 viruses from the 1997 outbreaks in Hong Kong. Virology. 1999; 261:15-9.
Yang M et al. Production and diagnostic application of monoclonal antibodies against influenza virus H5. J Virol Methods. 2009; 162:194-202.

## Claims

1. A composition of monoclonal antibodies against hemagglutinin of H5-serotype influenza viruses lacking hemagglutination inhibiting (HI) activity that bind to epitopes of native forms of H5 antigens, and exhibit no cross-reactivity with non-H5 serotype viruses,
(i) G-1-31-22 produced by mouse hybridoma cell line G-1-31-22 deposited with the DSMZ under the number DSM ACC3292,
(ii) G-2-14-10 produced by mouse hybridoma cell line G-2-14-10 deposited with the DSMZ under the number DSM ACC3293,
(iii) G-5-32-5 produced by mouse hybridoma cell line G-5-32-5 deposited with the DSMZ under the number DSM ACC3294,
(iv) G-6-42-42 produced by mouse hybridoma cell line G-6-42-42 deposited with the DSMZ under the number DSM ACC3295,
(v) G-7-24-17 produced by mouse hybridoma cell line G-7-24-17 deposited with the DSMZ under the number DSM ACC3296, and
(vi) G-7-27-18 produced by mouse hybridoma cell line G-7-27-18 deposited with the DSMZ under the number DSM ACC3297;
or wherein the composition comprises fragments of each of said antibodies wherein said fragments are Fab, Fab', F(ab')₂, Fv or single-chain variable fragments (scFv) consisting of V_{L} and V_{H} domains bound to each other by a peptide linker.

2. A composition of monoclonal antibodies according to claim 1, **characterized in that** hemagglutinin is from influenza viruses of the H5 serotype, including H5N1, H5N2, H5N3 and H5N9.

3. A composition of monoclonal antibodies according to any of the claims 1 -2, **characterized in that** a monoclonal antibody is conjugated with an analytically detectable label, prodrug, drug, therapeutic agent, peptide, protein, enzyme, virus, lipid, PEG.

4. Mouse hybridoma cell lines selected from the group comprising:
(i) G-1-31-22 deposited with the DSMZ under the number DSM ACC3292,
(ii) G-2-14-10 deposited with the DSMZ under the number DSM ACC3293,
(iii) G-5-32-5 deposited with the DSMZ under the number DSM ACC3294,
(iv) G-6-42-42 deposited with the DSMZ under the number DSM ACC3295,
(v) G-7-24-17 deposited with the DSMZ under the number DSM ACC3296 and
(vi) G-7-27-18 deposited with the DSMZ under the number DSM ACC3297.

5. Pharmaceutical or diagnostic composition comprising a composition of antibodies defined in claims 1-3 and a suitable carrier or label.

6. A composition as defined in claim 1 for use in treatment of infections evoked by influenza viruses of the H5 serotype.

7. A method for in vitro diagnosing infections with influenza viruses of the H5 serotype, comprising contacting a biological sample with a composition of monoclonal antibodies as defined in claims 1-3 and detecting the binding of said antibodies with H5-serotype influenza viruses or H5 hemagglutinin protein.

8. A diagnostic kit suitable for in vitro detection of infections with H5-serotype influenza viruses, containing the composition of monoclonal antibodies as defined in claim 1 or their fragments as defined in claim 1 or a composition of monoclonal antibodies as defined in claim 3, and reagents for the detection of antibodies or their functional fragments bound with H5-serotype influenza viruses or H5 hemagglutinin protein.

9. A diagnostic kit suitable for in vitro detection, quantification or semi-quantification of H5-serotype influenza viruses in biological samples, comprising the composition of monoclonal antibodies as defined in claims 1-3 and tools and reagents commonly used in the immunological tests.

10. A diagnostic kit suitable for in vitro detection, quantification or semi-quantification of antibodies against H5-serotype influenza viruses in biological samples, comprising the composition of monoclonal antibodies as defined in claims 1-3 and tools and reagents commonly used in the immunological tests.

11. The diagnostic kit according to one of the claims 8-10, in which the antibodies are in the non-isolated form or are isolated and purified.

12. The diagnostic kit according to one of the claims 8-11, **characterized in that** it is used in the tests selected from the group comprising: immunoenzymatic, preferably bELISA H5, immunofluorescent, immunochemiluminescent, radioimmunological, immunochromatographic, immunodiffusion, immunoprecipitation tests, a test using immunosensors that can be used as so-called DIVA tests for the differentiation of infected from vaccinated animals.

13. The diagnostic kit according to one of the claims 8-12, **characterized in that** it allows the differentiation of infected from vaccinated animals also known as DIVA (Differentiation of Infected from Vaccinated Animals) test.

## Patentansprüche

1. Eine Zusammensetzung monoklonaler Antikörper gegen Hämagglutinin von H5-Serotyp-Grippeviren ohne Hämagglutinin-Inhibitionsaktivität (HI), die an Epitope nativer Formen von H5-Antigenen binden und keine Kreuzreaktivität mit Nicht-H5-Serotyp-Viren aufweisen, umfassend:
(i) G-1-31-22 hergestellt von Maus-Hybridoma-Zelllinie G-1-31-22, die beim DSMZ unter Nummer DSM ACC3292 hinterlegt ist,
(ii) G-2-14-10 hergestellt von Maus-Hybridoma-Zelllinie G-2-14-10, die beim DSMZ unter Nummer DSM ACC3293 hinterlegt ist,
(iii) G-5-32-5 hergestellt von Maus-Hybridoma-Zelllinie G-5-32-5, die beim DSMZ unter Nummer DSM ACC3294 hinterlegt ist,
(iv) G-6-42-42 hergestellt von Maus-Hybridoma-Zelllinie G-6-42-42, die beim DSMZ unter Nummer DSM ACC3295 hinterlegt ist,
(v) G-7-24-17 hergestellt von Maus-Hybridoma-Zelllinie G-7-24-17, die beim DSMZ unter Nummer DSM ACC3296 hinterlegt ist, und
(vi) G-7-27-18 hergestellt von Maus-Hybridoma-Zelllinie G-7-27-18, die beim DSMZ unter Nummer DSM ACC3297 hinterlegt ist;
oder wobei die Zusammensetzung Fragmente jedes dieser Antikörper umfasst, wobei die Fragmente Fab, Fab', F(ab')₂, Fv oder einkettige variable Fragmente (scFv) sind, die aus V_{L}- und V_{H}-Domänen bestehen, die aneinander durch einen Peptidlinker gebunden sind.

2. Eine Zusammensetzung monoklonaler Antikörper nach Anspruch 1, dadurch charakterisiert, dass das Hämagglutinin von Grippeviren des H5-Serotyps ist, einschließlich H5N1, H5N2, H5N3 und H5N9.

3. Eine Zusammensetzung monoklonaler Antikörper nach einem der Ansprüche 1-2, dadurch charakterisiert, dass ein monoklonaler Antikörper mit einem analytisch detektierbaren Label, einer Wirkstoffvorstufe, einem Wirkstoff, einem therapeutischen Agens, einem Peptid, einem Protein, einem Enzym, einem Virus, einem Lipid oder PEG konjugiert ist.

4. Maushybridomzelllinien, ausgewählt aus der Gruppe umfassend:
(i) G-1-31-22, die beim DSMZ unter Nummer DSM ACC3292 hinterlegt ist,
(ii) G-2-14-10, die beim DSMZ unter Nummer DSM ACC3293 hinterlegt ist,
(iii) G-5-32-5, die beim DSMZ unter Nummer DSM ACC3294 hinterlegt ist,
(iv) G-6-42-42, die beim DSMZ unter Nummer DSM ACC3295 hinterlegt ist,
(v) G-7-24-17, die beim DSMZ unter Nummer DSM ACC3296 hinterlegt ist und
(vi) G-7-27-18, die beim DSMZ unter Nummer DSM ACC3297 hinterlegt ist.

5. Pharmazeutische oder diagnostische Zusammensetzung, umfassend eine Zusammensetzung von in den Ansprüchen 1-3 definierten Antikörpern und einem geeigneten Träger oder Label.

6. Eine Zusammensetzung wie in Anspruch 1 definiert, zur Verwendung in der Behandlung von Infektionen, die von Grippeviren des H5-Serotyps ausgelöst werden.

7. Ein Verfahren zur in-vitro-Diagnose von Infektionen mit Grippeviren des H5-Serotyps, umfassend das Kontaktieren einer biologischen Probe mit einer Zusammensetzung von monoklonalen Antikörpern wie in Ansprüchen 1-3 definiert, und das Detektieren der Bindung dieser Antikörper an H5-Serotyp-Grippeviren oder H5-Hämagglutinin-Protein.

8. Ein diagnostisches Kit, geeignet zur in-vitro-Detektion von Infektionen mit H5-Serotyp-Grippeviren, enthaltend die Zusammensetzung von monoklonalen Antikörpern wie in Anspruch 1 definiert oder deren Fragmente wie in Anspruch 1 definiert oder eine Zusammensetzung von monoklonalen Antikörpern wie in Anspruch 3 definiert, und Reagenzien zur Detektion von Antikörpern oder deren funktionalen Fragmenten, welche an H5-Serotyp-Grippeviren oder H5-Hämagglutinin-Protein gebunden sind.

9. Ein diagnostisches Kit, geeignet zur in-vitro-Detektion, Quantifizierung oder Semiquantifizierung von H5-Serotyp-Grippeviren in biologischen Proben, umfassend die Zusammensetzung von monoklonalen Antikörpern wie in Ansprüchen 1-3 definiert, und Hilfsmittel und Reagenzien, die üblicherweise in immunologischen Tests verwendet werden.

10. Ein diagnostisches Kit, geeignet zur in-vitro-Detektion, Quantifizierung oder Semiquantifizierung von Antikörpern gegen H5-Serotyp-Grippeviren in biologischen Proben, umfassend die Zusammensetzung von monoklonalen Antikörpern wie in Ansprüchen 1-3 definiert, und Hilfsmittel und Reagenzien, die üblicherweise in immunologischen Tests verwendet werden.

11. Das diagnostische Kit nach einem der Ansprüche 8-10, worin die Antikörper in der nichtisolierten Form oder isoliert und gereinigt sind.

12. Das diagnostische Kit nach einem der Ansprüche 8-11, dadurch charakterisiert, dass es in den Tests verwendet wird, die ausgewählt sind aus der Gruppe umfassend: immunoenzymatische, vorzugsweise bELISA H5, immunofluoreszente, immunochemolumineszente, radioimmunologische, immunochromatographische, Immunodiffusions-, Immunopräzipitationstests, und einen Test, der Immunosensoren verwendet, die als sogenannte DIVA-Tests zur Unterscheidung von infizierten und geimpften Tieren verwendet werden können.

13. Das diagnostische Kit nach einem der Ansprüche 8-12, dadurch charakterisiert, dass es die Unterscheidung von infizierten und geimpften Tieren erlaubt, auch bekannt als DIVA (Differentiation of Infected from Vaccinated Animals)-Test.

## Revendications

1. Composition d'anticorps monoclonaux dirigés contre l'hémagglutinine des virus de la grippe de sérotype H5, dépourvus de l'activité d'inhibition de l'hémagglutination (IH), qui se lient aux épitopes des formes natives des antigènes de H5, et ne présentent pas de réactivité croisée avec les virus qui ne sont pas de sérotype H5, comprenant :
(i) G-1-31-22 produit par la lignée cellulaire d'hybridome de souris G-1-31-22 déposée auprès de la DSMZ sous le numéro DSM ACC3292,
(ii) G-2-14-10 produit par la lignée cellulaire d'hybridome de souris G-2-14-10 déposée auprès de la DSMZ sous le numéro DSM ACC3293,
(iii) G-5-32-5 produit par la lignée cellulaire d'hybridome de souris G-5-32-5 déposée auprès de la DSMZ sous le numéro DSM ACC3294,
(iv) G-6-42-42 produit par la lignée cellulaire d'hybridome de souris G-6-42-42 déposée auprès de la DSMZ sous le numéro DSM ACC3295,
(v) G-7-24-17 produit par la lignée cellulaire d'hybridome de souris G-7-24-17 déposée auprès de la DSMZ sous le numéro DSM ACC3296, et
(vi) G-7-27-18 produit par la lignée cellulaire d'hybridome de souris G-7-27-18 déposée auprès de la DSMZ sous le numéro DSM ACC3297 ;
ou alors ladite composition comprenant des fragments de chacun desdits anticorps, où lesdits fragments sont Fab, Fab', F(ab')₂, Fv ou des fragments variables à chaîne unique (scFv) constitués de domaines V_{L} et V_{H} liés les uns aux autres par un lieur peptidique.

2. Composition d'anticorps monoclonaux selon la revendication 1, **caractérisée en ce que** l'hémagglutinine provient des virus de la grippe du sérotype H5, notamment H5N1, H5N2, H5N3 et H5N9.

3. Composition d'anticorps monoclonaux selon l'une quelconque des revendications 1-2, **caractérisée en ce qu'**un anticorps monoclonal est conjugué à un marqueur détectable par analyse, un promédicament, un médicament, un agent thérapeutique, un peptide, une protéine, une enzyme, un virus, un lipide, du PEG.

4. Lignée cellulaire d'hybridome de souris choisie dans le groupe comprenant :
(i) G-1-31-22 déposée auprès de la DSMZ sous le numéro DSM ACC3292,
(ii) G-2-14-10 déposée auprès de la DSMZ sous le numéro DSM ACC3293,
(iii) G-5-32-5 déposée auprès de la DSMZ sous le numéro DSM ACC3294,
(iv) G-6-42-42 déposée auprès de la DSMZ sous le numéro DSM ACC3295,
(v) G-7-24-17 déposée auprès de la DSMZ sous le numéro DSM ACC3296 et
(vi) G-7-27-18 déposée auprès de la DSMZ sous le numéro DSM ACC3297.

5. Composition pharmaceutique ou de diagnostic comprenant une composition d'anticorps définis dans les revendications 1-3 et un véhicule ou marqueur approprié.

6. Composition telle que définie dans la revendication 1, destinée à être utilisée dans le traitement d'infections induites par les virus de la grippe du sérotype H5.

7. Procédé pour diagnostiquer in vitro les infections provoquées par les virus de la grippe du sérotype H5, comprenant la mise en contact d'un échantillon biologique avec une composition d'anticorps monoclonaux tels que définis dans les revendications 1-3 et la détection de la liaison desdits anticorps avec les virus de la grippe de sérotype H5 ou la protéine hémagglutinine H5.

8. Trousse de diagnostic appropriée pour la détection in vitro d'infections provoquées par les virus de la grippe de sérotype H5, contenant la composition d'anticorps monoclonaux tels que définis dans la revendication 1 ou de leurs fragments tels que définis dans la revendication 1 ou une composition d'anticorps monoclonaux tels que définis dans la revendication 3, et des réactifs pour la détection des anticorps ou de leurs fragments fonctionnels liés aux virus de la grippe de sérotype H5 ou à la protéine hémagglutinine H5.

9. Trousse de diagnostic appropriée pour la détection, la quantification ou la semi-quantification in vitro de virus de la grippe de sérotype H5 dans des échantillons biologiques, comprenant la composition d'anticorps monoclonaux tels que définis dans les revendications 1-3 et les outils et réactifs couramment utilisés dans les tests immunologiques.

10. Trousse de diagnostic appropriée pour la détection, la quantification ou la semi-quantification in vitro d'anticorps dirigés contre les virus de la grippe de sérotype H5 dans des échantillons biologiques, comprenant la composition d'anticorps monoclonaux tels que définis dans les revendications 1-3 et les outils et réactifs couramment utilisés dans les tests immunologiques.

11. Trousse de diagnostic selon l'une des revendications 8-10, dans laquelle les anticorps sont sous la forme non isolée ou sont isolés et purifiés.

12. Trousse de diagnostic selon l'une des revendications 8-11, **caractérisée en ce qu'**elle est utilisée dans les tests choisis dans le groupe comprenant : les tests immuno-enzymatiques, de préférence bELISA H5, immunofluorescents, immunochimioluminescents, radio-immunologiques, immunochromatographiques, d'immunodiffusion, d'immunoprécipitation, un test utilisant des immunocapteurs qui peuvent servir de tests DIVA pour la différenciation entre les animaux infectés et les animaux vaccinés.

13. Trousse de diagnostic selon l'une des revendications 8-12, **caractérisée en ce qu'**elle permet la différenciation entre les animaux infectés et les animaux vaccinés, également connue sous le nom de test DIVA (Differentiation of Infected from Vaccinated Animais).
